# EUROPEAN PATENT APPLICATION

(11) **EP 4 378 474 A2**
(43) Date of publication of application: **05.06.2024**
(21) Application number: 24161470.0
(22) Date of filing: 16.06.2021
(51) Int. Cl.: A61K 39/00

(54) **RECOMBINANT HVT VECTORS EXPRESSING INFLUENZA HEMAGGLUTININ AND IMMUNOGENIC COMPOSITIONS, AND PRODUCTION AND USES THEREOF**

(30) Priority: 17.06.2020 US 202063040411 P
(62) Divisional of application: 21740337.7
(71) Applicant: Boehringer Ingelheim Vetmedica GmbH, 55216 Ingelheim am Rhein (DE)
(72) Inventor: KASSA, Aemro, Watkinsville (US); MEBATSION, Teshome, Watkinsville (US)
(74) Representative: D Young & Co LLP

(57) **Abstract**

An avian influenza virus antigen and a polynucleotide that encodes the avian influena virus antigen. Further provided is a plasmid and recombinant virus vector comprising the sequence of the avian influenza virus antigen and a composition or vaccine comprising the antigen, plasmid or recombinant virus vector.

## Description

### CROSS-REFERENCE TO RELATED APPLICATIONS

This application claims priority to U.S. provisional application 63/040,411 filed on June 17, 2020.

### SEQUENCE LISTING

The instant application contains a Sequence Listing which has been submitted electronically in ASCII format and is hereby incorporated by reference in its entirety. Said ASCII copy, created on June 15, 2021, is named BI20-AH022-WO-1_SL.txt and is 69,660 bytes in size.

### FIELD OF THE INVENTION

The invention relates to recombinant viral vectors for the insertion and expression of foreign genes for use as safe immunization vehicles to protect against a variety of pathogens. It also relates to multivalent composition or vaccine comprising one or more recombinant viral vectors for protection against a variety of pathogens. The present invention relates to methods of making and using the recombinant viral vectors, as well as immunogenic compositions that include the vectors and/or antigens.

### BACKGROUND OF THE INVENTION

Poultry vaccination is widely used to protect poultry flocks against devastating diseases including Newcastle disease (ND), infectious bursal disease (IBD), Marek's disease (MD), infectious bronchitis (IB), infectious laryngotracheitis (ILT) and avian influenza (AI), for example, highly pathogenic avian influenza (HPAI). ND is caused by the avian paramyxovirus 1 (APMV-1) also designated ND virus (NDV) belonging to the Paramyxoviridae family. In some instances, MD is caused by Gallid herpesvirus 2 (Herpesviridae family) also designated as MD virus serotype 1 (MDV1). IB is caused by IB virus (IBV) belonging to the Coronaviridae family, ILT is caused by Gallid herpesvirus 1 (Herpesviridae family) also designated ILT virus (ILTV) and AI is caused by AI virus (AIV) belonging to the Orthomyxoviridae family.

Recombinant vaccines may be used to provide a protective immune response in avians against one or more different diseases. In particular, vaccines may reduce mortality, clinical signs, and effects in avian animals. For example, vaccines developed may be used to provide protection against infectious bursal disease (also known as Gumboro Disease), Newcastle disease (ND), infectious bronchitis (IB), infectious laryngotracheitis (ILT), avian influenza (AI) and/or Marek's Disease in avians, such as chickens.

A number of recombinant avian viral vectors have been proposed with a view to vaccinating birds against these avian pathogens. The viral vectors used comprise avipox viruses, especially fowlpox (EP-A-0,517,292), Marek's virus, such as serotypes 1, 2 and 3 (HVT) (WO87/04463; WO2013/082317), or alternatively the ITLV, NDV and avian adenovirus. When some of these recombinant avian viral vectors were used for vaccination, they display variable levels of protection.

Marek's disease virus (MDV) belongs to the family alphaherpesvirideae and the genus Mardivirus. The MDV virion is enveloped and about 160 nm in size. The capsid includes a large genome of linear double stranded DNA, between 100 and 200 kbp in size.

Among the serotypes of MDV are MDV serotype 1 (MDV1) and MDV serotype 2 (MDV2) which are both pathogenic to poultry, while MDV serotype 3 (MDV3) is not. In some instances, MDV3 is referred to as: Meleagrid herpesvirus 1, turkey herpesvirus, and/or herpesvirus of turkeys (HVT).

Different strains of MDV3 (i.e., HVT), such as PB1 or FC-126, have been used to vaccinate avians (e.g., chickens) against MD caused by MDV1 and/or MDV2. When protection against more virulent variants of MDV1 is required, HVT has been used in combination with an MDV2 vaccine-strain, for example SB1, or with an attenuated MDV1 vaccine strain.

Several recombinant herpesviruses of turkeys (also designated Meleagrid herpesvirus 1 or MDV serotype 3) vectors expressing antigens from various pathogens (US patent Nos. 5,980,906, 5,853,733, 6,183,753, 5,187,087) including IBDV, NDV, ILTV and AIV have been developed and licensed.

Several recombinant herpesviruses of turkeys (HVT, also designated Meleagrid herpesvirus 1 or MDV serotype 3) vectors expressing antigens from various pathogens (U.S. Pat. Nos. 5,980,906, 5,853,733, 6,183,753, 5,187,087) including IBDV, NDV, ILTV and AIV have been developed and licensed. For example, an HVT vector-expressing IBDV VP2 protective gene that has shown clear advantages over classical IBD vaccines (Bublot et al J.Comp. Path.2007, Vol. 137, S81-S84; U.S. Pat. No. 5,980,906). Other HVT vectors of interest are those expressing either NDV (Morgan et al 1992, Avian dis. 36, 858-70; U.S. Pat. Nos. 6,866,852; 5,650,153), ILTV (Johnson et al, 2010 Avian Dis 54, 1251-1259; U.S. Pat. Nos. 6,299,882; 5,853,733, EP 1801204), or NDV and IBDV (U.S. Pat. No. 9,114,108; WO2016102647, WO2013/057235, WO2015032910, WO2013144355) protective gene(s). US2016/0158347 reported the use of the oligodeoxynucleotide TLR21 agonist to increase the immune response against the antigen that expressed by HVT vector.

Further, since its emergence in 1996 in Guangdong, China, H5N1 highly pathogenic avian influenza (HPAI) viruses of A/goose/Guangdong/1/1996 (Gs/GD) lineage have spread globally infecting domestic and wild birds and occasionally spilling over into mammals including humans. Over time, the H5N1 HPAI virus has diverged into multiple phylogenetically and antigenically distinct clades and subclades based on the H5 hemagglutinin (HA) gene and antigenic variants have emerged that are resistant to many vaccine seed strains. Such genetic and antigenic diversity has created challenges in maintaining relevant H5 seed strains for poultry vaccines. Therefore, the development of a broadly protective H5 influenza A vaccine that can provide coverage of antigenically distinct co-circulating viruses as well as future drift variants is highly desirable.

Several strategies have been investigated to overcome this challenge and broaden the repertoire of neutralizing antibodies, including multivalent H5N1 vaccines, targeting conserved domains of HA, and synthetic consensus DNA antigen-based vaccines. In line with the latter strategy, the previously described methodology termed "Computationally Optimized Broadly Reactive Antigen" (COBRA) was utilized to generate antigens with novel H5 HA consensus sequences. In previous studies using mice, ferrets, and cynomolgus macaques, COBRA HA antigen vaccines protected against lethal challenge with homologous and heterologous H5N1 HPAI virus, showing more efficient viral clearance and eliciting broader antibody responses against different clades and sub-clades than mono- or polyvalent vaccines. In a recent study, COBRA HA virus-like particle (VLP) vaccines provided clinical protection in chickens challenged with a lethal dose of homologous H5N1 HPAI virus. However, upon challenge with a drifting variant H5N1 HPAI virus, COBRA HA VLP vaccines provided no or partial clinical protection in chickens, and reduction of virus shedding was suboptimal with both challenge strains. Moreover, the robust HA antibody response elicited by COBRA vaccines against the drifted strain did not translate to protective efficacy upon challenge with this virus, evidencing that the positive predictive value of HA antibody titers is not absolute. Collectively, these results emphasized the need to further improve antigen consensus sequences and vaccine formulation in order to enhance both clinical protection and reduction of virus shedding upon challenge with antigenic diverse HPAI strains.

HVT replicates in the birds' peripheral blood lymphocytes (PB L's) and is thus a systemic virus which induces an immune response of long duration that is mostly aimed at the cellular immune system. HVT vaccines are commercially available as frozen HVT-infected cells and can be applied to chickens at an early age, as they are relatively insensitive to antibodies against HVT such as in MDA. Because a new born chick faces infective pressure of MDV from its first day, therefore HVT vaccines are inoculated into chicks as early as possible, e.g. at the day of their hatching from the egg (day one), or even before hatching, while still in the egg. This last approach, so-called *'in ovo* vaccination', is a form of embryo vaccination, which is commonly applied at day 18 of embryonic development (ED), about 3 days before hatch.

Next to being used as a vaccine per se, HVT is also used as a viral vector vaccine for the expression and delivery of various immunogenic proteins to poultry, see e.g. WO 87/04463. Typically, the expressed gene encodes (a part of) a protective antigen of a micro-organism pathogenic to poultry, against which vaccination is required. Through the years many heterologous genes have been expressed in HVT vectors, such as from: Newcastle disease virus (NDV) (Sondermeijer et al., 1993, Vaccine, vol. 11, p. 349-358), infectious bursal disease virus (IBDV) (Darteil et al., 1995, Virology, vol. 211, p. 481-490), and of a parasite antigen (Cronenberg et al., 1999, Acta Viral., vol. 43, p. 192-197).

Administration of an HVT vector vaccine to poultry has been described to generate an immune response against an expressed heterologous gene, as well as against HVT which protects against MD. This is applied in a variety of commercial HVT vector vaccine products, for instance: the NDV F protein gene: lnnovax^{™}-ND (MSD Animal Health), and Vectormune^{™} HVT-NDV (Ceva); or the IBDV VP2 gene: Vaxxitek^{™} HVT+IBD (Merial; previously named: Gallivac^{™} HVT-IBD; Aly, R. et al., Report and Opinion 2012;4(4):1-11)), and Vectormune^{™} HVT-IBD (Ceva).

Alternatively, an HVT vector has been used for the expression and delivery of a therapeutic protein, e.g. a cytokine, to manipulate the chicken's immune response (WO 2009/156.367; Tarpey et al., 2007, Vaccine, vol. 25, p. 8529-8535).

Several methods have been described for inserting heterologous genes into HVT, such as by using homologous recombination (Sondermeijer et al., supra), Cosmid regeneration (US 5,961,982), or Bacmids (bacterial artificial chromosomes) (Baigent et al., 2006, J. of Gen. Viral., vol. 87, p. 769-776).

Many genetic locations for the insertion of a heterologous gene-construct into the HVT genome have been investigated, and several suitable, non-essential loci have been described, e.g. in the unique short (Us) region of the HVT genome (EP 431.668); or in the HVT unique long (UL) region (EP 794.257).

Different promoters have been used to drive the expression of a heterologous gene in an expression cassette for HVT, such as: the PRV gpX promoter (WO 87/04.463), the Rous sarcoma virus L TR promoter, the SV40 early gene promoter, the chicken beta-actin gene promoter (EP 1.298.139), or the immediate early 1 gene promoter from human (hCMV IE1) or murine (mCMV IE1) cytomegalovirus, see: EP 728.842. Recently an HVT vector vaccine was described that expressed antigens from both NOV and IBDV from a single construct: WO 2013/057.235.

An HVT vector expressing IBDV VP2 protective gene has been used (Bublot et al J.Comp. Path.2007, Vol.137, S81-S84). Other HVT vectors of interest are those expressing either NDV (Morgan et al 1992, Avian dis. 36, 858-70) or ILTV (Johnson et al, 2010 Avian Dis 54, 1251-1259) protective gene(s). One of the practical problems of using several HVT-based recombinant vaccines together is their interference. Lower protection is induced at least against one of the disease when two HVT recombinant expressing different antigens are mixed (Rudolf Heine 2011; Issues of the Poultry Recombinant Viral Vector Vaccines which May Cause an Effect on the Economic Benefits of those Vaccines; paper presented at the XVII World Veterinary Poultry Association (WVPA) Congress in Cancun, Mexico, August 14-18, 2011; Slacum G, Hein R. and Lynch P., 2009, The compatibility of HVT recombinants with other Marek's disease vaccines, 58th Western Poultry Disease Conference, Sacramento, CA, USA, March 23rd-25th, p 84).

The combination of HVT and SB-1, a Gallid herpesvirus 3 (MDV serotype 2 or MDV-2) vaccine strain, has shown a synergistic effect on MD protection (Witter and Lee, 1984, Avian Pathology 13, 75-92). To address the interference problem, it is of interest to evaluate the SB-1 virus as a vaccine vector to express protective antigen(s) that could be compatible with HVT vector and improve MD protection.

The SB-1 genome was cloned and characterized in bacterial artificial chromosome (BAC) (Petherbridge, et al., J. Virol. Methods 158, 11-17, 2009; Singh et al, Research in Veterinary Science 89, 140-145, 2010). The MDV2 SB-1 sequence was recently obtained and analyzed (Spatz and Schat, Virus Gene 42, 331-338, 2011). A glycoprotein E deletion of SB-1 virus was described by Petherbridge et al. (J. Virol. Methods 158, 11-17, 2009). However, no research has been reported using SB-1 as a viral vector expressing foreign protective genes.

It has been shown that both U_{L}13 protein kinase and glycoprotein C (U_{L}44) genes individually are essential for horizontal transmission of MDV in chickens (Jarosinski, et al, J. of Virology 81, 10575-10587, 2007; Jarosinski, et al, J. of Virology 84, 7911- 7916, 2010).

One of the practical problems of using several HVT-based recombinant vaccines together is their interference. Lower protection is induced at least against one of the disease when two HVT recombinants expressing different antigens are mixed (Rudolf Heine 2011; Issues of the Poultry Recombinant Viral Vector Vaccines which May Cause an Effect on the Economic Benefits of those Vaccines; paper presented at the XVII World Veterinary Poultry Association (WVPA) Congress in Cancun, Mexico, Aug. 14-18, 2011; Slacum G, Hein R. and Lynch P., 2009, The compatibility of HVT recombinants with other Marek's disease vaccines, 5 8th Western Poultry Disease Conference, Sacramento, Calif., USA, March 23rd-25th, p 84).

Considering the potential effect of animal pathogens, such as MDV, AIV, NDV and/or IBDV on veterinary public health and the economy, efficient methods of preventing infection and protecting animals are needed. There is a need for a solution of combined effective vector vaccines and a suitable method for making the vaccine that could alleviate the problem of interference observed between 2 HVT-based vector vaccines.

Citation or identification of any document in this application is not an admission that such document is available as prior art to the present invention.

### SUMMARY OF THE INVENTION

Disclosed herein are recombinant vectors that encompass herpesvirus of turkeys, Marek's Disease virus (MDV), and/or Meleagrid herpesvirus. For example, recombinant vectors may include a mutated polynucleotide encoding Marek's Disease virus (MDV) such as vHVT509, vHVT522, and/or vHVT523. Also disclosed are compositions, for example immunogenic compositions, and/or vaccines that include recombinant vectors vHVT509, vHVT522, and/or vHVT523.

A recombinant virus vector may include at least one nucleic acid sequence derived from a herpesvirus of turkeys (rHVT), a Marek's disease virus, or a Meleagrid herpesvirus, a first exogenous nucleic acid sequence from an avian influenza virus such that the recombinant virus vector expresses *in vivo* an exogenous avian influenza virus hemagglutinin antigen, and a second exogenous nucleic acid sequence from an avian disease encoding for and expressing at least one antigen of the avian disease *in vivo.* In some embodiments, the recombinant virus vector may include Marek's disease virus serotype 3. Further, in some instances the recombinant virus vector may include a Marek's disease virus, serotype 3, strain FC-126 (MDV-3 FC-126).

The immunogenic compositions and the recombinant virus vectors described herein may be used to treat avians such as chickens, capons, ducks, geese, turkeys, pheasants, grouse, quails, swans, squabs, or pigeons. For example, avians treated with the immunogenic composition may be chickens.

Recombinant virus vectors may include an exogenous nucleic acid of avian influenza, subtype H5. Further, the exogenous avian influenza virus HA antigen may include a COBRA-C H5 hemagglutinin protein. In some instances, the exogenous nucleic acid sequence encoding the avian influenza HA antigen may be codon optimized.

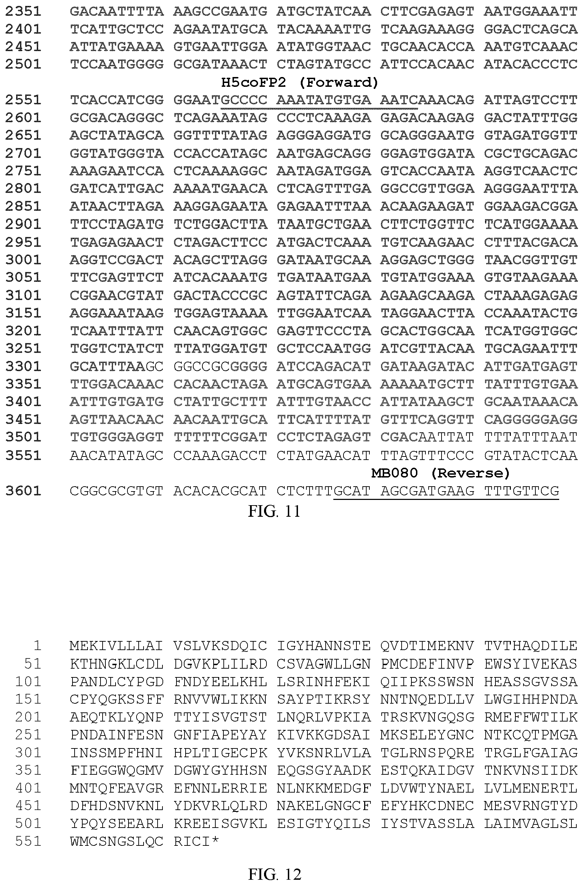

A recombinant virus vector may include at least one Avian Influenza virus amino acid sequence that has at least 80 percent, 90 percent, 92 percent, 95 percent or 99 percent sequence identity to a predetermined sequence, such as a sequence depicted in FIG. 12, FIG. 18, FIG. 28, FIG. 40, or FIG. 49. For example, a gene encoding an Avian Influenza virus HA H5 synthetic hemagglutinin (HA) protein may have a homology or identity greater than about 80 percent, 90 percent, 92 percent, 95 percent or 99 percent sequence identity to a reference sequence such as any depicted in FIG. 12, FIG. 18, FIG. 28, FIG. 40, OR FIG. 49. Further, the composition or vaccine may include a pharmaceutically or veterinarily acceptable carrier, excipient, vehicle, or adjuvant. In some embodiments, an expressed Avian Influenza virus antigen may have a homology or identity in a range from about 80 to about 100 percent to a reference sequence such as sequences depicted in FIG. 12, FIG. 18, FIG. 28, FIG. 40, OR FIG. 49.

In some embodiments, the recombinant virus vector may include one or more exogenous nucleic acid sequences that encodes an infectious bursal disease virus antigen, a Newcastle disease antigen, and/or an infectious laryngotracheitis virus (ILTV) antigen. For example, a recombinant virus vector may include an exogenous nucleic acid sequence that encodes an infectious bursal disease virus VP2 antigen and/or a Newcastle disease F antigen.

In some instances, a vaccine may be formed from multiple components. Components of a vaccine for inoculating avians may be stored in separate containers prior to use. For example, immunogenic agents, such as cells including a viral vector, viral vectors, antigens, and/or proteins, may be stored in a container such as an ampoule and combined with a diluent in a separate container prior to use. In particular, an ampoule may include infected cells frozen in liquid nitrogen that include viral vectors and/or antigens to a specific disease and may be combined with a diluent prior to injection into a target animal.

Vaccines for use in animals may combine the contents of multiple ampoules for different diseases with a diluent depending on the targeted diseases.

Immunogenic compositions may include a veterinarily acceptable carrier such as solvents, dispersion media, coatings, adjuvants, stabilizing agents, diluents, preservatives, antibacterial agents, antifungal agents, isotonic agents, adsorption delaying agents, and combinations thereof.

A recombinant virus vector may include at least one exogenous amino acid sequence having at least 80 percent, 85 percent, 90 percent, 95 percent, 96 percent, 97 percent, 98 percent or 99 percent sequence identity to a sequence that encodes an AIV H5 HA Cobra-C gene as shown in FIG. 12, FIG. 18, FIG. 28, FIG. 40, and/or FIG. 49. In some embodiments, a recombinant virus vector may at least one exogenous amino acid sequence having at least 90 percent sequence identity to a sequence that encodes an AIV H5 HA Cobra-C gene as shown in FIG. 12, FIG. 18, FIG. 28, FIG. 40, and/or FIG. 49. In particular, a recombinant virus vector may include at least one exogenous amino acid sequence having at least 95 percent sequence identity to a sequence that encodes an AIV H5 HA Cobra-C gene as shown in FIG. 12, FIG. 18, FIG. 28, FIG. 40, and/or FIG. 49. For example, a recombinant virus vector may include at least one sequence having a homology or identity in a range from about 80 to about 100 percent to a sequence that encodes an AIV H5 HA Cobra-C gene as shown in FIG. 12, FIG. 18, FIG. 28, FIG. 40, and/or FIG. 49.

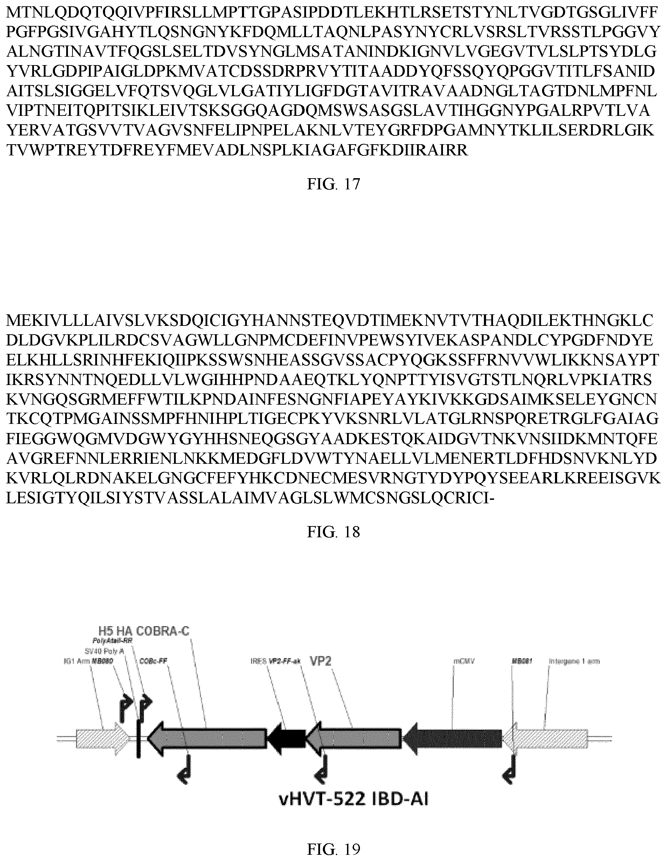

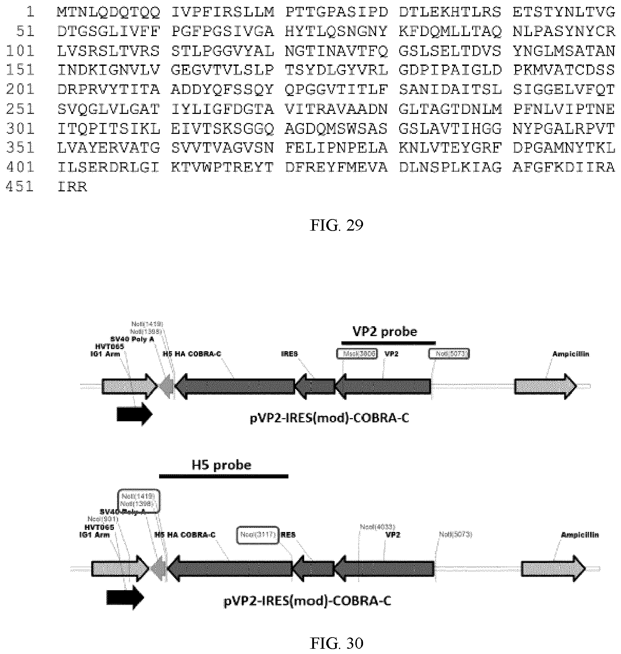

A recombinant virus vector may include at least one exogenous amino acid sequence having at least 80 percent, 85 percent, 90 percent, 95 percent, 96 percent, 97 percent, 98 percent, or 99 percent sequence identity to a sequence that encodes a capsid protein (VP2) protein of IBDV as shown in FIG. 17 or FIG. 29. In some embodiments, a recombinant virus vector may at least one exogenous amino acid sequence having at least 90 percent sequence identity to a sequence that encodes a capsid protein (VP2) protein of IBDV as shown in FIG. 17 or FIG. 29. In particular, a recombinant virus vector may include at least one exogenous amino acid sequence having at least 95 percent sequence identity to a sequence that encodes the capsid protein (VP2) protein of IBDV as shown in FIG. 17 or FIG. 29.

A recombinant virus vector may include at least one amino acid sequence having a homology or sequence identity in a range from about 80 to about 100 percent to sequence that encodes an IBDV capsid protein (VP2) protein as depicted in FIG. 17 or FIG. 29.

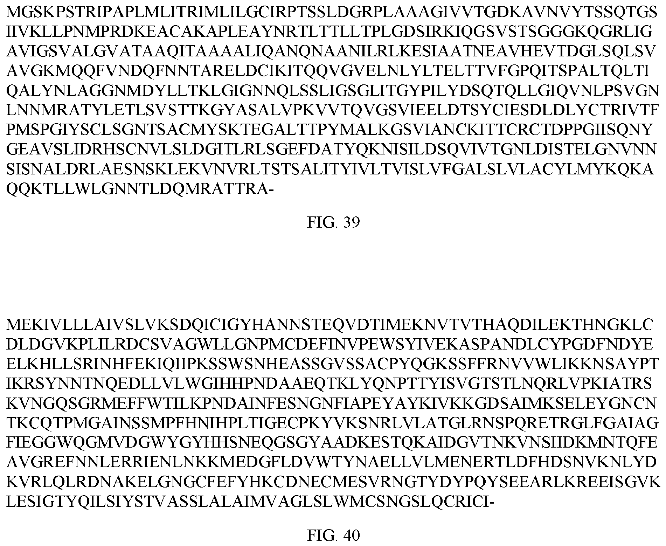

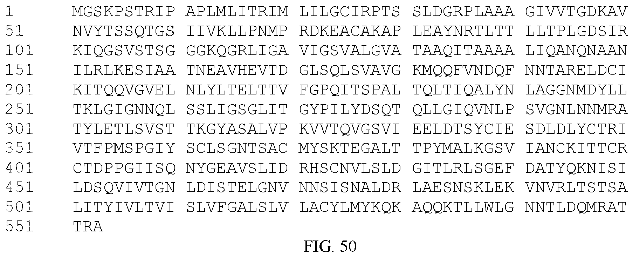

A recombinant virus vector may include at least one exogenous amino acid sequence having at least 80 percent, 85 percent, 90 percent, 95 percent, 96 percent, 97 percent, 98 percent, or 99 percent sequence identity to a sequence that encodes an NDV-F protein as shown in FIG. 39 or FIG. 50. In some embodiments, a recombinant virus vector may at least one exogenous amino acid sequence having at least 90 percent sequence identity to a sequence that encodes an NDV-F protein shown in FIG. 39 or FIG. 50. In particular, a recombinant virus vector may include at least one exogenous amino acid sequence having at least 95 percent sequence identity to a sequence that an NDV-F protein as shown in FIG. 39 or FIG. 50.

For example, the recombinant virus vector may have a homology or identity in a range from about 80 to about 100 percent to a sequence for an NDV-F protein as shown in FIG. 39 or FIG. 50.

Recombinant virus vectors may be constructed by a recombination method that includes transfecting a cell with the predetermined virus and an insertion plasmid for insertion into an Intergenic I (IG1) site comprising the first exogenous nucleic acid sequence encoding the infectious bursal disease virus VP2 antigen, an Internal Ribosome Entry Site (IRES), the second exogenous nucleic acid sequence encoding avian influenza HA and SV40 poly A tail, flanked by sequences from the IG1 region.

A method for producing the recombinant virus vector may include transfecting a cell with the predetermined virus and an insertion plasmid for insertion into HVT Intergenic 1 (IG1) site comprising the first exogenous nucleic acid sequence encoding the infectious bursal disease virus VP2 antigen, an Internal Ribosome Entry Site (IRES), the second exogenous nucleic acid sequence encoding avian influenza HA and a SV40 poly A tail. In some embodiments, the viral vector may include flanking sequences from IG1.

After production of the recombinant virus vector it may be recovered and/or purified. For example, prior to use in an immunogenic composition the produced recombinant virus vector may be recovered and then purified for use in an immunogenic composition.

In an immunogenic composition, a predetermined virus, such as a Marek's disease virus, a herpesvirus of turkeys (rHVT), or a Meleagrid herpesvirus is in an amount, when the composition is administered to an avian, sufficient to induce a response to a targeted disease, such as an immune response to any of the targeted diseases including Marek's disease virus, Newcastle disease virus, infectious bursal disease virus, and/or avian influenza.

Immunogenic compositions, such as vaccines, may be formed after mixing an immunogenic agent with one or more veterinary-acceptable carriers. For example, a vaccine may be formed by combining an ampoule that includes an immunogenic agent and a veterinarily acceptable carrier, such as a diluent. In particular, an ampoule for mixing a vaccine may include an immunogenic agent, for example, cells that include viral vectors. The contents of an ampoule may be mixed with a container of diluent. In some embodiments, a vaccine may be formed by combining an ampoule that includes immunogenic agents, such as cells containing viral vectors frozen in liquid nitrogen with a predetermined amount of diluent.

Ampoules may be provided with an immunologic composition having a titer in a range from about 10³ to about 10⁷ PFU/ml. In an embodiment, an immunologic composition in an ampoule may have a titer in a range from about 10⁵ to 10⁷ PFU/ml.

In some embodiments, an immunologic composition in an ampoule may be more concentrated than a desired administration dose. In some embodiments, an immunologic composition in an ampoule may be about 3 times more concentrated than a desired administration dose. For example, in an embodiment where a dose of a vaccine to be administered is about 2500 PFU per dose, the release dose (i.e., the amount in the ampoule) may be about three time that, that is, about 7500 per dose.

Minimally effective/protective doses (MPD) of a particular viral vector expressing one or more antigens and/or proteins that are included in a vaccine may be determined experimentally. Generally, the MPD may be used to determine a preferred titer in the immunologic composition. For example, a release titer in the US may be a multiple of the MPD value. In particular, the preferred titer in an immunologic composition, such as a vaccine, may be about 3 times the MPD.

In some cases, an immunologic composition, such as a vaccine may have a titer in a range from about 1000 PFU/dose to about 8000 PFU/dose. An embodiment may include a vaccine having a titer in the range from about 2000 PFU/dose to about 6000 PFU/dose.

An embodiment of a vaccine for avians may have a titer in a range from about 2200 PFU/dose to about 4000 PFU/dose. Some avian vaccines described herein may include a titer of greater than about 2250 PFU/dose. In particular, a vaccine may have a titer in a range from about 2250 PFU/dose to about 3000 PFU/dose. For example, a vaccine may have a titer of about 2500 PFU/dose.

Some embodiments of an immunogenic composition, such as a vaccine may include titers greater than about 5000 PFU/dose for MDV (i.e., HVT) in particular for avians that will have a relatively long lifespan, such as breeders and layers.

A method for inducing an immune response in an avian against Marek's Disease Virus, Avian Influenza, and Infectious Bursal Disease Virus, may include administering to the avian an effective amount of the recombinant virus vector or an effective amount of the immunogenic composition described herein.

In an embodiment, a recombinant virus vector may include and express *in vivo* an exogenous avian influenza virus HA antigen, and an exogenous nucleic acid molecule encoding a Newcastle Disease Virus F antigen. In some instances, the exogenous avian influenza sequence may be subtype H5.

A recombinant virus vector may include at least one amino acid sequence that encodes an NDV protein that has at least 80 percent, 90 percent, 92 percent, 95 percent or 99 percent sequence identity to a sequence as shown in FIG. 39 or FIG. 50. In some embodiments, an *in vivo* expressed protein, such as the NDV-F protein may have a homology or identity in a range from about 80 to about 100 percent to a reference sequence such as the sequence depicted in FIG. 50. The exogenous nucleic acid molecule encoding the avian influenza HA antigen in a recombinant virus vector may be codon optimized.

Recombinant virus vectors may be constructed by transfecting a cell with a predetermined virus and an insertion plasmid for the Intergenic 1 (IG1) site comprising a mouse CMV promoter the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.

A method for producing the recombinant virus vector may include transfecting a cell with Marek disease virus and an insertion plasmid for the Intergenic 1 (IG1) site may include a mouse CMV promoter the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.

Any production of a recombinant virus vector may be recovered after production.

Immunogenic compositions as described herein may include veterinarily acceptable carrier.

Immunogenic compositions may include a recombinant virus vector in an amount sufficient to induce an immune response in an avian against Marek's Disease Virus and/or avian influenza virus and/or Newcastle Disease Virus after administration of the immunogenic composition.

A method for inducing an immune response in an avian against Marek's Disease Virus, Avian Influenza and Newcastle Disease Virus may include administering to an avian an effective amount of one or more immunogenic agents, such as cells infected with a recombinant virus vector. In some embodiments, an effective amount of an immunogenic composition such as a vaccine is determined.

A plasmid for insertion into an Intergenic 1 (IG1) site of a predetermined virus comprising an exogenous nucleic acid sequence encoding the Newcastle Disease Virus F antigen and/or protein, an Internal Ribosome Entry Site (IRES), an exogenous nucleic acid sequence encoding avian influenza HA and SV40 poly A tail.

For example, a plasmid for insertion into an Intergenic 1 (IG1) site of a MDV-3 may include a mouse CMV promoter the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.

In some instances, the AIV used in the plasmid may be of subtype H5. Exogenous nucleic acid molecules or sequences encoding the avian influenza HA antigen may be codon optimized.

A composition or vaccine may include a recombinant virus vector that includes a virus selected from Marek's Disease serotype 3 (MDV-3) or Meleagrid herpesvirus 1 (MeHV-1). In addition, one or more exogenous nucleotides, for example, an Avian Influenza virus nucleotide coding for and expressing a synthetic hemagglutinin (HA) protein gene. Further, an additional heterologous nucleotide coding for and expressing at least one antigen of an avian pathogen such as an Infectious Bursal Disease Virus (IBDV) capsid protein (VP2), or a Newcastle Disease Virus fusion protein (NDV-F), and a pharmaceutically or veterinarily acceptable carrier, excipient, vehicle, or adjuvant.

A composition or vaccine may include at least one Avian Influenza virus amino acid sequence having at least 80 percent, 90 percent, 92 percent, 95 percent or 99 percent sequence identity to a sequence as shown in FIG. 12, FIG. 18, FIG. 28, FIG. 40, AND FIG. 49. Further, the composition or vaccine may include a pharmaceutically or veterinarily acceptable carrier, excipient, vehicle, or adjuvant.

A composition or vaccine may include a viral vector having an amino acid sequence encoding at least one Infectious Bursal Disease Virus (IBDV) capsid protein (VP2) protein having at least 80 percent, 90 percent, 92 percent, 95 percent, or 99 percent sequence identity with a predetermined sequence as shown in FIG. 17 or FIG. 29. A composition or vaccine may include a viral vector that includes an amino acid sequence having at least 80 percent, 90 percent, 92 percent, 95 percent or 99 percent sequence identity to a predetermined sequence that encodes a synthetic hemagglutinin (HA) protein gene of Avian Influenza virus A (subtype H5) such as depicted in FIG. 12, FIG. 18, FIG. 28, FIG. 40 or FIG. 49 and another amino acid sequence having at least at least 80 percent, 90 percent, 92 percent, 95 percent or 99 percent sequence identity to a predetermined sequence that encodes an IBDV VP2 protein as shown in FIG. 17 or FIG. 29.

A composition or vaccine may include a viral vector that includes an amino acid sequence having at least 80 percent, 90 percent, 92 percent, 95 percent or 99 percent sequence identity to a sequence encoding an NDV protein, such as an NDV-F protein, as shown in FIG. 50 or FIG. 39. Further, the composition or vaccine may include a pharmaceutically or veterinarily acceptable carrier, excipient, vehicle, or adjuvant.

A composition or vaccine may include a viral vector that includes an amino acid sequence having at least 80 percent, 90 percent, 92 percent, 95 percent or 99 percent sequence identity to a predetermined sequence that encodes a synthetic hemagglutinin (HA) protein gene of Avian Influenza virus A (subtype H5) such as depicted in FIG. 12, FIG. 18, FIG. 28, FIG. 40 or FIG. 49 and another amino acid sequence having at least at least 80 percent, 90 percent, 92 percent, 95 percent or 99 percent sequence identity to a predetermined sequence that encodes a Newcastle Disease Virus fusion protein (NDV-F) as shown in FIG. 50 or FIG. 39.

Accordingly, it is an object of the invention not to encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product. It may be advantageous in the practice of the invention to be in compliance with Art. 53(c) EPC and Rule 28(b) and (c) EPC. All rights to explicitly disclaim any embodiments that are the subject of any granted patent(s) of applicant in the lineage of this application or in any other lineage or in any prior filed application of any third party is explicitly reserved. Nothing herein is to be construed as a promise.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

This patent or application file contains at least one drawing executed in color. Copies of this patent or patent application publication with color drawing(s) will be provided by the Office upon request and payment of the necessary fee.

The following detailed description, given by way of example, but not intended to limit the invention solely to the specific embodiments described, may best be understood in conjunction with the accompanying drawings.
FIG. 1 is a diagram showing virus constructs and the some of the elements thereof that may be used in the immunogenic compositions described herein.
FIG. 2 depicts location maps for trivalent viral vectors vHVT-522 and vHVT-523.
FIG. 3 is a primer location map for vHVT509.

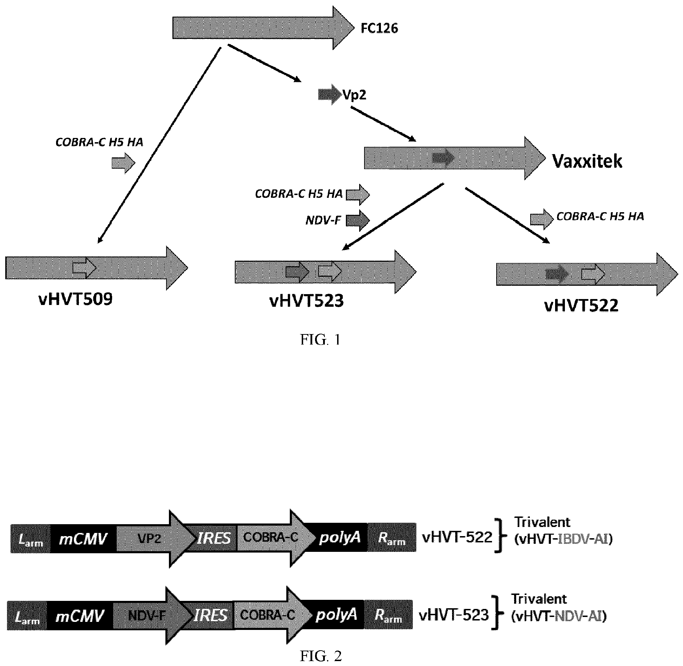

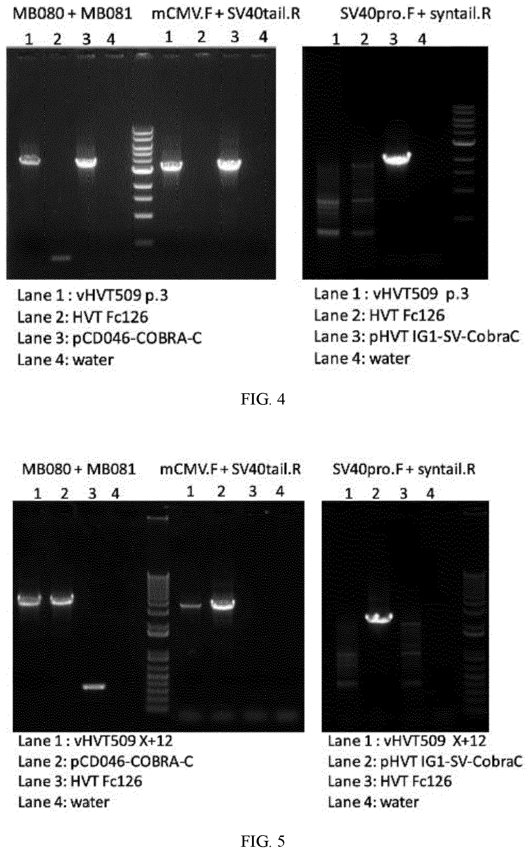

FIG.2FIG. 4 shows the PCR amplification of vHVT509 pre-MSV stock.

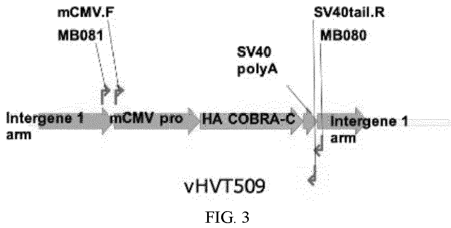

FIG.3FIG. 5 shows the PCR amplification of vHVT509 X+12 stock.

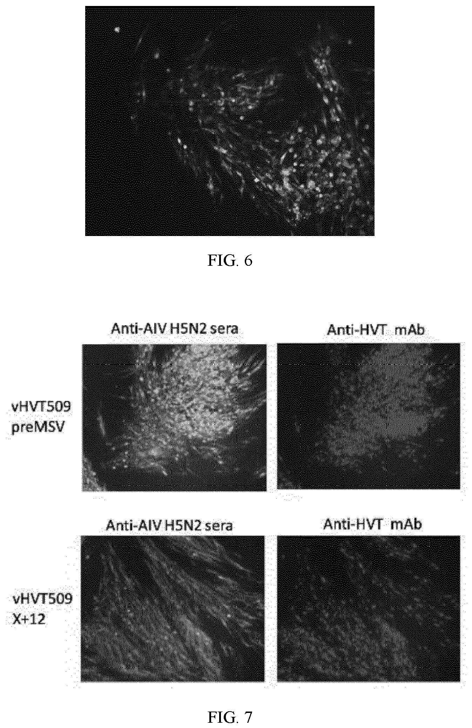

FIG. 6 shows the results of the indirect immunofluorescent assay of vHVT509 with chicken anti-AIV H5N2 sera (Charles Rivers Laboratories, Lot#J0210) and rabbit anti-chicken IgG-FITC conjugated (Sigma cat# F8888, lot#075M4812V).
FIG. 7 shows results from the dual indirect immunofluorescence assay of vHVT509.

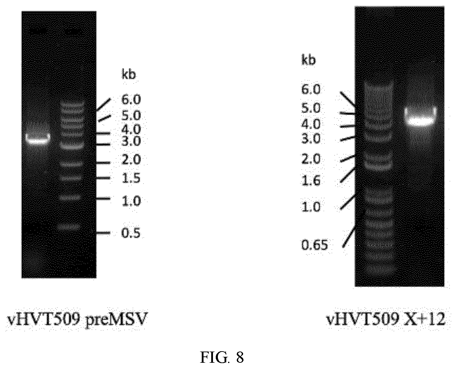

FIG. 8 shows the PCR amplicon for nucleotide sequence analysis of vHVT509.

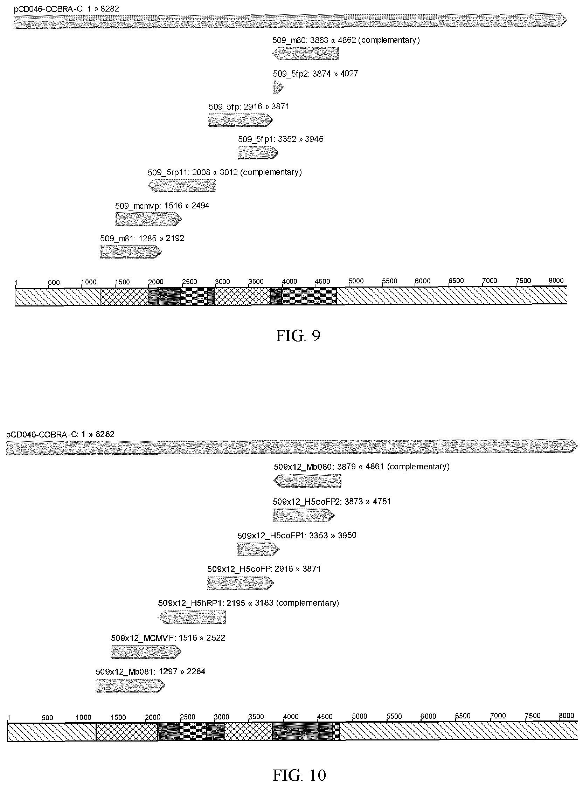

FIG. 9 is the graphic view of the contig that generated from each sequence reaction of vHVT509 pre-MSV.

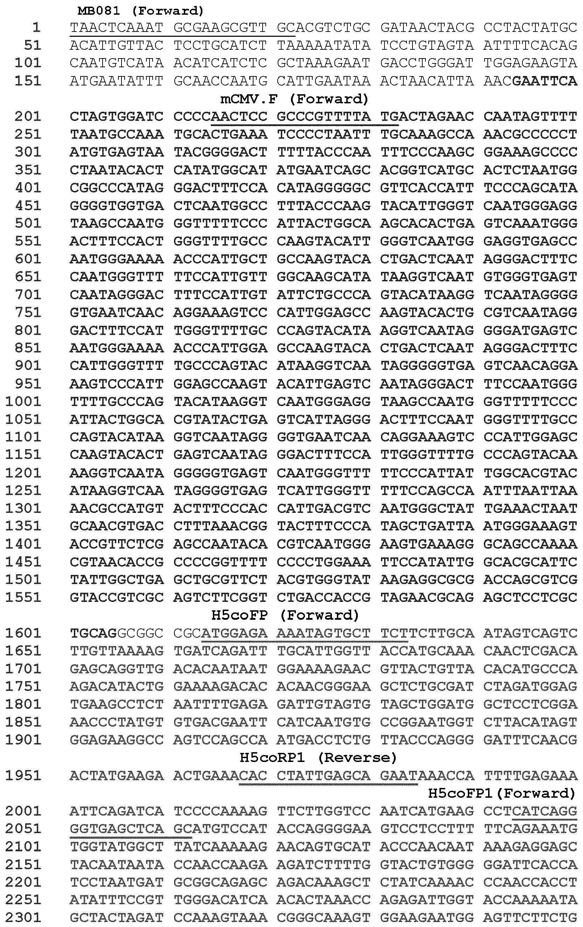

FIG. 10 is the graphic view of the contig that generated from each sequence reaction of vHVT509 X+12.

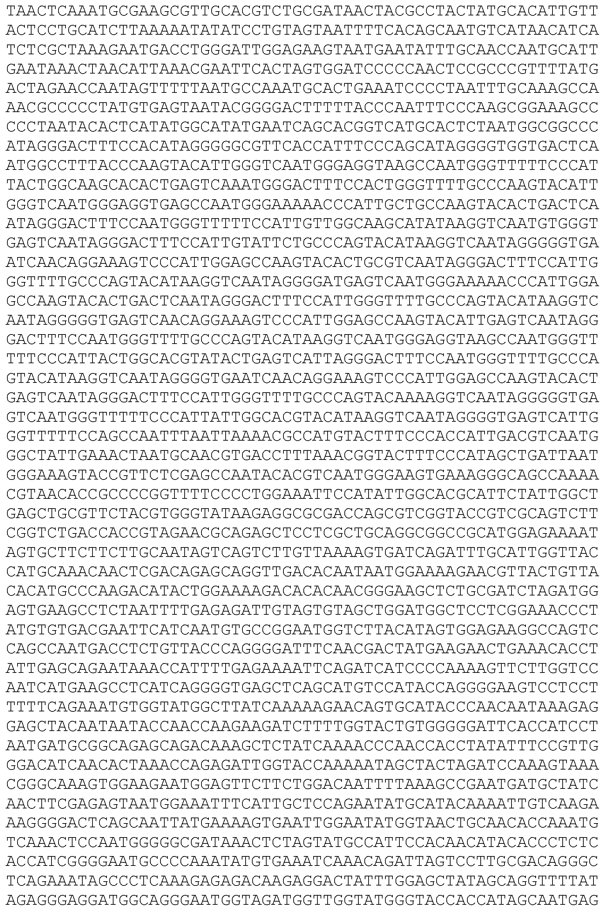

FIG. 11 shows the nucleotide sequences of vHVT509 pre-MSV and X+12 passage stock contig and sequencing primer location (SEQ ID NO: 47). Black = recombination arm regions, Blue = mCMV promoter, Red = AIV HA COBRA-C, Gray = synthetic poly A, Underline = Sequencing primer. Sequencing primers are underlined with direction of the primer. The part of recombination arms is colored black and AIV HA COBRA-C gene is colored red.
FIG. 12 shows the amino acid sequences of AIV HA COBRA-C gene in vHVT509 (SEQ ID NO: 48).

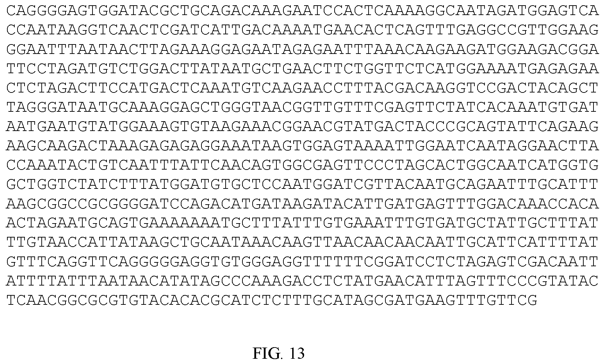

FIG. 13 shows the nucleotide sequences of the sequenced region of vHVT509 (SEQ ID NO: 47).

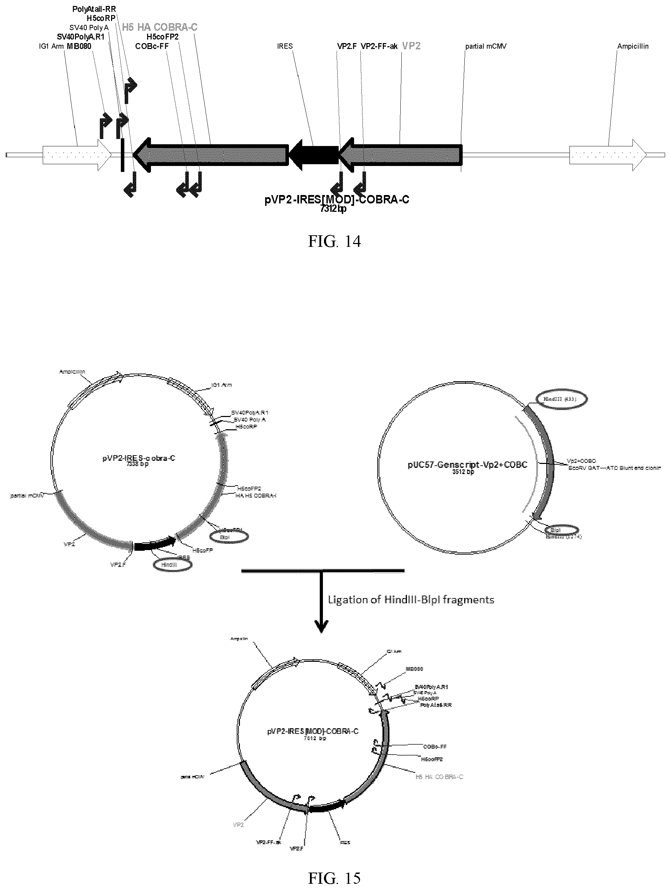

FIG. 14 is a diagram of the construction of the pVP2-IRES[MOD]-COBRA-C plasmid.

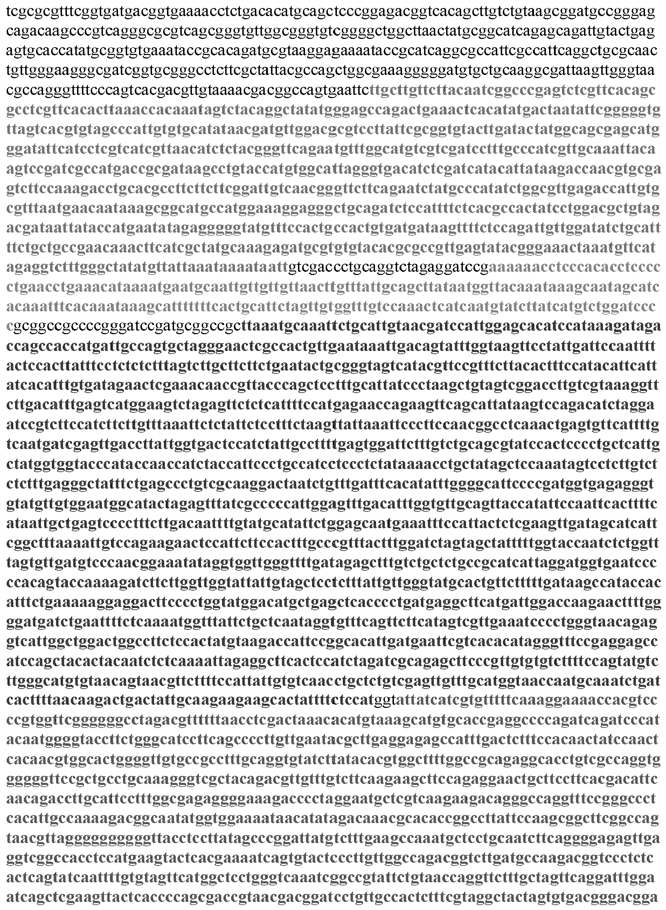

FIG. 15 is a plasmid diagram showing the ligation of HindIII-BIpl fragments.

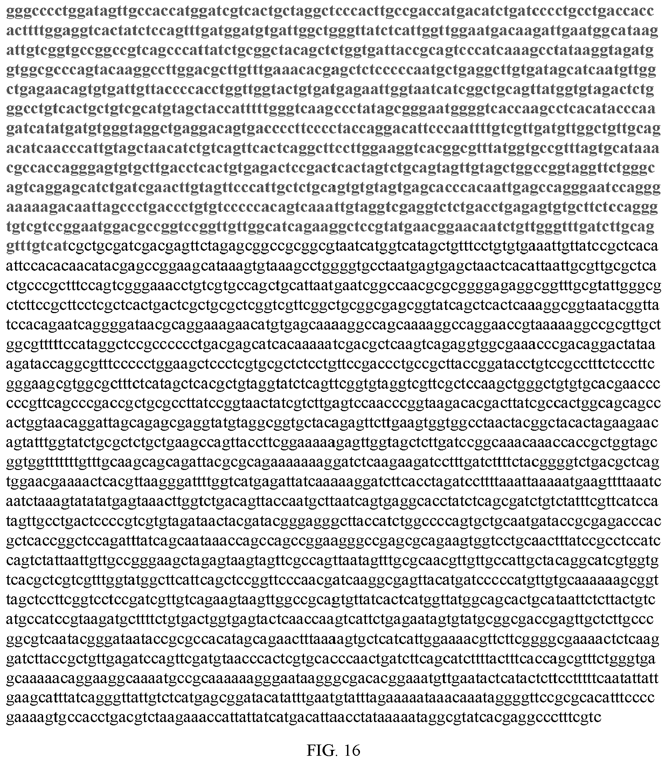

FIG. 16 is the nucleotide sequence of plasmid pVP2-IRES[MOD]-Cobra-C (SEQ ID NO: 49) where: Green = Intergene 1 Arms, Orange = SV40 Poly A, Dark Red = H5 HA COBRA-C, Blue = IRES, Red = VP2, Black = Vector.
FIG. 17 is the amino acid sequence of VP2 (SEQ ID NO: 50).
FIG. 18 is the amino acid sequence of H5 HA COBRA-C (SEQ ID NO: 48).
FIG. 19 is a diagram showing PCR primer locations on vHVT522.

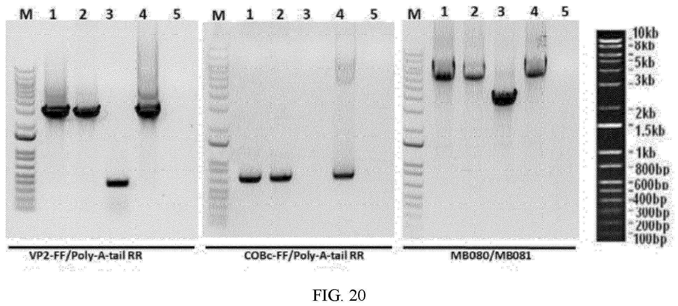

FIG. 20 is a picture of the agarose gel loaded with the PCR products using the following templates: vHVT522 pre-MSV (lane 1), vHVT522 pre-MSV+12 (lane 2), vHVT013 (lane 3), donor plasmid (pVP2-IRES[MOD]-Cobra-C (lane 4) and negative control (lane 5) and the different primer pairs indicated in Table 5.

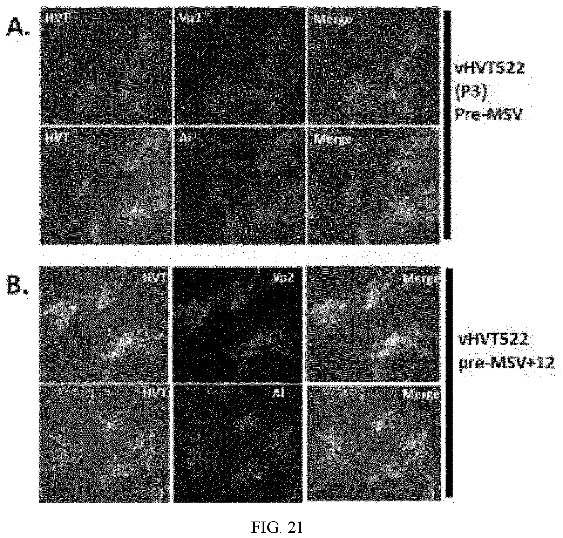

FIG. 21 shows dual immunofluorescence assay results, which shows a representative picture from the pre-MSV (P3) passage (A) and from the pre-MSV+12 passage (B).

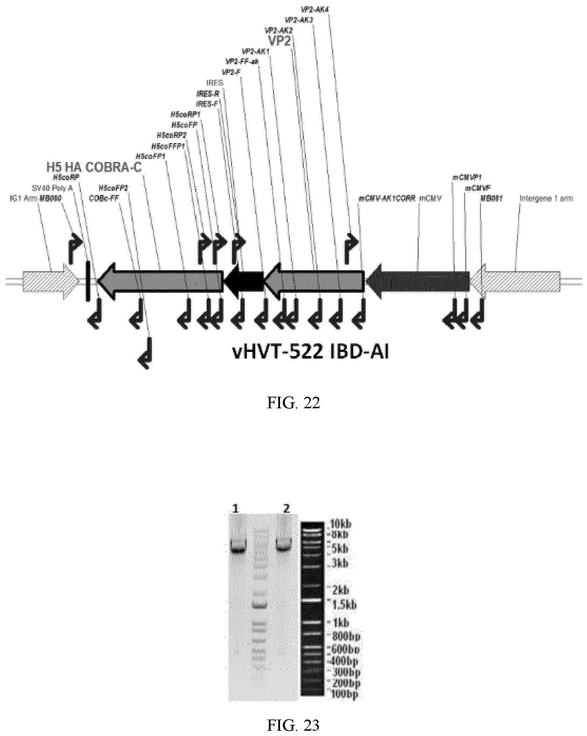

FIG. 22 is a diagram showing the locations of sequencing primers on vHVT522.
FIG. 23 shows vHVT522 pre-MSV (P3) and X+12 (pre-MSV+12) PCR for sequencing.

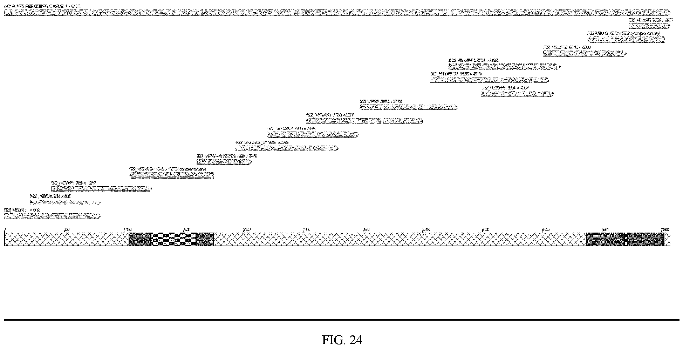

FIG. 24 is the vHVT522 (P3) pre-MSV sequencing contig.

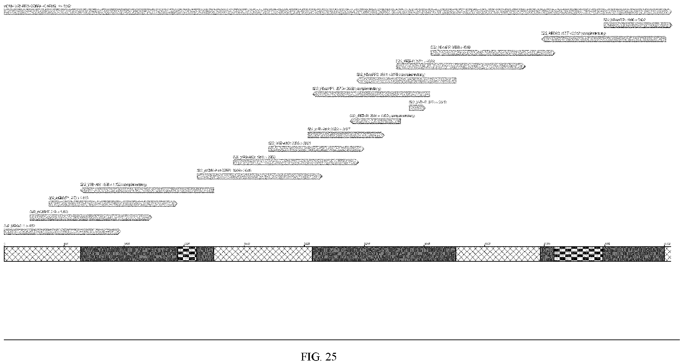

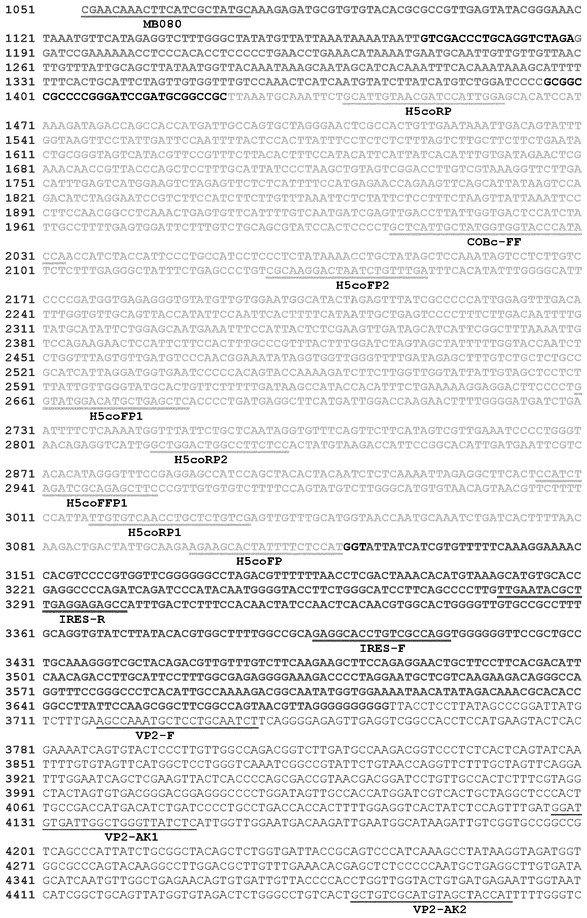

FIG. 25 is the vHVT522 pre-MSV+12 sequencing contig.

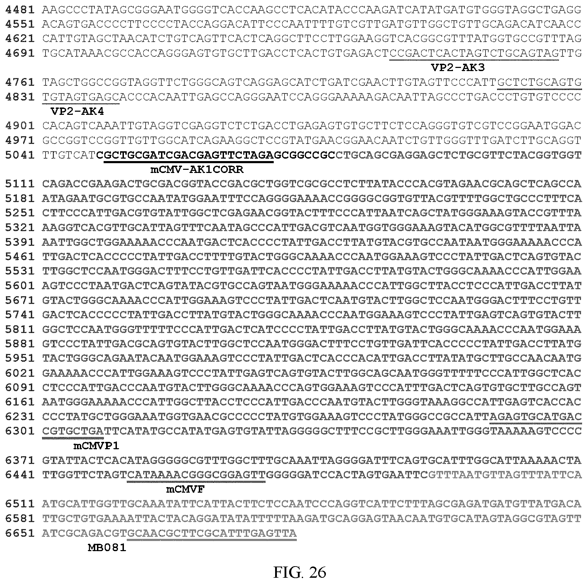

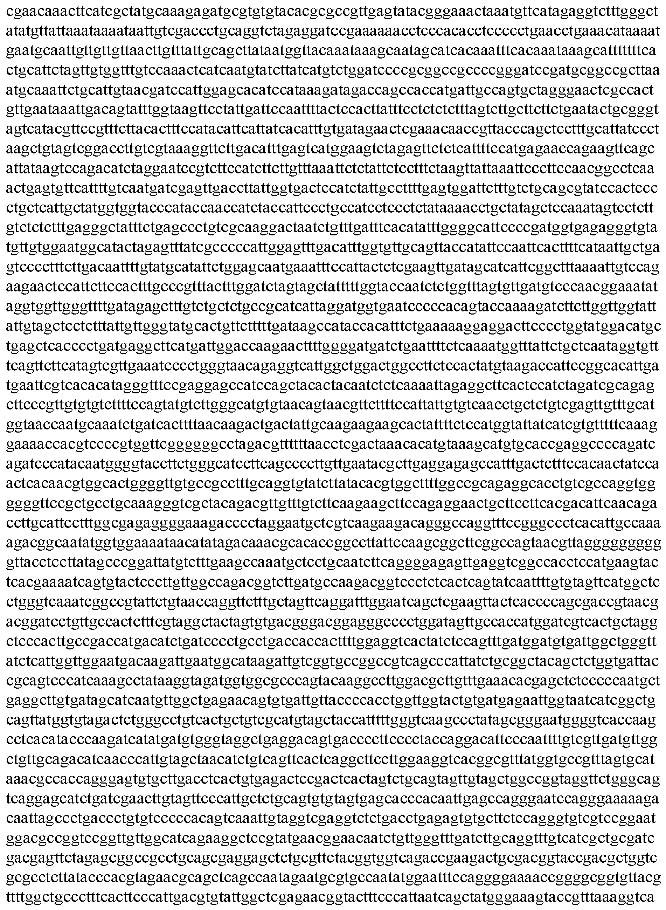

FIG. 26 is the nucleotide sequence of vHVT522 (SEQ ID NO: 51) with primer locations where: Green = IG1 Arm (pFIRESVP2 donor plasmid), Light Blue = SV40 poly A, Light Green = IG1 Arm (vHVT013 donor plasmid), Orange = NDV-F, Purple = IRES, Dark Red = VP2, Blue = mCMV, Underlined = Sequencing Primers.

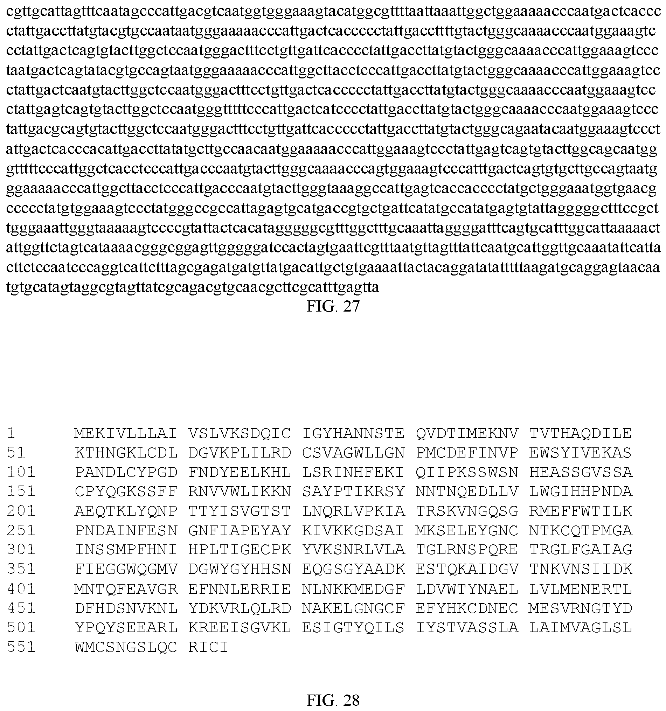

FIG. 27 shows the nucleotide sequence of the sequenced region of vHVT522 (SEQ ID NO: 51).
FIG. 28 shows the amino acid sequence of AIV HA COBRA-C gene in vHVT522 (SEQ ID NO: 48).
FIG. 29 shows the amino acid sequence of IBDV VP2 gene in vHVT522 (SEQ ID NO: 50).
FIG. 30 is the restriction map of the pVP2-IRES[MOD]-COBRA-C plasmid (donor plasmid) used to prepare the two Southern Blot probes. The H5 and VP2 probes, in black bars, are shown spanning the restriction sites, in red circles.

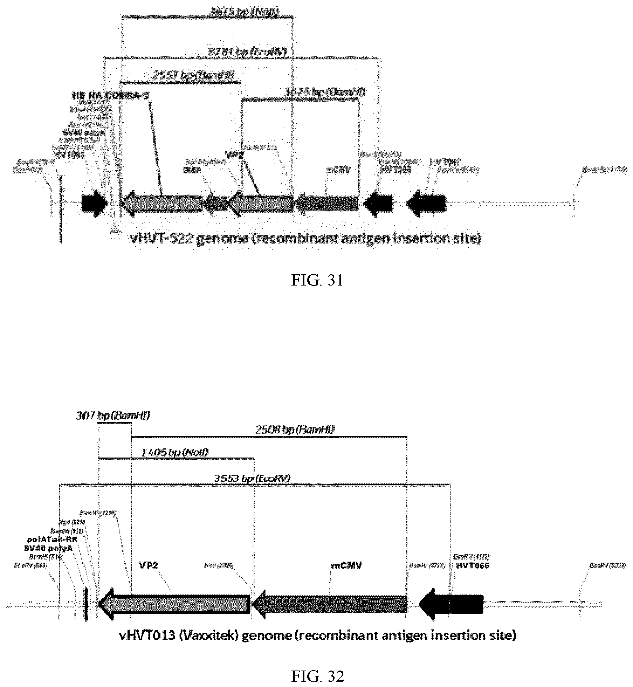

FIG. 31 is a schematic of the vHVT522 antigen insertion site, restriction map of endonucleases used for southern blot, and expected length of fragments.
FIG. 32 is a schematic of the vHVT013 (Vaxxitek) antigen insertion site, restriction map of endonucleases used for southern blot, and expected length of fragments.

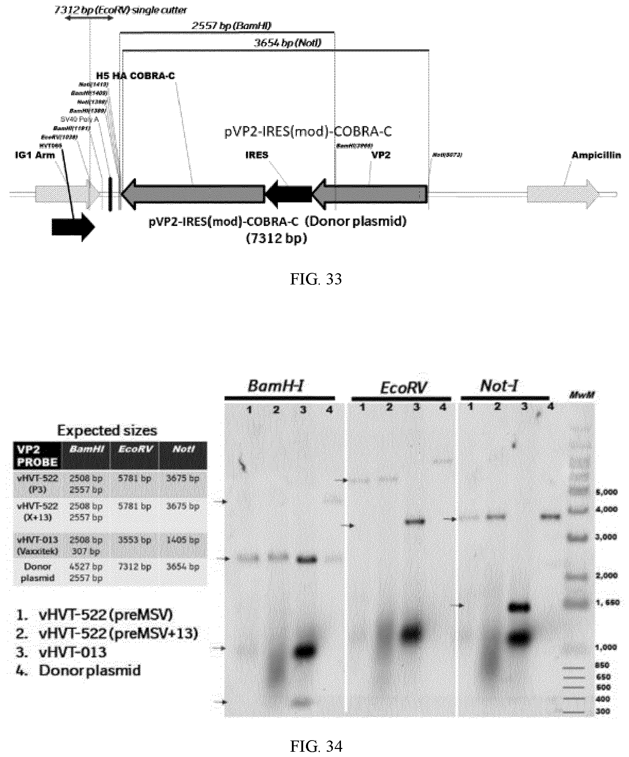

FIG. 33 is a schematic of the donor plasmid (pVP2-IRES[MOD]-COBRA-C), restriction map of endonucleases used for southern blot, and expected length of fragments.
FIG. 34 shows the expected Southern Blot bands using the VP2 Probe on vHVT522, vHVT013 and donor plasmid (pVP2-IRES [MOD]-COBRA-C) DNAs digested either with *BamHI*, *EcoRV* or *Not-I restriction enzymes.*

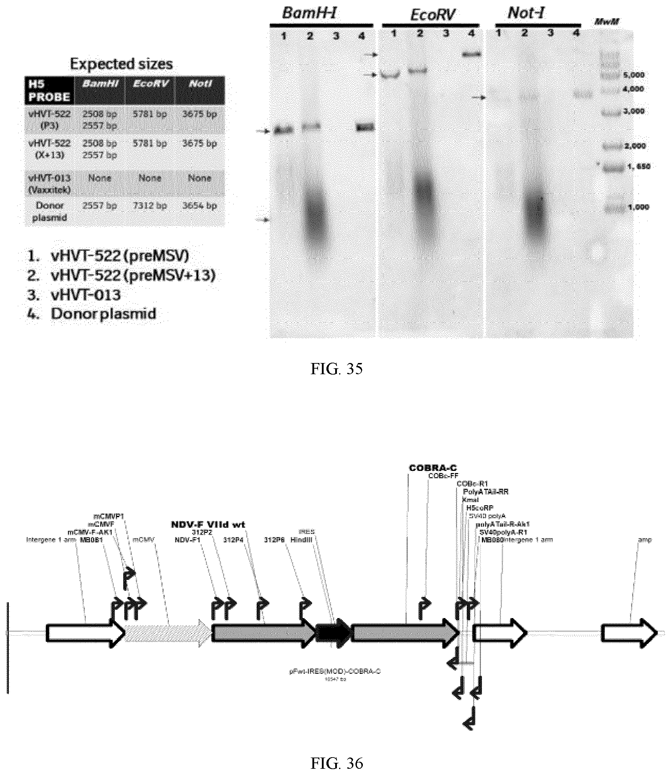

FIG. 35 shows the expected Southern Blot bands using the **H5 Probe** on vHVT522, vHVT-013 and donor plasmid (pVP2-IRES [MOD]-COBRA-C) DNAs digested either with *Bam*HI, EcoRV or *Not-I restriction enzymes.*
FIG. 36 shows the diagram of the donor plasmid construction of pFwt-IRES[MOD]-COBRA-C.

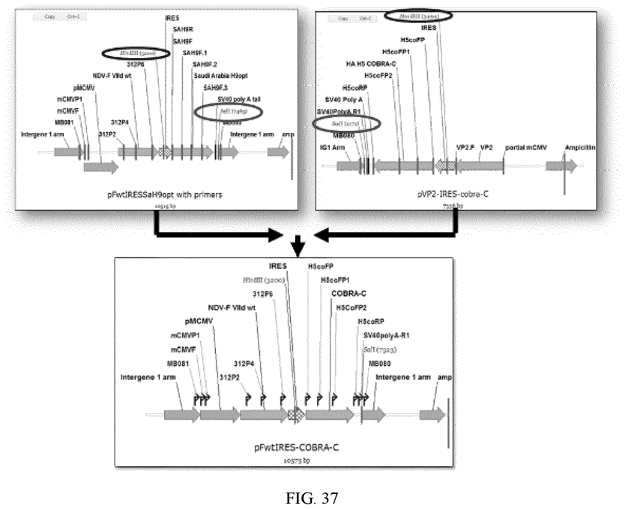

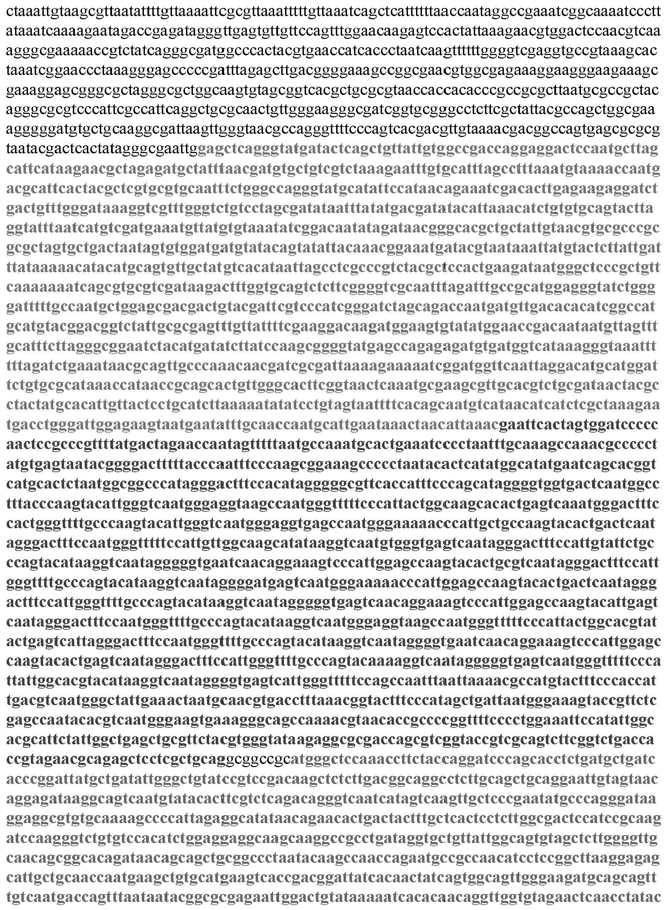

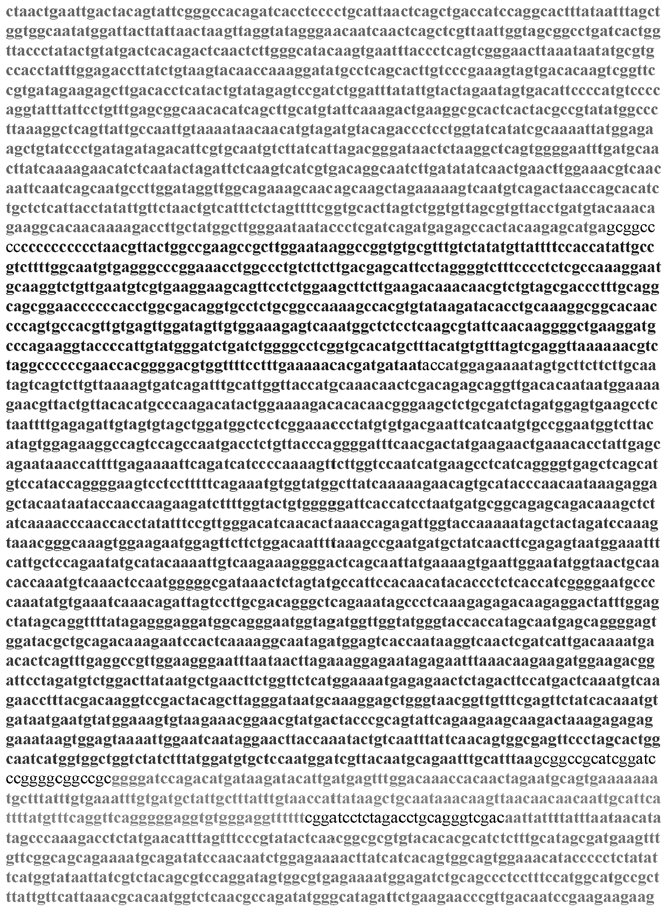

FIG. 37 shows a plasmid diagram showing the construction of the pFwt-IRES-COBRA-C plasmid through the replacement of the Saudi-Arabia H9 gene in a plasmid that was created previously (pFwt-IRES-SaH9opt) with COBRA-C gene extracted from pVP2-IRES-COBRA-C.

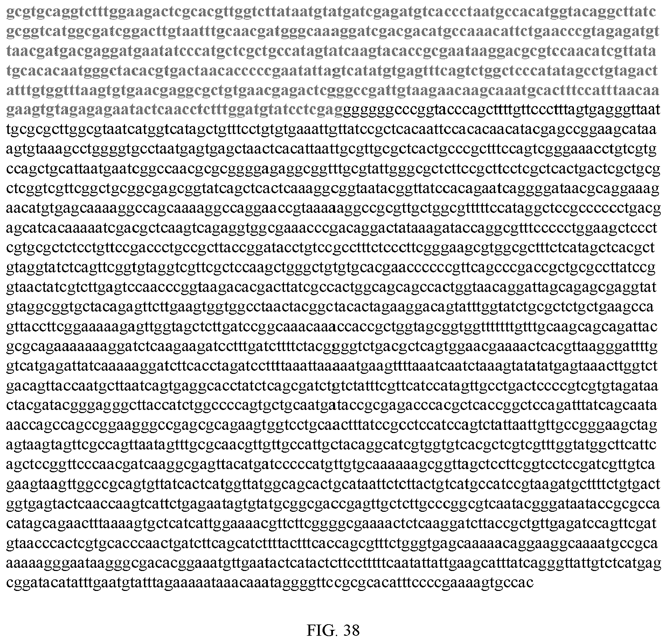

FIG. 38 shows the nucleotide sequence of the pFwt-IRES[MOD]-Cobra-C plasmid (SEQ ID NO: 52) where: Green = Intergene 1 Arms, Orange = SV40 Poly A, Dark Red = H5 HA COBRA-C, Blue = IRES, Red = NDV-F, Brown = mCMV promoter, Black = Vector.
FIG. 39 shows the amino acid sequence of NDV-F (SEQ ID NO: 53).
FIG. 40 shows the amino acid sequence of H5 HA COBRA-C (SEQ ID NO: 48).

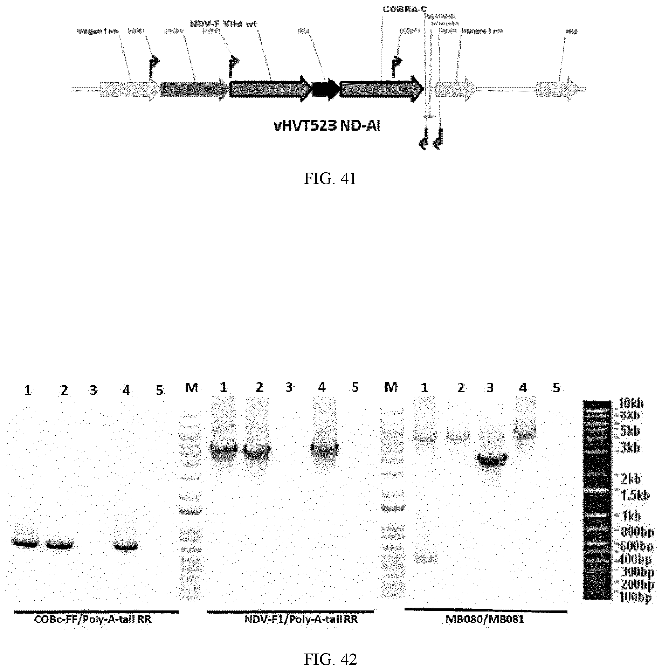

FIG. 41 is a diagram showing the PCR primer locations on vHVT523.
FIG. 42 is a picture of the agarose gel loaded with the PCR products using the following templates: vHVT523 pre-MSV (lane 1), vHVT523 pre-MSV+12 (lane 2), vHVT013 (lane 3), donor plasmid (pFwt-IRES[MOD]-Cobra-C (lane 4) and negative control (lane 5) and the different primer pairs indicated in Table 8.

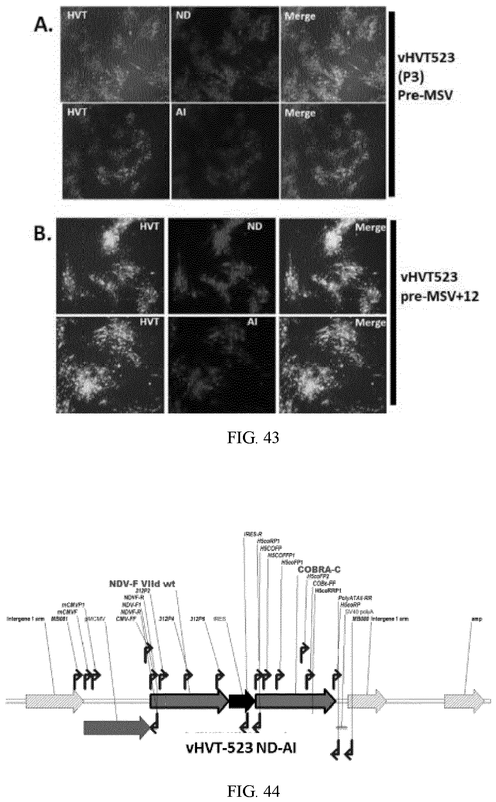

FIG. 43 shows results from a dual immunofluorescence assay, which shows a representative picture from the pre-MSV (P3) passage (A) and from the pre-MSV+12 passage (B).
FIG. 44 is a diagram showing sequencing primer locations on vHVT523.

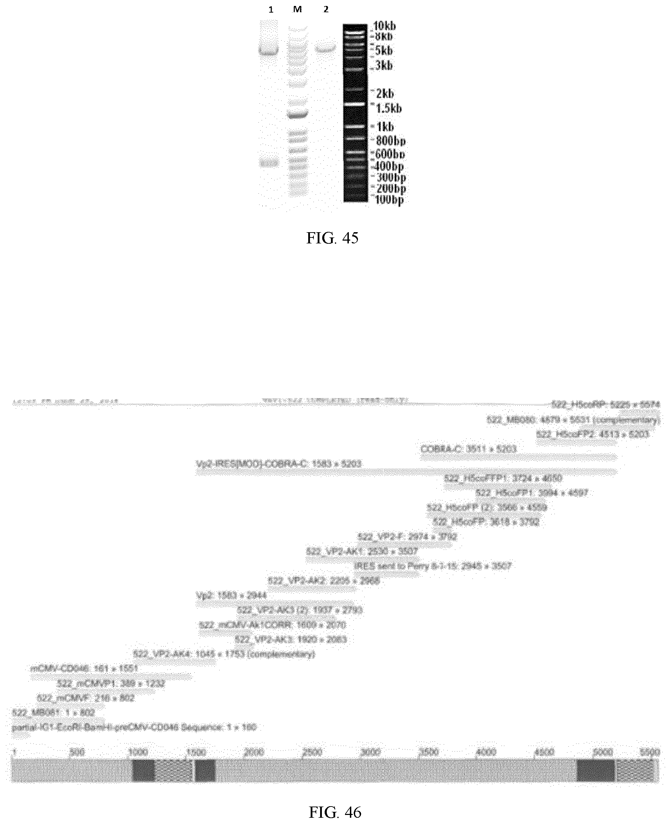

FIG. 45 shows vHVT523 pre-MSV (P3) and X+12 (pre-MSV+12) PCR for sequencing.

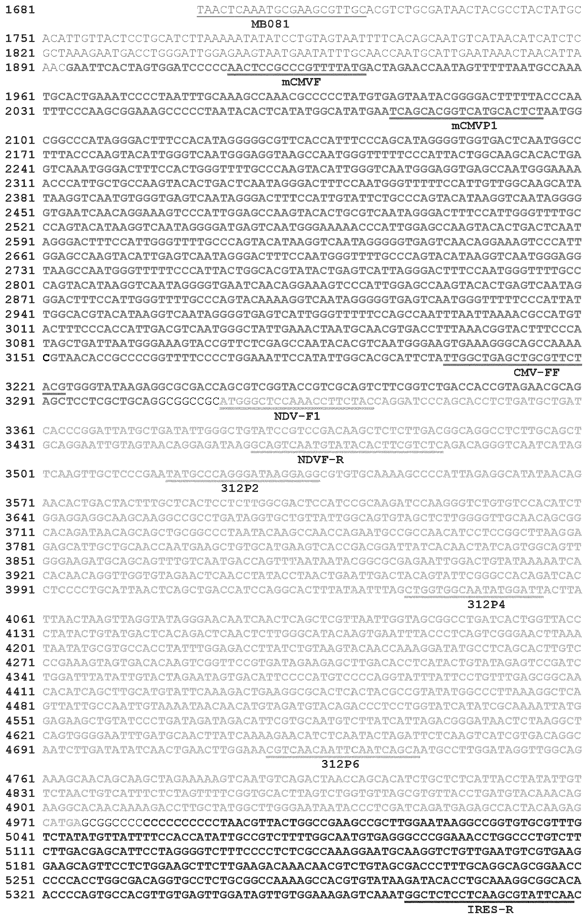

FIG. 46 shows the vHVT523 (P3) pre-MSV sequencing contig.

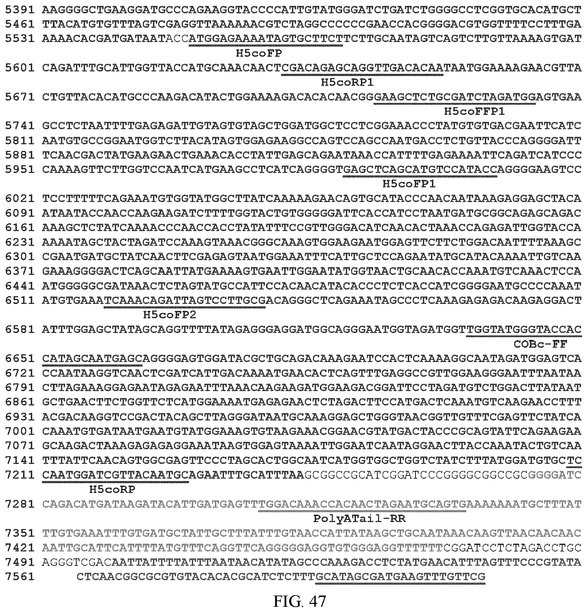

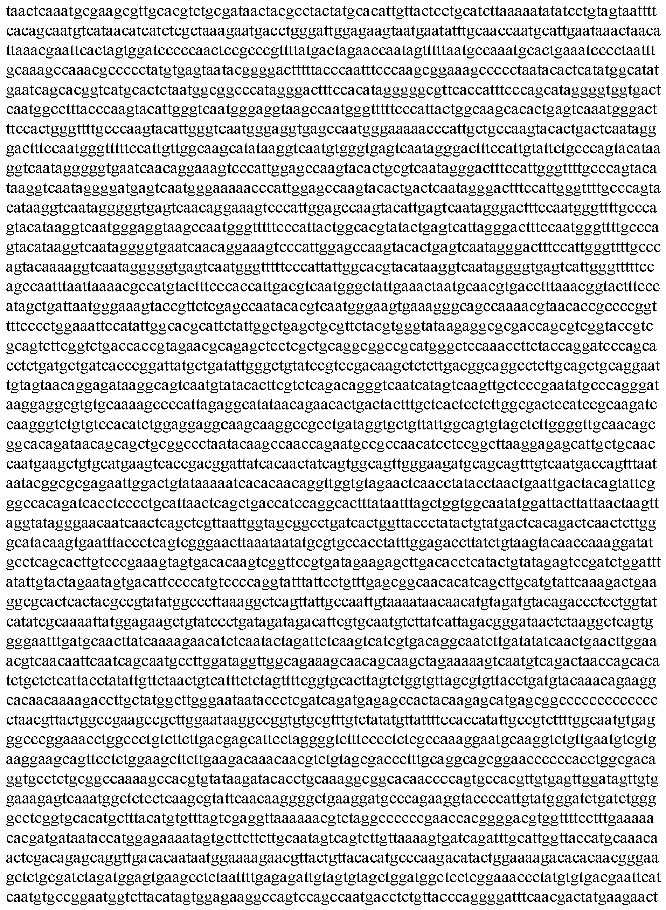

FIG. 47 shows the nucleotide sequence of vHVT523 (SEQ ID NO: 54) with primer locations where: Light Green = IG1 Arm, Blue = mCMV, Orange = NDV-F, Purple = IRES, Dark Red = H5 HA, Light Blue = SV40 poly A, Green = IG1 Arm, Underlined = Sequencing Primers.

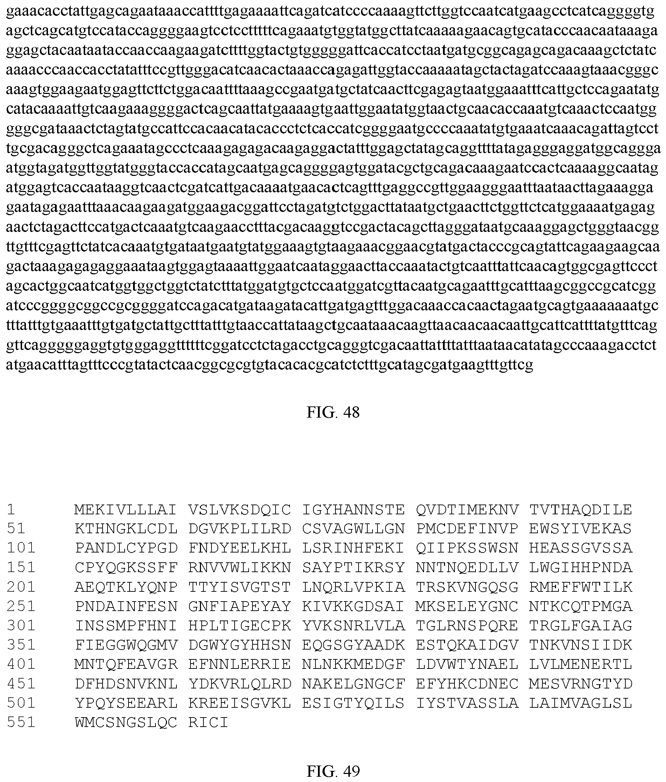

FIG. 48 shows the nucleotide sequence of the sequenced region of vHVT523 (SEQ ID NO: 54).
FIG. 49 shows the amino acid sequence of the AIV HA COBRA-C gene in vHVT523 (SEQ ID NO: 48).
FIG. 50 shows the amino acid sequence of the NDV-F gene in vHVT523 (SEQ ID NO: 53).

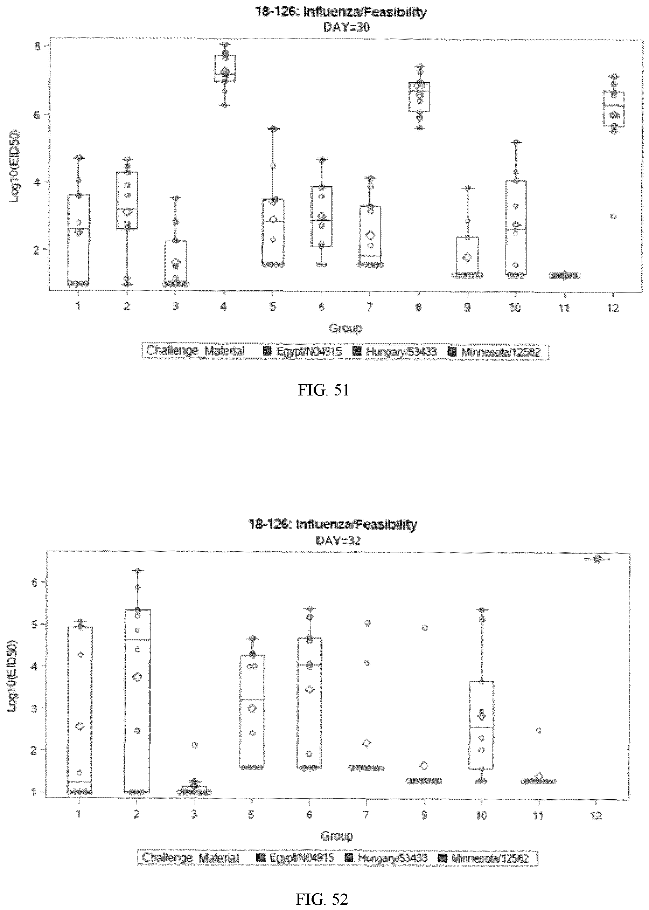

FIG. 51 shows a boxplot of the calculated shedding titers for study day 30.
FIG. 52 shows a boxplot of the calculated shedding titers for study day 32.

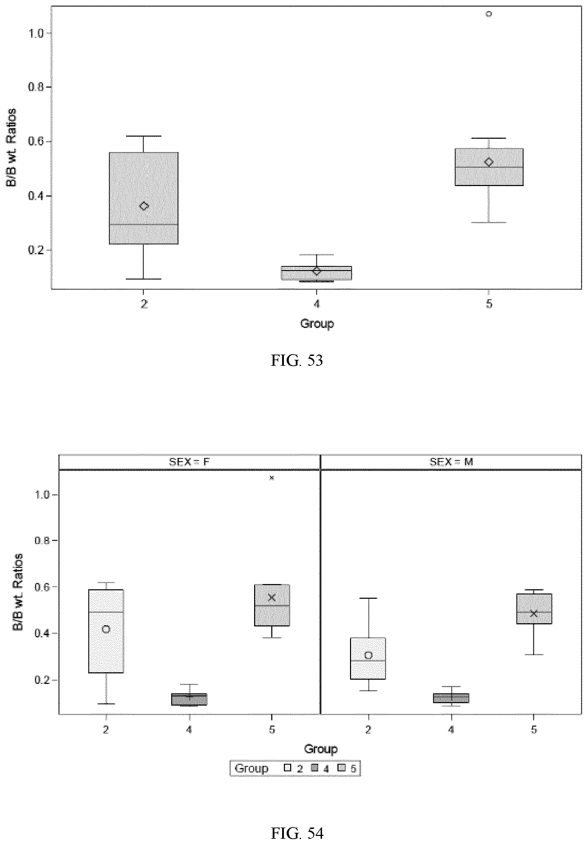

FIG. 53 shows a boxplot of the calculated bursal to body weight ratios by study group.
FIG. 54 shows a boxplot of the calculated bursal to body weight ratios by sex and study group.

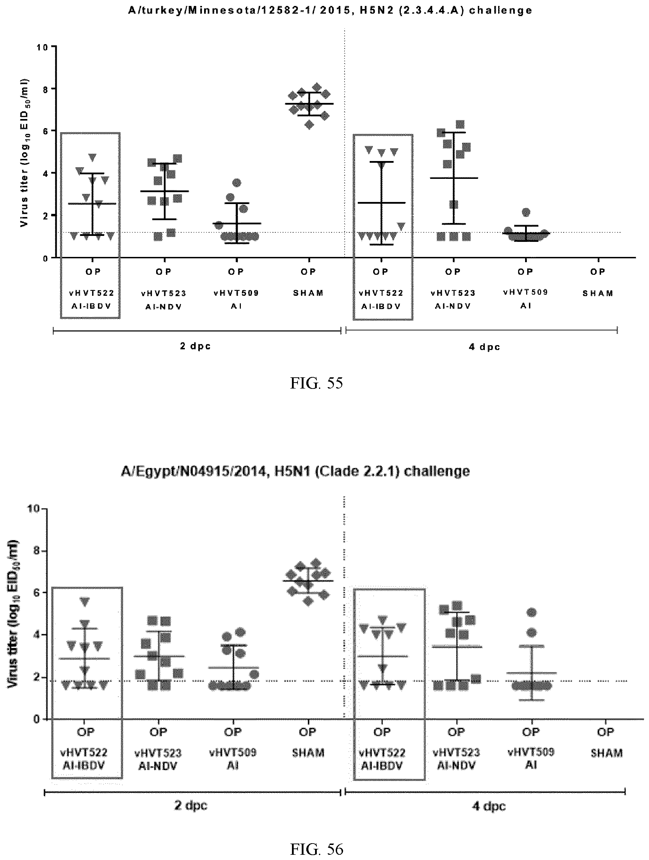

FIG. 55 is a graph showing results from virus shedding experiments for chickens administered vHVT509, vHVT522, and vHVT523 or a sham vaccine before challenge with Minnesota/12582-1. The results show measured shedding for two days post-challenge and four days post-challenge.
FIG. 56 is a graph showing results from virus shedding experiments for chickens administered vHVT509, vHVT522, and vHVT523 or a sham vaccine before challenge with Egypt/N04915. The results show measured shedding for two days post-challenge and four days post-challenge.

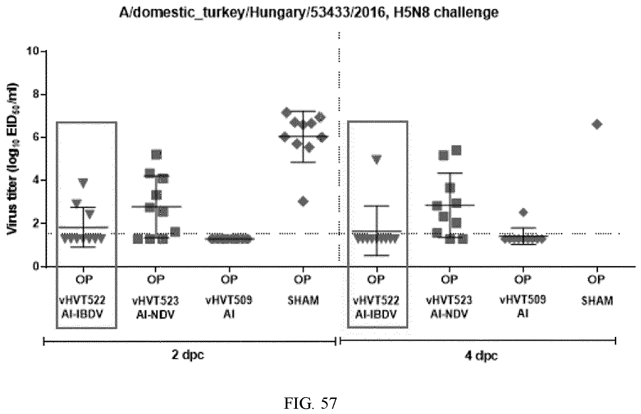

FIG. 57 is a graph showing results from virus shedding experiments for chickens administered vHVT509, vHVT522, and vHVT523 or a sham vaccine before challenge with Hungary/53433. The results show measured shedding for two days post-challenge and four days post-challenge.

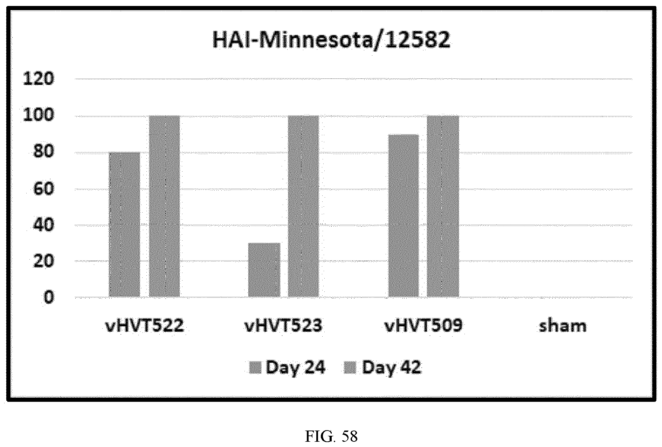

FIG. 58 is a bar graph showing results of hemagglutination inhibition assay performed on chickens vaccinated with one of the constructs vHVT509, vHVT522, and vHVT523 or a sham vaccine pre-challenge (day 24, shown in blue) and post-challenge (day 42, shown in orange). The chickens in this study were challenged with Minnesota/12582.

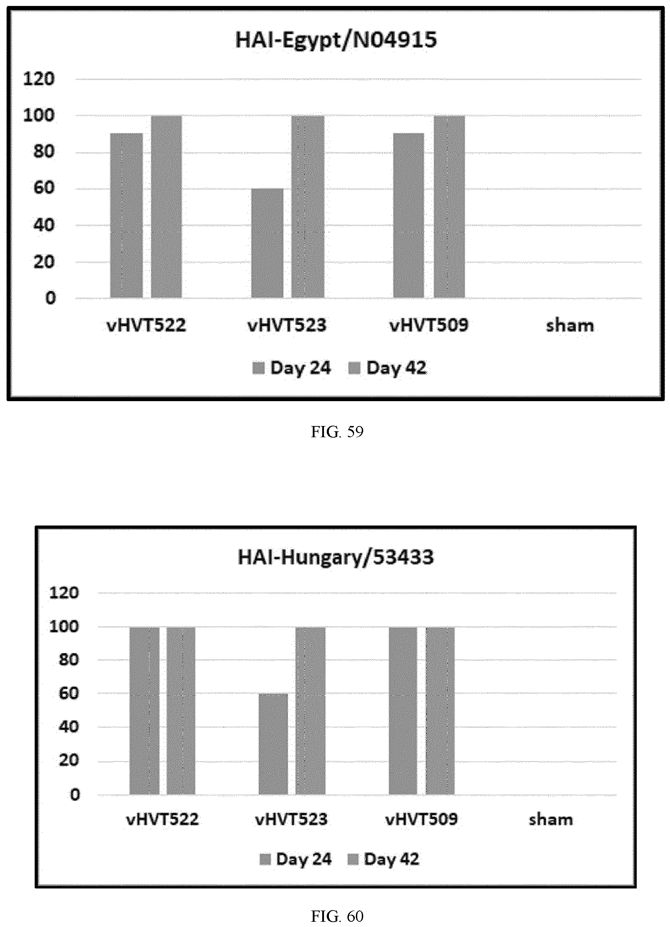

FIG. 59 is a bar graph showing results of hemagglutination inhibition assay performed on chickens vaccinated with one of the constructs vHVT509, vHVT522, and vHVT523 or a sham vaccine pre-challenge (day 24, shown in blue) and post-challenge (day 42, shown in orange). The chickens in this study were challenged with Egypt/N04915.
FIG. 60 is a bar graph showing results of hemagglutination inhibition assay performed on chickens vaccinated with one of the constructs vHVT509, vHVT522, and vHVT523 or a sham vaccine pre-challenge (day 24, shown in blue) and post-challenge (day 42, shown in orange). The chickens in this study were challenged with Hungary/53433.

### DETAILED DESCRIPTION OF THE INVENTION

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

Unless otherwise noted, technical terms are used according to conventional usage. Definitions of common terms in molecular biology may be found in Benjamin Lewin, Genes V. published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8). The singular terms "a," "an," and "the" include plural referents unless context clearly indicates otherwise. Similarly, the word "or" is intended to include "and" unless the context clearly indicate otherwise. The word "or" means any one member of a particular list and also includes any combination of members of that list.

The term "animal" is used herein to include all mammals, birds, and fish. The animal as used herein may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), bovine (e.g., cattle), porcine (e.g., pig), ovine (e.g., sheep, goats, llamas, bison), avian (e.g., chicken, duck, goose, turkey, quail, pheasant, parrot, finches, hawk, crow, ostrich, emu and cassowary), primate (e.g., prosimian, tarsier, monkey, gibbon, ape), humans, and fish. The term "animal" also includes an individual animal in all stages of development, including embryonic and fetal stages.

The term "avian" may refer to a chicken, capon, duck, goose, turkey, pheasant, grouse, quail, swan, squab, pigeon, parrot, finches, hawk, crow, ostrich, emu, and cassowary. In many embodiments, avian may be used to refer a chicken.

The terms "polypeptide" and "protein" are used interchangeably herein to refer to a polymer of consecutive amino acid residues.

The term "nucleic acid", "nucleotide", and "polynucleotide" are used interchangeably and refer to RNA, DNA, cDNA, or cRNA and derivatives thereof, such as those containing modified backbones. It should be appreciated that the invention provides polynucleotides comprising sequences complementary to those described herein. The "polynucleotide" contemplated in the present invention includes both the forward strand (5' to 3') and reverse complementary strand (3' to 5'). Polynucleotides according to the invention can be prepared in different ways (e.g. by chemical synthesis, by gene cloning etc.) and can take various forms (e.g. linear or branched, single or double stranded, or a hybrid thereof, primers, probes etc.).

The term "genomic DNA", or "genome" is used interchangeably and refers to the heritable genetic information of a host organism. The genomic DNA comprises the DNA of the nucleus (also referred to as chromosomal DNA) but also the DNA of the plastids (e.g., chloroplasts) and other cellular organelles (e.g., mitochondria). The genomic DNA or genome contemplated in the present invention also refers to the RNA of a virus. The RNA may be a positive strand or a negative strand RNA. The term "genomic DNA" contemplated in the present invention includes the genomic DNA containing sequences complementary to those described herein. The term "genomic DNA" also refers to messenger RNA (mRNA), complementary DNA (cDNA), and complementary RNA (cRNA).

The term "gene" is used broadly to refer to any segment of polynucleotide associated with a biological function. Thus, genes or polynucleotides include introns and exons as in genomic sequence, or just the coding sequences as in cDNAs, such as an open reading frame (ORF), starting from the start codon (methionine codon) and ending with a termination signal (stop codon). Genes and polynucleotides can also include regions that regulate their expression, such as transcription initiation, translation, and transcription termination. Thus, also included are promoters and ribosome binding regions (in general these regulatory elements lie approximately between 60 and 250 nucleotides upstream of the start codon of the coding sequence or gene; Doree S M et al; Pandher K et al; Chung J Y et al), transcription terminators (in general the terminator is located within approximately 50 nucleotides downstream of the stop codon of the coding sequence or gene; Ward C K et al). Gene or polynucleotide also refers to a nucleic acid fragment that expresses mRNA or functional RNA, or encodes a specific protein, and which includes regulatory sequences.

The term "heterologous DNA" as used herein refers to the DNA derived from a different organism, such as a different cell type or a different species from the recipient. The term also refers to a DNA or fragment thereof on the same genome of the host DNA wherein the heterologous DNA is inserted into a region of the genome which is different from its original location.

A "dosing regimen" may refer to a schedule that outlines both an amount of immunogenic composition, times for administration, and/or routes of administration when treating avians with an immunogenic composition that is developed to induce a desired effect in a given predetermined population and/or individual animals, such as reducing incidence of a disease in a given population, for example, vaccinated avians, reducing severity of or inhibiting symptoms of a targeted disease, and/or inducing an immune response in an animal and/or their progeny.

An "immune response" refers to the response induced by a vaccine or an exposure to a virus. Immune response may be measured as, but is not limited to a humoral immune response, for example, virus neutralizing antibodies, a cellular immune responses, for example, enhancement of CD4+ and/or CD8+ T cell responses, serological response, such as ELISA and/or hemagglutination inhibition test, and/or mitigation and/or reduction of clinical symptoms and/or measurable markers of the disease targeted by the immunogenic composition administered.

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, an epitope, a hapten, or any combination thereof. Alternately, the immunogen or antigen may comprise a toxin or antitoxin.

The term "immunogenic protein or peptide" as used herein includes polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the invention comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871. Similarly, conformational epitopes are readily identified by determining spatial conformation of amino acids such as by, e.g., x-ray crystallography and 2-dimensional nuclear magnetic resonance. See, e.g., Epitope Mapping Protocols, supra.

The term "immunogenic protein or peptide" further contemplates deletions, additions, and substitutions to the sequence, so long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic-aspartate and glutamate; (2) basic- lysine, arginine, histidine; (3) non-polar- alanine, valine, leucine, isoleucine, proline, phenylalanine, methionine, tryptophan; and (4) uncharged polar-glycine, asparagine, glutamine, cysteine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a maj or effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

An "immunological composition" as used herein refers to any composition that is capable of inducing an immune response in an animal.

The term "immune stimulant" as used herein, means any agent or composition that can trigger the immune response, preferably without initiating or increasing a specific immune response, for example the immune response against a specific pathogen. It is further instructed to administer the immune stimulant in a suitable dose.

The terms "recombinant" and "genetically modified" are used interchangeably and refer to any modification, alteration or engineering of a polynucleotide or protein in its native form or structure, or any modification, alteration or engineering of a polynucleotide or protein in its native environment or surrounding. The modification, alteration or engineering of a polynucleotide or protein may include, but is not limited to, deletion of one or more nucleotides or amino acids, deletion of an entire gene, codon- optimization of a gene, conservative substitution of amino acids, insertion of one or more heterologous polynucleotides.

The terms "polyvalent vaccine or composition", "combination or combo vaccine or composition" and "multivalent vaccine or composition" are used interchangeably to refer to a composition or vaccine containing more than one composition or vaccines. The polyvalent vaccine or composition may contain two, three, four or more compositions or vaccines. The polyvalent vaccine or composition may comprise recombinant viral vectors, active or attenuated or killed wild-type viruses, or a mixture of recombinant viral vectors and wild-type viruses in active or attenuated or killed forms.

"Sequence Identity" as it is known in the art refers to a relationship between two or more polypeptide sequences or two or more polynucleotide sequences, namely a reference sequence and a given sequence to be compared with the reference sequence. Sequence identity is determined by comparing the given sequence to the reference sequence after the sequences have been optimally aligned to produce the highest degree of sequence similarity, as determined by the match between strings of such sequences. Upon such alignment, sequence identity is ascertained on a position-by-position basis, e.g., the sequences are "identical" at a particular position if at that position, the nucleotides or amino acid residues are identical. The total number of such position identities is then divided by the total number of nucleotides or residues in the reference sequence to give % sequence identity. Sequence identity can be readily calculated by known methods, including but not limited to, those described in Computational Molecular Biology, Lesk, A. N., ed., Oxford University Press, New York (1988), Biocomputing: Informatics and Genome Projects, Smith, D. W., ed., Academic Press, New York (1993); Computer Analysis of Sequence Data, Part I, Griffin, A. M., and Griffin, H. G., eds., Humana Press, New Jersey (1994); Sequence Analysis in Molecular Biology, von Heinge, G., Academic Press (1987); Sequence Analysis Primer, Gribskov, M. and Devereux, J., eds., M. Stockton Press, New York (1991); and Carillo, H., and Lipman, D., SIAM J. Applied Math., 48: 1073 (1988), the teachings of which are incorporated herein by reference. Preferred methods to determine the sequence identity are designed to give the largest match between the sequences tested. Methods to determine sequence identity are codified in publicly available computer programs which determine sequence identity between given sequences. Examples of such programs include, but are not limited to, the GCG program package (Devereux, J., et al., Nucleic Acids Research, 12(1):387 (1984)), BLASTP, BLASTN and FAS TA (Altschul, S. F. et al., J. Molec. Biol., 215:403-410 (1990). The BLASTX program is publicly available from NCBI and other sources (BLAST Manual, Altschul, S. et al., NCVI NLM NIH Bethesda, Md. 20894, Altschul, S. F. et al., J. Molec. Biol., 215:403-410 (1990), the teachings of which are incorporated herein by reference). These programs optimally align sequences using default gap weights in order to produce the highest level of sequence identity between the given and reference sequences. As an illustration, by a polynucleotide having a nucleotide sequence having at least, for example, 80%, preferably 85%, even more preferably 90%, most preferably 95% "sequence identity" to a reference nucleotide sequence, it is intended that the nucleotide sequence of the given polynucleotide is identical to the reference sequence except that the given polynucleotide sequence may include up to 20, preferably up to 15, preferably up to 10, even more preferably up to 5 point mutations per each 100 nucleotides of the reference nucleotide sequence. In other words, in a polynucleotide having a nucleotide sequence having at least 80%, preferably 85%, even more preferably 90%, most preferably 95% identity relative to the reference nucleotide sequence, up to 20%, preferably up to 15%, even more preferably 10%, most preferably 5% of the nucleotides in the reference sequence may be deleted or substituted with another nucleotide, or a number of nucleotides up to 20%, preferably up to 15%, even more preferably 10%, most preferably 5% of the total nucleotides in the reference sequence may be inserted into the reference sequence. These mutations of the reference sequence may occur at the 5 or 3' terminal positions of the reference nucleotide sequence or anywhere between those terminal positions, interspersed either individually among nucleotides in the reference sequence or in one or more contiguous groups within the reference sequence. Analogously, by a polypeptide having a given amino acid sequence having at least, for example, 80%, preferably 85%, even more preferably 90%, most preferably 95% sequence identity to a reference amino acid sequence, it is intended that the given amino acid sequence of the polypeptide is identical to the reference sequence except that the given polypeptide sequence may include up to 20%, preferably up to 15%, even more preferably 10%, most preferably 5% amino acid alterations per each 100 amino acids of the reference amino acid sequence. In other words, to obtain a given polypeptide sequence having at least 80%, preferably 85%, even more preferably 90%, most preferably 95% sequence identity with a reference amino acid sequence, up to 20%, preferably up to 15%, even more preferably 10%, most preferably 5% of the amino acid residues in the reference sequence may be deleted or substituted with another amino acid, or a number of amino acids up to 20%, preferably up to 15%, even more preferably 10%, most preferably 5% of the total number of amino acid residues in the reference sequence may be inserted into the reference sequence. These alterations of the reference sequence may occur at the amino or the carboxy terminal positions of the reference amino acid sequence or anywhere between those terminal positions, interspersed either individually among residues in the reference sequence or in the one or more contiguous groups within the reference sequence. Preferably, residue positions which are not identical differ by conservative amino acid substitutions. However, conservative substitutions are not included as a match when determining sequence identity.

"Sequence homology", as used herein, refers to a method of determining the relatedness of two sequences. To determine sequence homology, two or more sequences are optimally aligned, and gaps are introduced if necessary. However, in contrast to "sequence identity", conservative amino acid substitutions are counted as a match when determining sequence homology. In other words, to obtain a polypeptide or polynucleotide having 95% sequence homology with a reference sequence, 80%, preferably 85%, even more preferably 90%, most preferably 95% of the amino acid residues or nucleotides in the reference sequence must match or comprise a conservative substitution with another amino acid or nucleotide, or a number of amino acids or nucleotides up to 20%, preferably up to 15%, even more preferably 10%, most preferably 5% of the total amino acid residues or nucleotides, not including conservative substitutions, in the reference sequence may be inserted into the reference sequence. Preferably the homolog sequence comprises at least a stretch of 50, even more preferably at least 100, even more preferably at least 250, and even more preferably at least 500 nucleotides.

As used herein, the term "homologs" includes orthologs, analogs and paralogs. The term "analogs" refers to two polynucleotides or polypeptides that have the same or similar function, but that have evolved separately in unrelated organisms. The term "orthologs" refers to two polynucleotides or polypeptides from different species, but that have evolved from a common ancestral gene by speciation. Normally, orthologs encode polypeptides having the same or similar functions. The term "paralogs" refers to two polynucleotides or polypeptides that are related by duplication within a genome. Paralogs usually have different functions, but these functions may be related. Analogs, orthologs, and paralogs of a wild-type polypeptide can differ from the wild-type polypeptide by post-translational modifications, by amino acid sequence differences, or by both. In particular, homologs of the invention will generally exhibit at least 80-85 percent, 85-90 percent, 90-95 percent, or 95 percent, 96 percent, 97 percent, 98 percent, 99 percent sequence identity, with all or part of the polynucleotide or polypeptide sequences of antigen described above and will exhibit a similar function.

A "conservative substitution" refers to the substitution of an amino acid residue or nucleotide with another amino acid residue or nucleotide having similar characteristics or properties including size, hydrophobicity, etc., such that the overall functionality does not change significantly.

"Isolated" means altered "by the hand of man" from its natural state, i.e., if it occurs in nature, it has been changed or removed from its original environment, or both. For example, a polynucleotide or polypeptide naturally present in a living organism is not "isolated," but the same polynucleotide or polypeptide separated from the coexisting materials of its natural state is "isolated", as the term is employed herein.

Variants include allelic variants. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1- 5 percent variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest, are intended to be within the scope of the invention.

The term "identity" with respect to sequences can refer to, for example, the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman). The sequence identity or sequence similarity of two amino acid sequences, or the sequence identity between two nucleotide sequences can be determined using Vector NTI software package (Invitrogen, 1600 Faraday Ave., Carlsbad, CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

The polynucleotides of the disclosure include sequences that are degenerate as a result of the genetic code, e.g., optimized codon usage for a specific host. As used herein, "optimized" refers to a polynucleotide that is genetically engineered to increase its expression in a given species. To provide optimized polynucleotides coding for polypeptides, the DNA sequence of the protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of protein in said species can be achieved by utilizing the distribution frequency of codon usage in eukaryotes and prokaryotes, or in a particular species.

The term "frequency of preferred codon usage" refers to the preference exhibited by a specific host cell in usage of nucleotide codons to specify a given amino acid. There are 20 natural amino acids, most of which are specified by more than one codon. Therefore, all degenerate nucleotide sequences are included in the disclosure as long as the amino acid sequence of the polypeptide encoded by the nucleotide sequence is functionally unchanged.

As used herein, "a veterinary-acceptable carrier" includes any and all solvents, dispersion media, coatings, adjuvants, stabilizing agents, diluents, preservatives, antibacterial and antifungal agents, isotonic agents, adsorption delaying agents, and the like.

Those of skill in the art will understand that the composition used herein may incorporate known injectable, physiologically acceptable sterile solutions. For preparing a ready-to-use solution for parenteral injection or infusion, aqueous isotonic solutions, such as e.g. saline or corresponding plasma protein solutions, are readily available. In addition, the immunogenic and vaccine compositions of the present disclosure can include diluents, isotonic agents, stabilizers, or adjuvants. Diluents can include water, saline, dextrose, ethanol, glycerol, and the like. Isotonic agents can include sodium chloride, dextrose, mannitol, sorbitol, and lactose, among others. Stabilizers include albumin and alkali salts of ethylenediaminetetraacetic acid, among others.

"Adjuvants" as used herein, can include aluminum hydroxide and aluminum phosphate, saponins e.g., Quil A, QS-21 (Cambridge Biotech Inc., Cambridge Mass.), GPI-0100 (Galenica Pharmaceuticals, Inc., Birmingham, Ala.), water-in-oil emulsion, oil-in-water emulsion, water-in-oil-in-water emulsion. The emulsion can be based in particular on light liquid paraffin oil (European Pharmacopea type); isoprenoid oil such as squalane or squalene oil resulting from the oligomerization of alkenes, in particular of isobutene or decene; esters of acids or of alcohols containing a linear alkyl group, more particularly plant oils, ethyl oleate, propylene glycol di-(caprylate/caprate), glyceryl tri-(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylene-polyoxyethylene copolymer blocks, in particular the Pluronic products, especially L121. See Hunter et al., The Theory and Practical Application of Adjuvants (Ed.Stewart-Tull, D. E. S.). JohnWiley and Sons, NY, pp 51-94 (1995) and Todd et al., Vaccine 15:564-570 (1997).

Immunogenic compositions are described herein. In particular, the present invention relates to the field of veterinary vaccines, namely to vaccines based on recombinant viral vectors. In particular, described herein are immunogenic compositions that include recombinant viral vectors for use in avians against one or more diseases including, but not limited to Avian Influenza, Marek's Disease, Newcastle Disease, and Infectious Bursal Disease.

Diseases to be targeted by the immunogenic composition disclose herein include Marek's disease (MD) which is a highly infectious avian disease. Clinical symptoms of MD include, but are not limited to loss of weight, paralysis, for example of legs or wings, tumors, such as tumors in the heart, ovaries, testes, muscles, lungs, feather follicles etc., eye lesions, irregular pupils, and mortality. MD is associated with the presence of T-cell lymphomas in several organs and nerves. In some instances, maternally derived antibodies (MDA) from immune mothers may provide protection to newly born avians.

As described the present invention provides recombinant vectors that include and express antigens of avian pathogens. In addition, the recombinant vectors may be used in immunogenic compositions, for example, vaccines that include the recombinant vectors. For example, an immunogenic composition or vaccine may include recombinant vectors that include heterologous polynucleotides coding for and expressing at least one antigen of an avian pathogen. In addition, methods of treating avians, for example, methods of vaccination against a variety of avian pathogens and method of producing the recombinant vectors are described.

Vectors may include, but are not limited to viruses, for example, live viruses, inactivated viruses, such as a killed vaccine, recombinant viruses and/or bacteria. Immunogenic compositions, as described herein, may include one or more vectors.

Vaccine vectors may be constructed from a virus. In some instances, a virus for use in a recombinant vector for use in an immunogenic composition may be predetermined based on properties of the virus. For example, a predetermined virus for use in a recombinant vector may include viruses including but not limited to herpesvirus from turkeys (HVT), Marek's disease virus such as Marek's disease virus serotype 3 (MDV-3), MDV-1, MDV-2, and/or a Meleagrid herpesvirus such as Meleagrid herpesvirus serotype 1 (MeHV-1).

For example, administration of an MDV-3 (HVT) vector vaccine to poultry may generate an immune response against an expressed heterologous gene. In some instances, an MDV-3 vector vaccine may be used to express and/or deliver a therapeutic protein, e.g. a cytokine, to manipulate an animal's (e.g., an avian) immune response.

An MDV-3 strain such as FC-126 may be used to construct the viral vectors. The genomic nucleotide sequence of HVT (MDV-3) is available, for example from GenBank^{™} as: AF291866 (strain FC-126).

Genetic locations for the insertion of a heterologous gene-construct into the MDV-3 (i.e., HVT) may include, but may not limited to non-essential loci. For example, insertion may occur at a position in the unique short (Us) region of the MDV-3 genome, in the HVT unique long (UL) region of the MDV-3 genome.

Different promoters may be used to drive expression of a heterologous gene including, but not limited to a PRV gpX promoter, a Rous sarcoma virus L TR promoter, an SV40 early gene promoter, a chicken beta-actin gene promoter, and/or an immediate early 1 gene promoter from human (hCMV IE1), an mCMV promoter, or murine (mCMV IE1) cytomegalovirus. In any given viral vector, one or more promoters may be used to drive expression of the included gene sequences.

For the construction of recombinant vectors, a heterologous nucleic acid that is to be inserted into the vector's genome, usually comprises at least one heterologous gene or coding region, which encodes (at least an immunogenic part of) an antigen. In some instances, the construct may comprise a promoter sequence to drive the expression of the heterologous gene, and regulatory signals such as an enhancer, or a transcription terminator. Such a combined insert is often termed an 'expression cassette'.

The effect of the insertion of an expression cassette into a vector's genome differs, depending on the location and on the way it is inserted: the vector genome may become larger, the same, or smaller in size, depending from whether the net result on the genome is an addition, replacement or deletion of genetic material, respectively. Also, the location of the insertion may have an effect: placed inside a coding-, a non-coding, or a regulatory region of the genome. Among others, these choices influence the characteristics of the resulting vector vaccine, in terms of its ability for replication and expression, and its genetic stability.

In some instances, viral vectors may be constructed by including one or more exogenous sequence inserts in MDV-1, MDV-2, and/or MDV-3 using a donor plasmid. Further, some immunogenic compositions may include two or more viral constructs that include exogenous sequences. For example, various combinations of viral vectors formed from MDV-1, MDV-2, and/or MDV-3 may each include one or more exogenous sequences against diseases to form an immunogenic composition. Further, multiple viral vectors may be combined in an immunogenic composition to provide protection against 3 or more diseases.

As shown in FIG. 1, some embodiments of the vaccine vectors described herein use MDV-3 strain FC-126 as the parental virus (e.g., Ross, LJ et al., J. Gen. Virol. 70, 1789-1804, 1989).

Depending on the parental virus the construction of the donor plasmid may vary. Donor plasmids may include, but are not limited to, including a promoter such as a mCMV promoter, a gene of interest for the target disease, and/or a transcription stop such as a polyA. For example, in an embodiment that targets AIV, a selected donor plasmid may include an mCMV promoter, AIV HA COBRA-C, and/or SV40 polyA tail. Such a donor plasmid may be designated as pCD046-COBRA-C and modify MDV-3 strain FC-126 to form vHVT509 as depicted in FIG. 1.

A nucleotide sequence encoding a donor plasmid used to construct the viral vector that includes antigens for AIV may have at least 80 percent, 85 percent, 90 percent, 95 percent, 96 percent, 97 percent, 98 percent, or 99 percent sequence identity to a sequence of the plasmid as shown in FIG. 16. In particular, a nucleotide sequence encoding the donor plasmid used to construct the viral vector that includes antigens for AIV and IBDV may have greater than 90 percent sequence identity to a sequence of the plasmid shown in FIG. 16. For example, a nucleotide sequence encoding the donor plasmid used to construct the viral vector that includes antigens for AIV and IBDV may have greater than 95 percent sequence identity to a sequence of the plasmid shown in FIG. 16.

In some embodiments, MDV-3 strain FC-126 may be modified to include a sequence encoding the VP2 protein from the infectious bursal disease (IBDV) to form the virus known as vHVT013 or Vaxxitek (EPAR-Scientific Discussion at www.ema.europa.eu/en/documents/scientific-discussion/vaxxitek-hvtibd-epar-scientific-discussion_en.pdf; www.biodic.gojp/bch/download/lmo/H28.9.23_doubutsuiyaku_sp.pdf). For instance, the starting viral vector used to construct the modified viral vector described herein (which is a virus vaccine strain) may be HVT013 or vHVT013 or the active ingredient in VAXXITEK(R) (also sometimes called vHVT013-069).

In particular, vHVT13 (or HVT013-69) is an active ingredient of Merial's (Boehringer Ingelheim Animal Health's) VAXXITEK(R) (HVT+IBD) Vaccine. In U.S. Pat. No. 5,980,906, the HVT+IBD construct commonly known as vHVT013 or vHVT013-69 or HVT013 or the active ingredient in VAXXITEK(R) is called vHVT17. The vHVT13 (or vHVT013-69 or HVT013) vector includes an expression cassette comprising an mCMV IE promoter (SEQ ID NO:6), an IBDV VP2 gene (SEQ ID NO: 1 encoding SEQ ID NO:2), and a SV40 poly A tail (SEQ ID NO:8) inserted into the IG1 insertion site.

This recombinant vaccine strain, vHVT013 (an HVT vector expressing the IBDV VP2 gene), has been constructed following a protocol which included targeting a region of insertion through homologous recombination between the DNA of the parental FC-126 strain of HVT and a plasmid that included the gene of interest surrounded by homology arms. The resulting recombinant may be further modified to create viral vectors, vHVT522 and/or vHVT523.

In some embodiments, vHVT013 may be modified by adding a sequence encoding a protein. In particular, vHVT013 may be modified to include a sequence encoding an antigen and/or protein of a disease. For example, vHVT013 may be modified to include a sequence encoding an antigen and/or protein of avian influenza. As shown in FIG. 1, vHVT013 may be modified using a donor plasmid to include a sequence encoding an avian influenza antigen as was formed in Cobra-C H5 HA to form viral vector vHVT522.

For example, vHVT013 may be modified using a donor plasmid to include a sequence encoding an avian influenza antigen. As shown in FIG. 1, vHVT013 may be modified using a plasmid that includes a sequence encoding for AIV antigen, such as Cobra-C H5 HA to form viral vectors such as vHVT522 and/or vHVT523. In particular, pFwt-IRES[MOD]-Cobra-C, pVP2-IRES[MOD]-Cobra-C, as well as other plasmids incorporating different combinations of exogenous sequences may be used.

In some embodiments, vHVT522 may include a gene encoding a synthetic hemagglutinin (HA) protein of Avian Influenza virus A (subtype H5) designed by computationally optimized broadly reactive approaches (COBRA). A synthetic Infectious Bursal Disease Virus structural capsid protein VP2.

A recombinant viral vector may be formed using an insertion plasmid for the Intergenic I (IG1) site that may include a SV40 poly A tail, COBRA-C (H5 HA protein), Internal Ribosome Entry Site (IRES), and Infectious Bursal Disease Virus capsid protein (IBDV VP2). This plasmid may be inserted using homologous recombination into Vaxxitek virus right IG1 Arm and IBDV VP2.

In some embodiments, the donor plasmid may be pVP2-IRES [MOD]-COBRA-C, which may be a synthetically ordered plasmid that includes the right Intergenic I arm of Vaxxitek virus, SV40 poly A tail, COBRA-C (H5 HA protein), IRES, and Infectious Bursal Disease Virus capsid protein. The plasmid may include a mCMV promoter from the parent virus. In particular, an embodiment of vHVT522 may include the mCMV promoter from the starting point, mHVT013 virus as shown in FIG. 1.

Recombination may occur *in vitro* in secondary chicken embryo fibroblast (2° CEF) cells. Recombinant selection may be accomplished via indirect immunofluorescence assay (IFA) using chicken anti-Avian influenza sera and PCR amplification with specific primers. Plasmid diagrams are shown in FIG. 14 and FIG. 15.

In some embodiments, plasmid pVP2-IRES-Cobra-C may be created by cloning a synthetic COBR-C fragment (Genscript Lot U5872BA260-3) into pVP2-IRES-Cobra-A. Further, the pVP2-IRES-Cobra-C plasmid may be modified by replacing the HindIII-BlpI fragment by a new synthetic fragment VP2+COBC to remove an intervening stretch of sequence between the end of IRES sequence and start of COBRA-C sequence present in pVP2-IRES-Cobra-C. The resulting donor plasmid is called pVP2-IRES [MOD]-Cobra-C. Donor plasmid, pVP2-IRES[MOD]-Cobra-C, may be used to generate recombinant virus.

A nucleotide sequence encoding a donor plasmid used to construct the viral vector that includes antigens for AIV and IBDV may have at least 80 percent, 85 percent, 90 percent, 95 percent, 96 percent, 97 percent, 98 percent, or 99 percent sequence identity to a sequence of the plasmid shown in FIG. 16. In particular, a nucleotide sequence encoding the donor plasmid used to construct the viral vector that includes antigens for AIV and IBDV may have greater than 90 percent sequence identity to a sequence of the plasmid shown in FIG. 16. For example, a nucleotide sequence encoding the donor plasmid used to construct the viral vector that includes antigens for AIV and IBDV may have greater than 95 percent sequence identity to a sequence of the plasmid shown in FIG. 16.

*In vitro* recombination (IVR) may be performed by co-electroporation of 2° CEF cells using a linearized pVP2-IRES[MOD]-COBRA-C as the donor plasmid and viral DNA isolated from vHVT013. Co-electroporation may be performed using 1×10⁷ 2° CEF in 300 µl Opti-MEM and may be shocked at 150 volts with 950 capacitance in a 2mm electroporation cuvette. The transfected cells may then be seeded into 96-well plate and incubated for 4 days. The cells grown in the 96-well plate may then be duplicated into two 96-well plates. One set of 96-well plates may be used for IFA using chicken polyclonal sera against AIV H5 HA to identify positive wells containing recombinants and another set of 96-well plates may be used for recovering the infected cells from the corresponding positive wells.

Recombinant viral purification methods may be performed first by 96-well plate duplication and IFA selection for the wells containing the most IFA positive plaques with the least amount of IFA negative plaques. Wells matching those criteria may then be harvested and adjusted to 1 mL in DMEM+2% FBS. From the 1 mL stock, 10-20 µl may be removed and mixed with 1×10⁷ 2° CEFs in 10 mL DMEM+2% FBS and may then aliquoted onto a new 96-well plate in an attempt to have single HVT plaques per well. The supernatant of the wells that contains single plaques may be tested for the absence of parental virus by PCR. This process may be repeated for few rounds until all tested wells showed pure recombinant banding patterns and no parental virus.

In an embodiment vHVT013 may be modified to encode two or more exogenous sequences. For example, an embodiment may include vHVT013 modified with sequences encoding a Newcastle Disease (NDV) protein, such as NDV-F protein and a further sequence encoding an avian influenza protein such as Cobra-C H5 HA as is depicted in FIG. 1.

In particular, vHVT523 may include a gene encoding a synthetic hemagglutinin (HA) protein of Avian influenza virus (AIV) A (subtype H5) designed by computationally optimized broadly reactive approaches (COBRA). This particular protein is also called in this document as COBRA-C for **C**omputationally **O**ptimized **B**roadly **R**eactive **A**ntigen-version **C**. Further, vHVT523 may include a synthetic Newcastle Disease Virus (NDV) fusion protein (F).

A vHVT523 construct formed from a recombinant HVT virus may include an Intergenic I site in the BamHI I fragment that may include an mCMV promoter, NDV fusion protein F, Internal Ribosome Entry Site (IRES), HA protein of Avian Influenza virus (Subtype H5, COBRA-C) and an SV40 poly A tail.

The donor plasmid may be pFwt-IRES[MOD]-COBRA-C, whose backbone includes a plasmid that includes synthetic DNA fragment from the Intergenic I arm of vHVT013 virus, SV40 poly A tail, COBRA-C (H5 HA protein), IRES, NDV-F protein and mCMV promoter. Recombination may occur *in vitro* in secondary chicken embryo fibroblast (2° CEF) cells. Recombinant selection may be accomplished via indirect immunofluorescent assay (IFA) using chicken anti-Avian influenza sera and PCR amplification with specific primers.

In some embodiments, the plasmid pFwt-IRES-COBRA-C may be constructed by replacing the Saudi-Arabia H9 gene in a plasmid that was created previously (pFwt-IRES-SaH9opt) with COBRA-C gene extracted from pVP2-IRES-COBRA-C. The COBRA-C gene may be extracted from pVP2-IRES-COBRA-C by using the restriction enzymes using SalI and HindIII and cloned into pFwt-IRES-SaH9opt that was also digested with same enzymes. The pFwt-IRES-Cobra-C plasmid was further modified by replacing the HindIII-BlpI fragment by a new synthetic fragment F+COBC to remove an intervening stretch of sequence between the end of IRES sequence and start of COBRA-C sequence present in pFwt-IRES-Cobra-C. The new and correct donor plasmid is called pFwt-IRES[MOD]-Cobra-C. This plasmid may be used to generate recombinant virus. Diagrams of an embodiment of the plasmid construction are shown in FIG. 36 and FIG. 37.

A nucleotide sequence encoding the donor plasmid used to construct the viral vector that includes antigens for AIV and NDV may have at least 80 percent, 85 percent, 90 percent, 95 percent, 96 percent, 97 percent, 98 percent, or 99 percent sequence identity to a sequence of the plasmid shown in FIG. 38. In particular, a nucleotide sequence encoding the donor plasmid used to construct the viral vector that includes antigens for AIV and NDV may have greater than 90 percent sequence identity to a sequence of the plasmid shown in FIG. 38. For example, a nucleotide sequence encoding the donor plasmid used to construct the viral vector that includes antigens for AIV and NDV may have greater than 95 percent sequence identity to a sequence of the plasmid shown in FIG. 38.

In some embodiments, an *in vitro* recombination (IVR) may be performed by co-electroporation of 2° CEF cells using a linearized pFwt-IRES[MOD]-COBRA-C as the donor plasmid and viral DNA isolated from vHVT013. Co-electroporation may be performed using 1×10⁷ 2° CEF in 300µl Opti-MEM and shocked at 150 volts with 950 capacitance in a 2mm electroporation cuvette. The transfected cells may be seeded into 96-well plate and incubated for 4 days. The cells grown in the 96-well plate may then duplicated into two 96-well plates. One set of 96-well plates may be used for IFA using chicken polyclonal sera against AIV H5 HA to identify positive wells containing recombinants and another set of 96-well plates may be used for recovering the infected cells from the corresponding positive wells.

Recombinant viral purification methods may be used to recover the recombinant viral vectors. These purification methods may be performed first by 96-well plate duplication and IFA selection for the wells containing the most IFA positive plaques with the least amount of IFA negative plaques. Wells matching those criteria may be harvested and adjusted to 1 mL in DMEM+2% FBS. From the 1 mL stock, 10-20µl may be removed and mixed with 1×10⁷ 2° CEFs in 10 mL DMEM+2% FBS and aliquoted onto a new 96-well plate in an attempt to have single HVT plaques per well. The supernatant of the wells that contained single plaques may be tested for the absence of parental virus by PCR. This process may be repeated for few rounds until all tested wells showed pure recombinant banding patterns and no parental virus.

A nucleotide sequence of for the plasmid pVP2-IRES[MOD]-Cobra-C may have at least 80 percent, 85 percent, 90 percent, 95 percent, 96 percent, 97 percent, 98 percent, or 99 percent sequence identity to a sequence of the plasmid shown in FIG. 16. As shown, the virus construct, vHVT509 is formed from MDV-3 strain FC-126 with the addition of a sequence encoding Cobra-C H5 HA.

Selection of a virus vector may include considering several properties of the virus including, but not limited to the replication of the virus in the host, the ability of the virus to induce an immune response, such as humoral and/or cell-mediated immunity, a reduction in mortality, reduction in symptoms of interest, the ease of producing the virus, ability to incorporate the virus into a immunogenic composition, ease of administration to an avian, etc. Viruses selected for use in the production the immunogenic compositions may include one of more strains of viruses such as herpesvirus of turkeys (rHVT) such as Gallid herpesvirus, Marek's disease virus, Meleagrid herpesvirus and/or other viruses known in the art. In particular, various strains of HVT, MDV, or MeHV may be selected for use as a live viral vector vaccine to protect against many important diseases, in particular viral diseases affecting poultry.

Immunization using Marek's disease virus (MDV) serotype 3 (MDV-3), also known as herpesvirus of turkey (HVT), has been used worldwide to protect chicken populations against MDV. In addition, HVT has been used to develop as a live viral vector vaccine to protect against many important viral poultry diseases thanks to its persistent replication in the host, its ability to induce both humoral and cell-mediated immunity, and its relatively easy production and administration, among other advantages.

A particular strain of MDV (i.e., HVT), MDV-1, MDV-2, and/or MDV-3 may be selected for predetermined properties that are of interest for the desired product or application (i.e., administration to a particular class of animal). These properties may include, but are not limited to, efficacy against the disease of interest, in particular in some instances the efficacy of a viral vector against diseases of interest. For example, the efficacy of a viral vector against the disease it encodes itself and/or against the disease from which the exogenous sequence(s) are derived. For example, the exogenous sequences encoding the antigens and/or proteins derived from IBD, ND, AIV, such as an AIV H5, and/or any other virus of interest. An MDV strain, in particular, MDV-3, may be used to construct a recombinant virus vector. For example, the MDV-3 FC-126 strain may be used for an immunogenic composition due to its persistent replication in the host, its ability to induce both humoral and cell-mediated immunity, and its relatively easy production and administration, among other advantages. For example, MDV-3 FC-126 clone 2 may be used to form the recombinant construct of interest.

Recombinant vaccine vectors may be constructed from predetermined viruses using plasmids. Plasmids may be used to insert one or more desired sequences into the predetermined virus to form a recombinant virus vector. Further, exogenous nucleic acid molecule encoding for preselected antigens may be included in some recombinant vaccine vectors. In particular, a sequence encoding proteins of interest, for example, sequences which encode for antigens of one or more avian diseases, may be inserted into a viral vector using a plasmid.

In some instances, plasmids may be used to insert an expression cassette into a virus vector. For example, an expression cassette may include preselected DNA that may include one or more genes and sequences controlling their expression (e.g., regulatory sequence). The expression cassette may instruct the cell's machinery to make RNA and/or protein(s). Expression cassettes may allow for modular cloning of protein-encoding sequences.

In some instances, an expression cassette may include multiple components, for example, a promoter sequence, an open reading frame, and/or untranslated regions. Expression cassettes can be transfected into different vectors. In particular, a promotor and DNA from a desired gene may be inserted in a vector.

Sequences derived from one or more diseases such as avian influenza (AI), for example, highly pathogenic avian influenza (HPAIV), infectious bursal disease (IBDV), Newcastle Disease (NDV), infectious laryngotracheitis virus (ILTV), HVT, MDV, for example MDV-1, MDV-2, or MDV-3, MeHV, and/or avian viruses of interest may be combined to form a immunogenic agent for use in an immunogenic composition. For example, an immunogenic composition may be a vaccine, such as a multivalent vaccine.

Sequences, including exogenous sequences, may be used to construct a viral vector. For example, some viral vectors may include sequences derived from avian pathogens including but not limited to avian encephalomyelitis virus, avian reovirus, avian paramyxovirus, avian metapneumo virus, avian influenza virus, avian adenovirus, fowl pox virus, avian coronavirus, avian rotavirus, chick anemia virus, avian astrovirus, avian parvovirus, Newcastle disease virus, Marek's disease virus, infectious bursal disease virus, coccidiosis (Eimeria sp.), Campylobacter sp., Salmonella sp., Pasteurella sp., Avibacterium sp., Mycoplasma gallisepticum, Mycoplasma synoviae, Clostridium sp., and E. coli.

In some embodiments, viral vectors for use in an immunogenic composition, such as a vaccine may include genes, sequences, or nucleotides encoding an antigen or polypeptide of interest including, but not limited to Newcastle Disease Virus fusion protein (NDV-F), Newcastle Disease Virus hemagglutinin neuraminidase (NDV-HN), Marek's Disease Virus glycoprotein C (gC), Marek's Disease Virus glycoprotein B (gB), Marek's Disease Virus glycoprotein E (gE), Marek's Disease Virus glycoprotein I (gl), Marek's Disease Virus glycoprotein H (gH) or Marek's Disease Virus glycoprotein L (gL), IBDV VP2, IBDV VPX, IBDV VP3, IBDV VP4, ILTV glycoprotein B, ILTV glycoprotein I, ILTV UL32, ILTV glycoprotein D, ILTV glycoprotein E, ILTV glycoprotein C, influenza hemagglutinin (HA), influenza neuraminidase (NA), protective genes derived from Mycoplasma gallisepticum (MG), or Mycoplasma synoviae (MS), or combinations thereof. Examples of trivalent viral vectors are shown in FIG. 2.

A recombinant virus vector may be constructed by a recombination method that includes transfecting a cell with a virus and an insertion plasmid or donor plasmid for insertion into a particular site on the virus.

Nucleotide inserts delivered to the virus by a donor plasmid may include sequences encoding for desired proteins such as antigens or may be selected based on the effect expression of the sequences will have *in vivo.* Donor plasmids may be selected for specific properties of the plasmid such as ability to introduce sequences at a specific site, for example, a plasmid may include flanking homologous regions to direct recombination with the virus, strength of expression of recombinant antigen, and/or stage of expression in the virus lifecycle.

For example, a donor plasmid may be used to introduce the desired sequences at a recombination locus of a virus. For example, sequences may be inserted at a recombination locus of an MDV-3, such as an intergene 1 locus located in the BamHI-I fragment.

In some embodiments, a selected donor plasmid may include one or more of an mCMV promoter, an SV40 poly A tail, an exogenous nucleic acid sequence encoding avian influenza antigen, an Internal Ribosome Entry Site (IRES), an exogenous sequence encoding the infectious bursal disease virus antigen and/or an exogenous sequence encoding a Newcastle disease virus antigen.

In particular, a recombinant virus vector may be constructed by a recombination method that includes transfecting a cell with MDV-3 (e.g., herpesvirus of turkeys (HVT)) and an insertion plasmid for insertion into MDV-3 Intergenic I (IG1) site that may include a SV40 poly A tail, an exogenous nucleotide sequence encoding an avian influenza antigen, an Internal Ribosome Entry Site (IRES), an exogenous nucleotide sequence encoding an infectious bursal disease virus antigen, and/or an exogenous nucleotide sequence encoding a Newcastle disease virus antigen.

Inserts, for inclusion, in a donor plasmid and the subsequently formed viral vector, may include sequences encoding for desired proteins such as antigens or may be selected based on the effect expression of the sequences will have *in vivo.*

Exogenous sequences that are inserted into a virus to form a virus vector may be codon optimized in some instances

Immunogenic compositions, such as vaccines that include vMDV-H5 (i.e., vHVT-H5) constructs may be developed that include different H5 inserts and/or inserts for antigens of other diseases. In some embodiments, a recombinant viral vector may be constructed to include a synthetic hemagglutinin (HA) protein gene of Avian influenza virus A (subtype H5) using computationally optimized broadly reactive (COBRA). For example, a recombinant MDV (i.e., HVT) may be constructed to include a synthetic hemagglutinin (HA) protein gene of Avian influenza virus A (subtype H5) using COBRA.

In particular, recombinant MDV HA H5 vaccines may be developed that include different H5 inserts, inserts for antigens of other diseases, and/or other functional inserts. *In vivo* recombinant vector HVT with H5 insert (vMDV-H5 and/or vHVT-H5) have demonstrated promising results against HPAI in poultry. In some embodiments, a single H5 insert may be provided in one viral vector. However, embodiments of a viral vector may include multiple gene inserts of H5.

Coupling COBRA and vMDV (i.e., vHVT) technologies could improve H5 HPAI virus control in the field. Use of a live virus as vector platform may improve COBRA HA efficacy.

Utilizing recombinant technologies along with COBRA to generate immunogenic agents for immunogenic compositions to be provided to avians may improve virus control in the field. For example, use of COBRA and recombinant viral vectors to construct an immunologic composition may result in improved H5 HPAI virus control.

For example, an exogenous nucleic acid molecule encoding the avian influenza HA antigen may be codon optimized when used in a viral vector, such as MDV-3.

Viral vectors may be formed that include one or more sequences of interest. In particular, a viral vector may be a recombinant MDV that includes a promoter, an avian virus antigen, and/or a SV40 polyA tail at a recombination region of MDV, as well as other sequences encoding genes and/or functionalities of interest.

In some embodiments, a recombinant MDV may be constructed to include an antigen, such as a protein gene of Avian influenza virus. Such recombinant MDV constructs may also include a promoter, AIV protein gene, preselected sequences to be inserted at predetermined sites of an MDV.

A recombinant MDV construct may include a mCMV promoter, AIV HA COBRA-C, SV40 polyA tail at recombination region intergenic site 1 of MDV-3 strain. For example, viral vector vHVT509 may be a recombinant MDV-3 that includes an mCMV promoter, an AIV HA COBRA-C, a SV40 polyA tail at recombination region intergenic site 1 of MDV-3 FC-126 strain.

A nucleotide sequence of a viral vector vHVT509 may have at least 80 percent, 85 percent, 90 percent, 95 percent, 96 percent, 97 percent, 98 percent, or 99 percent sequence identity to a sequence of the sequenced region of vHVT509 of FIG. 13. In some embodiments, it may be desired to utilize a nucleotide sequence that has greater than about 90% identity and/or homology with the sequence for vHVT509 as shown in FIG. 13.

In one embodiment, an amino acid sequence for a AIV HA COBRA-C gene as used in vHVT509 has at least 80 percent, 85 percent, 90 percent, 95 percent, 96 percent, 97 percent, 98 percent, or 99 percent sequence identity to a sequence as shown in FIG. 12. It may be desired depending on the targeted disease, targeted animals, and/or properties of the animals to utilize a viral vector having at least a portion of a nucleotide sequence that has greater than about 90% identity and/or homology with a sequence for vHVT509 as shown in FIG. 13.

In some embodiments, a donor plasmid used to form a viral vector may include a sequence encoding an avian influenza antigen. The viral vector may be modified using a plasmid that includes a sequence encoding for AIV antigen. In some embodiments, vHVT522 may include a gene encoding a synthetic hemagglutinin (HA) protein of Avian Influenza virus A (subtype H5) designed by computationally optimized broadly reactive approaches (COBRA).

A donor plasmid In some instances, a recombinant viral construct may be formed from a parental virus, such as an MDV-3. In particular a strain such as MDV-3 FC-126 (Igarashi T. et al., J. Gen. Virol. 70, 1789-1804, 1989) may be used. For example, in some embodiments of a viral vector the parental virus may be MDV-3 FC-126 clone 2. A donor plasmid may be used to introduce the desired sequences at a recombination locus of an MDV-3. In some instances, a donor plasmid may be constructed such that at least a portion of the plasmid is homologous to the target virus. For example, the donor plasmid may be constructed such that at least a portion of the insertion occurs at a recombination locus of an MDV3, such as an intergene 1 locus located in the BamHI-I fragment.

Donor plasmids may be selected and/or constructed based on properties of interest. A donor plasmid may include a promoter, a gene of interest, and a polyA (transcription stop). Promoters may be selected for various properties determined to be of use to the developer. For example, promoters may vary in strength (e.g., strong, medium, weak) and/or in kinetic class (i.e., time of activation, e.g., immediate early, early, late promoters) and may be selected for a particular strength, a particular time of activation, or a combination of variables. A gene of interest may include a wild-type sequence or codon optimized sequence. In some instances, a donor plasmid may include polyA sequences. Selection of a specific polyA sequence for use in a plasmid may be due to the polyA sequence's ability to combine with a predetermined gene, its ability to inhibit and/or avoid recombination with another gene using similar polyA, its effect on level of expression of the recombinant gene, its compatibility with the sequence coding for the recombinant gene, and/or other desired properties or effects known in the art.

Donor plasmids may be constructed to have one or more antigens and/or proteins that are derived from diseases against which the immunogenic composition provides protection. For example, donor plasmids may include antigens derived from IBD, ILT, ND, AI, MD and/or any combination thereof.

Donor plasmids may include a promoter such as a mCMV promoter, a gene of interest for the target disease and a polyA (transcription stop). For example, in an embodiment that targets AIV, a selected donor plasmid may include an mCMV promoter, AIV HA COBRA-C, and/or SV40 polyA tail. Such a donor plasmid may be designated as pCD046-COBRA-C.

Examples of donor plasmids include, but are not limited to, pCD046-COBRA-C, pFwt-IRES[MOD]-Cobra-C, pVP2-IRES[MOD]-Cobra-C, as well as other plasmids incorporating different combinations of exogenous sequences.

A nucleotide sequence of for the plasmid pFwt-IRES [MOD]-Cobra-C may have at least 80 percent, 85 percent, 90 percent, 95 percent, 96 percent, 97 percent, 98 percent, or 99 percent sequence identity to a sequence of the plasmid shown in FIG. 38. A nucleotide sequence of for the plasmid pVP2-IRES[MOD]-Cobra-C may have at least 80 percent, 85 percent, 90 percent, 95 percent, 96 percent, 97 percent, 98 percent, or 99 percent sequence identity to a sequence of the plasmid shown in FIG. 16.

A recombinant virus for use as a vector may be generated in the manner disclosed herein. In particular, an *in vitro* recombination (IVR) may be performed by co-electroporation of secondary chicken embryo fibroblast (CEF) cells using a donor plasmid, such as pCD046-COBRA-C, and viral DNA isolated from a desired virus, such as MDV-3 strain FC-126. Co-electroporation may be performed using 1×10⁷ secondary CEF in 200 µL of a reduced serum media and shocked at 150 volts with 950 capacitance in a 2 mm electroporation cuvette. The transfected CEF cells may be seeded with a cell growth media, such as a 2% fetal bovine serum (FBS) containing a basal medium (such as Dulbecco's Modified Eagle Medium "DMEM") into 96-well plate and incubated at 37°C, 5% CO₂ incubator for 4-5 days. The cells grown in the 96-well plate may then be duplicated into two 96-well plates and incubated for 2 or 3 more days at 37 °C, 5% CO₂ incubator. One set of duplicated 96-well plates may be screened for a recombinant virus by indirect immunofluorescence assay (IFA) using chicken anti-AIV H5N2 sera and rabbit anti-chicken IgY (IgG) (whole molecule)-FITC conjugated. To obtain a pure population of recombinant virus, CEF from the IFA positive wells in a corresponding 96-well plate may be recovered and processed in a purification step.

Purification of the recombinant virus may be conducted by dilution of the population mixture (recombinant and parent virus) in 96-well plate followed by IFA and PCR screen. After identifying the recombinant virus, further dilution may be conducted from CEF in corresponding wells of the duplicated plate until only pure recombinant virus is in a well of the plate. Briefly, the wells in corresponding 96-well plate that IFA positive may be collected by trypsinization with TrypLE express and adjusted to 1 mL with 10% FBS-DMEM. From the 1 mL stock, 5-20µl of stock, depending on the number of plaques in the well, may be removed and mixed with 3-5×10⁶ CEFs in 20 mL 2% FBS-DMEM and aliquoted into a new 96-well plate in an attempt to have single HVT plaque per well. The 96-well plates may be duplicated after 4 days of incubation at 37 °C, 5% CO₂ incubator and each well may be tested for the presence of recombinant virus by IFA and PCR. The wells that appeared to have more recombinant virus, by comparing the PCR amplification patterns, may be harvested and the purification step repeated as described above until have the pure recombinant populations.

In order to complete PCR Analysis of the recombinant virus, viral DNA may be extracted from viral vector, for example, vHVT509. PCR primers may be designed to identify the presence of the AIV HA COBRA-C, the mCMV promoter, SV40 polyA tail, and/or the flanking recombination arms of HVT virus (MDV-3). PCR amplifications may be performed using DNA template along with the primer pairs such as MB080 (CGAACAAACTTCATCGCTATGC (SEQ ID NO: 1)) and MB081 (TAACTCAAATGCGAAGCGTTGC (SEQ ID NO: 2)), mCMV-F (AACTCCGCCCGTTTTATG (SEQ ID NO: 3)) and SV40 tail-R (TCGACTCTAGAGGATCCG (SEQ ID NO: 4)), and SV40 pro-F (AGCTTGGCTGTGGAATGT (SEQ ID NO: 5)) and syntail-R (ATGTTCTGGCACCTGCAC (SEQ ID NO: 6)) where the sequences are listed according to the forward strand (5' to 3'). PCR may be run using standard conditions known in the art and/or the conditions outlined in the examples described herein.

Other viral vectors that incorporate different inserts, including but not limited to vHVT522 and vHVT523, may include different exogenous sequences. For example, recombinant viral vector, such as recombinant virus vHVT522 may include the following exogenous sequences, including: MB080 (CGAACAAACTTCATCGCTATGC (SEQ ID NO: 7)) and MB081 (TAACTCAAATGCGAAGCGTTGC (SEQ ID NO: 8)), COBc-FF (TGGTATGGGTACCACCATAGCAATGAGC (SEQ ID NO: 9)), Vp2-FF-ak (GTCACACTAGTAGCCTACGAAAGAGTGGC (SEQ ID NO: 10)), and poly A tail-RR (CACTGCATTCTAGTTGTGGTTTGTCCA (SEQ ID NO: 11)) where the sequences are listed according to the forward strand (5' to 3').

Another embodiment of a recombinant viral vector, such as recombinant virus vHVT523 may include the following exogenous sequences, including: MB080 (CGAACAAACTTCATCGCTATGC (SEQ ID NO: 7)) and MB081 (TAACTCAAATGCGAAGCGT (SEQ ID NO: 12)), COBc-FF (TGGTATGGGTACCACCATAGCAATGAGC (SEQ ID NO: 9)), NDV-F1 (ATGGGCTCCAAACCTTCTAC (SEQ ID NO: 13)), and polyA tail-RR (CACTGCATTCTAGTTGTGGTTTGTCCA (SEQ ID NO: 11)) where the sequences are listed according to the forward strand (5' to 3').

Expression may be determined using the analysis described herein. An indirect immunofluorescence assay (IFA) may be performed on the generated viral vector, for example, vHVT509. The CEFs that inoculated with the viral vector, for example, vHVT509 may be fixed with ice-cold 95% acetone for five minutes at room temperature and air-dried for 10 min. After rehydrating the plate with PBS, two primary antibodies 1) chicken anti-AIV H5N2 sera at 1:500 dilution and 2) L78 monoclonal antibody against MDV-3 at 1:3000 dilution may be added and incubated for 1 hour at 37°C. After three washes with PBS, two secondary antibodies, 1) rabbit anti-chicken IgG-FITC at 1:500 dilution and 2) donkey anti-mouse IgG-Alexa Fluor 568 at 1:300 dilution may be added. The plates may be incubated at 37°C for 1 hour and followed by three times with PBS. The cells may be observed to identify the IFA positive plaques with a fluorescent microscope using fluorescein isothiocyanate (FITC)-filters and tetramethylrhodamine isothiocyanate (TRITC)-filters of an inverted microscope.

The analysis of the genomic DNA of a viral vector, such as vHVT509, may be conducted by nucleotide sequence determination of part of recombination arm region as well as the inserted gene cassette. Primer set, MB080 and MB081 were used to amplify the entire inserted gene cassette including part of recombination arm. PCR amplification may be performed as follows: 94°C for 2 min, 30 cycles of 94°C for 30 sec, 60°C for 30 sec, 68°C for 3 min, and final extension at 68°C for 5 min. The 3.6 kb PCR product may be purified. Total eight PCR reactions of 25 µl per reaction may be pooled and purified using the kit. The final elution may be confirmed by agarose gel electrophoresis. The detailed sequencing primers are listed in Table 4.

Genetic stability analysis may be conducted using serial passages. The preMSV stocks may be passed into secondary CEF by blind passages. Briefly, 100 µl of previous passage may be inoculated into CEF monolayers in T25 flask. The cells may be incubated in 37 °C, 5% CO₂ incubator until it makes good size of virus plaques (typically 3-4 days). The virus infected CEF may be collected by trypsinization with TrypLE express (Gibco # 12604) and may be adjusted to total 1 mL with 10% FBS-DMEM and 10% DMSO for LN2 storage. Approximately 50- 100 µl of the stock may be passed to fresh CEF culture in T25 flask. Total 13 passages (X+12) from preMSV (X-1) may be conducted. *In vitro* characterization of X+12 stocks may be conducted for PCR screening, the nucleotide sequence analysis of the transgene cassette, and the expression of AIV HA COBRA-C by dual IFA as described earlier.

After multiple rounds of plaque purification, pure recombinant virus (e.g., vHVT509) may be isolated. The vHVT509 may be tested by IFA and PCR to validate the appropriate transgene insertion as well as to confirm that there is no remnant parental virus. The recombinant virus (e.g., vHVT509) may then be passed from one well of 96-well plate to T-25 flasks, T-150 flasks, and finally roller bottles (850cm surface area) to generate pre-MSV stock.

PCR analysis, in particular, PCR amplification with various primers may be used to confirm that the viral vector, for example an vHVT509, may have an expected amplification pattern and amplicon at preMSV stock and at X+12 passage level.

Analyzing in this manner may help to ensure, for example, that the recombinant MDV-3 viral vector (e.g., herpesvirus of turkeys (rHVT)) formed, may include and express *in vivo* an exogenous nucleic acid molecule encoding the desired avian disease antigens, for example, an exogenous avian influenza virus antigen.

Recombinant viral vector, vHVT509, is shown in FIG. 13 which depicts the nucleotide sequences of vHVT509.

In some instances, an AIV antigen, such as a synthetic hemagglutinin (HA) protein gene of Avian Influenza virus A (subtype H5) designed using a computationally optimized broadly reactive (COBRA) approach (i.e., COBRA-C (H5 HA protein)) may be combined with at least one avian virus antigen.

A recombinant virus vector may include at least one exogenous amino acid sequence having at least 80 percent, 85 percent, 90 percent, 95 percent, 96 percent, 97 percent, 98 percent or 99 percent sequence identity to a sequence that encodes an AIV H5 HA Cobra-C gene shown in FIG. 12, FIG. 18, FIG. 28, FIG. 40, and/or FIG. 49. In some embodiments, a recombinant virus vector may at least one exogenous amino acid sequence having at least 90 percent sequence identity to a sequence that encodes an AIV H5 HA Cobra-C gene shown in FIG. 12, FIG. 18, FIG. 28, FIG. 40, and/or FIG. 49. In particular, a recombinant virus vector may include at least one exogenous amino acid sequence having at least 95 percent sequence identity to a sequence that encodes an AIV H5 HA Cobra-C gene shown in FIG. 12, FIG. 18, FIG. 28, FIG. 40, and/or FIG. 49.

For example, a recombinant virus vector may include at least one sequence having a homology or identity in a range from about 80 to about 100 percent to a sequence that encodes an AIV H5 HA Cobra-C gene as shown in FIG. 12, FIG. 18, FIG. 28, FIG. 40, and/or FIG. 49.

In some embodiments, a COBRA-C H5 HA protein gene or a portion thereof may be combined with a synthetic Infectious Bursal Disease Virus protein, such as a IBDV capsid protein VP2, a Newcastle Disease Virus protein, such as a Newcastle Disease F protein (NDV-F) gene, or any avian disease antigen protein of interest.

For example, in some instances, cDNA from a VP2 gene of Infectious Bursal Disease Virus and a poly-A sequence may be inserted. For example, the cDNA may be made from genomic RNA from virions isolated from the Bursa of chickens. The locus of insertion may be defined, and insertion may target plasmid sequences homologous to the virus. Thus, in some instances, for example, when the parental virus is HVT FC-126, the plasmid targeted may include sequences homologous to HVT FC-126 sequences of the virus.

In some embodiments, a sequence, or a partial sequence from an IBDV antigen, may be used in a viral vector. For example, an IBDV protein sequence flanked by a sequence derived from the IG1 region of an MDV-3 may allow for homologous recombination into an MDV-3 (e.g., Vaxxitek virus).

In one aspect of the embodiment, a nucleotide and/or an amino acid sequence encoding a IBDV antigen or polypeptide, has at least 80 percent, 85 percent, 90 percent, 95 percent, 96 percent, 97 percent, 98 percent or 99 percent sequence identity to a polypeptide having a reference sequence, or a conservative variant, an allelic variant, a homolog or an immunogenic fragment comprising at least eight or at least ten consecutive amino acids of one of these polypeptides, or a combination of these polypeptides.

A recombinant virus vector may include at least one exogenous amino acid sequence having at least 80 percent, 85 percent, 90 percent, 95 percent, 96 percent, 97 percent, 98 percent, or 99 percent sequence identity to a sequence that encodes a capsid protein (VP2) protein of IBDV as shown in FIG. 17 or FIG. 29. In some embodiments, a recombinant virus vector may at least one exogenous amino acid sequence having at least 90 percent sequence identity to a sequence that encodes a capsid protein (VP2) protein of IBDV as shown in FIG. 17 or FIG. 29. In particular, a recombinant virus vector may include at least one exogenous amino acid sequence having at least 95 percent sequence identity to a sequence that encodes the capsid protein (VP2) protein of IBDV as shown in FIG. 17 or FIG. 29. For example, a recombinant virus vector may include at least one exogenous amino acid sequence having at least at least 95 percent sequence identity to a sequence that encodes the capsid protein (VP2) protein of IBDV as shown in FIG. 17 or FIG. 29.

A recombinant virus vector may include at least one amino acid sequence having a homology or sequence identity in a range from about 80 to about 100 percent to sequence that encodes an IBDV capsid protein (VP2) protein as depicted in FIG. 17 or FIG. 29.

In an embodiment, a viral vector may include polynucleotides encoding at least a portion of MDV, an AIV antigen, and/or a NDV antigen. Heterologous polynucleotides coding for and expressing an NDV-F antigen may be used to form a donor plasmid. In some embodiments, sequences encoding Newcastle Disease Virus Fusion protein (NDV-F) may be used in place of sequences encoding the VP2 protein. In particular, a Newcastle Disease Virus Fusion protein (NDV-F) that is flanked by a sequence derived from the IG1 region. This may enhance homologous recombination into a virus to for the recombinant virus vector. For example, the use of NDV-F protein sequence that is flanked by the sequence derived from the IG1 region may allow for homologous recombination into an MDV-3 (e.g., Vaxxitek virus).

In one aspect of the embodiment, a nucleotide sequence encoding and expressing an NDV-F antigen has at least 80 percent, 85 percent, 90 percent, 95 percent, 96 percent, 97 percent, 98 percent or 99 percent sequence identity to a sequence as set forth in FIG. 39 or FIG. 50, or a conservative variant, an allelic variant, a homolog or an immunogenic fragment comprising at least eight or at least ten consecutive amino acids of one of these polypeptides, or a combination of these polypeptides.

A recombinant virus vector may include at least one exogenous amino acid sequence having at least 80 percent, 85 percent, 90 percent, 95 percent, 96 percent, 97 percent, 98 percent, or 99 percent sequence identity to a sequence that encodes an NDV-F protein as shown in FIG. 39 or FIG. 50. In some embodiments, a recombinant virus vector may at least one exogenous amino acid sequence having at least 90 percent sequence identity to a sequence that encodes an NDV F (NDV-F) protein shown in FIG. 39 or FIG. 50. In particular, a recombinant virus vector may include at least one exogenous amino acid sequence having at least 95 percent sequence identity to a sequence that an NDV-F protein shown in FIG. 39 or FIG. 50.

For example, the recombinant virus vector may have a homology or identity in a range from about 80 to about 100 percent to a sequence for an NDV-F protein as shown in FIG. 39 or FIG. 50.

Promoters may be present in the parental virus or a promoter may be inserted into the viral vector using a plasmid. For example, in an embodiment a viral vector may only include a promoter from the parental virus such as a mCMV promoter.

A functional insert may include an SV40 polyA tail. An SV40 polyA tail may be inserted at a specific site of a viral vector. For example, an SV40 polyA tail may be inserted at a recombination region intergenic site 1 of an MDV-3 FC-126 strain. Such a construct is used in the viral vectors vHVT522 and vHVT523 as described herein.

In particular, a recombinant virus construct may include a gene encoding a synthetic hemagglutinin (HA) protein of Avian Influenza virus A designed using a computationally optimized broadly reactive (COBRA) approach. For example, viral vector vHVT522 may include a synthetic hemagglutinin protein gene of Avian Influenza virus A, specifically COBRA-C (H5 HA protein) designed using COBRA, as well as a synthetic infectious bursal disease virus structural capsid protein VP2. Recombinant viral vector, vHVT522, is shown in FIG. 26 which depicts the nucleotide sequences of vHVT522.

In some embodiments, at least a portion of the viral vector may include a nucleotide sequence having at least 80 percent, 85 percent, 90 percent, 95 percent, 96 percent, 97 percent, 98 percent, or 99 percent sequence identity to a sequence of the sequenced region of vHVT522 shown in FIG. 27.

In one embodiment, an amino acid sequence for a AIV HA COBRA-C gene as used in vHVT522 has at least 80 percent, 85 percent, 90 percent, 95 percent, 96 percent, 97 percent, 98 percent, or 99 percent sequence identity to the sequence as shown in FIG. 28. An amino acid sequence for an IBDV VP2 gene in a viral vector vHVT522 may have at least 80 percent, 85 percent, 90 percent, 95 percent, 96 percent, 97 percent, 98 percent, or 99 percent sequence identity to the sequence as shown in FIG. 29.

An embodiment may include an insertion plasmid for the Intergenic I (IG1) site of the viral vector, such as an MDV-3 strain, that may include a SV40 poly A tail, COBRA-C (H5 HA protein), Internal Ribosome Entry Site (IRES), and infectious bursal disease virus capsid protein (IBDV VP2). For example, an insertion plasmid may be used to insert a SV40 poly A tail, COBRA-C (H5 HA protein), Internal Ribosome Entry Site (IRES), and infectious bursal disease virus capsid protein (IBDV VP2) into the right IG1 arm of a MDV-3 virus (e.g., MDV-3 FC126 as used in the Vaxxitek vaccine).

For example, a plasmid used in the invention may include pVP2-IRES[MOD]-COBRA-C.

In some instances, the invention may include a synthetically ordered plasmid that includes the right Intergenic I arm of an MDV-3 (e.g., Vaxxitek virus), SV40 poly A tail, COBRA-C (H5 HA protein), IRES, and Infectious Bursal Disease Virus capsid protein.

Some embodiments of a viral vector may only include an external promoter from the parental virus which had been previously modified. For example, an external promoter, such as mCMV may be present in the parental virus that had been previously modified, such as vHVT013.

Further, some plasmid embodiments may include genes that confer antibiotic resistance to one or more antibiotics including but not limited to tetracycline, kanamycin, ampicillin, etc. and/or combinations thereof. In particular, a gene conferring antibiotics resistance to ampicillin may be introduced into a viral vector.

Exogenous DNA may be introduced into a plasmid which is then used to introduce the exogenous DNA into a viral vector to create a recombinant virus. For example, a donor plasmid, for example pVP2-IRES-Cobra-C, may be created by cloning a synthetic COBRA-C fragment (Genscript Lot U5872BA260-3) into pVP2-IRES-Cobra-A. The pVP2-IRES-Cobra-C plasmid may further be modified by replacing the HindIII-BlpI fragment by a new synthetic fragment VP2+COBC to remove an intervening stretch of sequence between the end of IRES sequence and start of COBRA-C sequence present in pVP2-IRES-Cobra-C.

A nucleotide sequence for plasmid pVP2-IRES [MOD]-Cobra-C used to construct vHVT522 may have at least 80 percent, 85 percent, 90 percent, 95 percent, 96 percent, 97 percent, 98 percent, or 99 percent sequence identity to the sequence shown in FIG. 16.

In one embodiment, an amino acid sequence encoding the H5 HA Cobra-C protein in plasmid pVP2-IRES[MOD]-Cobra-C used to construct vHVT522 has at least 80 percent, 85 percent, 90 percent, 95 percent, 96 percent, 97 percent, 98 percent, or 99 percent sequence identity to the sequence as shown in FIG. 18.

Donor plasmids for use in generating the recombinant virus may include various configurations including, but not limited to pCD046-COBRA-C, pVP2-IRES [MOD]-Cobra-C, pFwt-IRES [MOD]-Cobra-C.

For example, as can be seen in FIG. 16, the depicted nucleotide sequence of plasmid pVP2-IRES [MOD]-Cobra-C, which is used in the formation of vHVT522 viral vector, shows a plasmid construction having Intergene 1 Arms (Green), SV40 Poly A (Orange), H5 HA COBRA-C (Dark Red), IRES (Blue), VP2 (Red), and Vector (Black).

Another example of a vaccine vector for immunogenic compositions is vHVT523 as described herein. vHVT523 may include multiple sections of exogenous DNA. An embodiment of recombinant viral vector, vHVT523, is partially shown in FIG. 48 which depicts a partial nucleotide sequence of vHVT523.

The depicted nucleotide sequence of plasmid pFwt-IRES-COBRA-C, which is used in the formation of vHVT523 viral vector, shows a plasmid construction having IG1 Arm (Light Green), mCMV (Blue), NDV-F (Orange), IRES (Purple), H5 HA (Dark Red), SV40 poly A (Slight Blue), and IG1 Arm (Green). Further, FIG. 38 indicates the locations of specific nucleotides of interest in the plasmid for the vHVT523 viral vector.

These sections of exogenous DNA may be selected for desired properties and/or encoding and expressing desired genes *in vivo.* For example, vHVT523 may include a gene encoding one or more of a synthetic hemagglutinin (HA) protein of Avian Influenza virus A (subtype H5) designed using computationally optimized broadly reactive (COBRA), a synthetic Newcastle Disease Virus (NDV) fusion protein (F).

In some embodiments, at least a portion of the viral vector may include a nucleotide sequence having at least 80 percent, 85 percent, 90 percent, 95 percent, 96 percent, 97 percent, 98 percent, or 99 percent sequence identity to a sequence of the sequenced region of vHVT523 shown in FIG. 48.

An amino acid sequence for an NDV-F gene as used in viral vector vHVT523 may have at least 80 percent, 85 percent, 90 percent, 95 percent, 96 percent, 97 percent, 98 percent, or 99 percent sequence identity to a sequence that encodes an NDV-F protein shown in FIG. 39 or FIG. 50. In some embodiments, an amino acid sequence for an NDV-F gene as used in viral vector vHVT523 may have at least 90 percent sequence identity to a sequence that encodes an NDV-F protein shown in FIG. 39 or FIG. 50.

In particular, a recombinant virus vector may include at least one exogenous amino acid sequence having at least 95 percent sequence identity to a sequence of an NDV-F protein as shown in FIG. 39 or FIG. 50.

For example, the recombinant virus vector includes an amino acid sequence having a homology or an identity in a range from about 80 percent to about 100 percent to a sequence for an NDV-F protein as shown in FIG. 39 or FIG. 50.

Genes may be inserted into a vector using a plasmid. An insertion plasmid for the Intergenic I (IG1) site of a viral vector may include a SV40 poly A tail, COBRA-C (H5 HA protein), Internal Ribosome Entry Site (IRES), Newcastle Disease Virus Fusion protein (NDV-F) and mouse CMV promoter flanked by sequence derived from the IG1 region of the virus of interest to facilitate homologous recombination. In particular, an insertion plasmid for the Intergenic I (IG1) site of a MDV-3 viral vector, may include, for example, a SV40 poly A tail, COBRA-C (H5 HA protein), Internal Ribosome Entry Site (IRES), Newcastle Disease Virus Fusion protein (NDV-F) and mouse CMV promoter flanked by sequence derived from the IG1 region of MDV-3 to facilitate homologous recombination.

A plasmid for use in creating a viral vector for use in an immunogenic composition may include one or more sections homologous to DNA sections at the insertion site of the predetermined virus, as well as sequences encoding genes and/or proteins of interest. For example, a plasmid used in creating a viral vector for use in an immunogenic composition may include homologous sections corresponding to the right and left Intergenic I arm of an MDV-3 virus, SV40 poly A tail, a COBRA-C H5 HA protein, IRES, and NDV Fusion protein (F).

A nucleotide sequence for plasmid pFwt-IRES [MOD]-Cobra used to construct vHVT523 may have at least 80 percent, 85 percent, 90 percent, 95 percent, 96 percent, 97 percent, 98 percent, or 99 percent sequence identity to the sequence shown in FIG. 38.

In one embodiment, an amino acid sequence for H5 HA Cobra-C in plasmid pFwt-IRES[MOD]-Cobra used to construct vHVT523 has at least 80 percent, 85 percent, 90 percent, 95 percent, 96 percent, 97 percent, 98 percent, or 99 percent sequence identity to the sequence as set forth in FIG. 40.

An amino acid sequence for NDV-F in plasmid pFwt-IRES [MOD]-Cobra used to construct viral vector vHVT523 has at least 80 percent, 85 percent, 90 percent, 95 percent, 96 percent, 97 percent, 98 percent, or 99 percent sequence identity to the sequence as shown in FIG. 39 or FIG. 50.

In addition, exogenous sequences encoding functional properties may be introduced into a plasmid, such as promoters or genes that would confer resistance to antibiotics. Promoters such as an mCMV promoter may be introduced into a plasmid for insertion into a viral vector such as an MDV-3 vector. Further, an ampicillin resistance gene may be introduced into a plasmid for use in formation of viral vectors.

For example, a plasmid (i.e., pFwt-IRES[MOD]-Cobra-C) for use in viral vector construction may be modified from pFwt-IRES-COBRA-C. The plasmid pFwt-IRES-COBRA-C may be constructed by replacing the Saudi-Arabia H9 gene in a plasmid that was created previously (pFwt-IRES-SaH9opt) with COBRA-C gene extracted from pVP2-IRES-COBRA-C. The COBRA-C gene may be extracted from pVP2-IRES-COBRA-C by using the restriction enzymes using SalI and HindIII and may be cloned into pFwt-IRES-SaH9opt that may be digested with same enzymes. The pFwt-IRES-Cobra-C plasmid may be further modified by replacing the HindIII-BlpI fragment by a new synthetic fragment F+COBC to remove an intervening stretch of sequence between the end of IRES sequence and start of COBRA-C sequence present in pFwt-IRES-Cobra-C. The donor plasmid, pFwt-IRES[MOD]-Cobra-C, may be used to generate a recombinant virus.

The immunogenic compositions described herein may include one or more of the following recombinant virus constructs including, but not limited to vHVT509, vHVT522, and/or vHVT523.

A method for producing the recombinant virus vector may include transfecting a cell with MDV (i.e., herpesvirus of turkeys (HVT)), such as MDV-3, and an insertion plasmid for insertion into an Intergenic 1 (IG1) site of MDV-3 that includes a SV40 poly A tail, the exogenous nucleic acid molecule encoding avian influenza HA, an Internal Ribosome Entry Site (IRES), and the exogenous nucleic acid molecule encoding the infectious bursal disease virus VP2 antigen. In some embodiments, the insertion plasmid may include flanking sequences from IG1 to facilitate insertion of the plasmid into the parental virus to form a viral vector.

Any of the viral vectors may be incorporated described herein may be used in an immunogenic composition. Immunogenic compositions may include veterinarily acceptable carrier as well as recombinant virus vectors.

Viral vectors, such as recombinant viruses of MDV-3, may be present in an immunogenic composition in an amount, such that when the composition is administered to an avian, is sufficient to induce an immune response in the avian. For example, an immunogenic composition may induce an immune response to one or more avian diseases such as Marek's Disease Virus, infectious bursal disease virus and/or avian influenza.

A method for inducing an immune response in an avian against Marek's Disease virus, Avian Influenza, infectious bursal disease virus and/or Newcastle disease virus by administering to the avian an effective amount of a recombinant virus vector or an effective amount of a composition that includes antigens of the targeted virus(es).

An effective amount of the active ingredient (e.g., a viral vector) may be measured in plaque forming units. In particular, a viral vector may be administered at a dose greater than about 1000 plaque forming units (PFU) per 0.2 mL dose. In some embodiments, a 0.2 mL dose may include a viral vector at a dose greater than about 2500 PFU.

For example, in an embodiment a dose of vaccine (e.g., 0.2 mL) may include at least 3.0 log₁₀ PFU of the recombinant virus administered via the subcutaneous route. In some instances, a dose administered via the in ovo route may include greater than about 3.6 log10 PFU per dose.

Depending on the route of administration the dose may be delivered to an animal, may be the same, however, it may be delivered in a different volume of carrier. For example, the dose delivered to an avian may be the same for both an in ovo or subcutaneous administration, however, the volume would be 50 µl and 200 µl, respectively.

Minimally effective/protective doses (MPD) of a particular viral vector expressing one or more antigens and/or proteins that are included in a vaccine may be determined experimentally. Generally, the MPD may be used to determine a preferred titer in the immunologic composition. For example, a titer in an immunogenic composition may be a multiple of the MPD value. In particular, the preferred titer in an immunologic composition, such as a vaccine, may be about 3 times the MPD.

In some cases, an immunologic composition, such as a vaccine may have a titer in a range from about 1000 PFU/dose to about 8000 PFU/dose. An embodiment may include a vaccine having a titer in the range from about 2000 PFU/dose to about 6000 PFU/dose.

An embodiment of a vaccine for avians may have a titer in a range from about 2200 PFU/dose to about 4000 PFU/dose. Some avian vaccines described herein may include a titer of greater than about 2250 PFU/dose. In particular, a vaccine may have a titer in a range from about 2250 PFU/dose to about 3000 PFU/dose. For example, a vaccine may have a titer of about 2500 PFU/dose.

In some instances, an effective amount of the viral vector may depend on the type or function of the animal. For example, the poultry industry may need to utilize high PFU titers for long lasting birds, such as breeders and layers. For example, doses having titers greater than about 5000 PFU/dose for MDV (i.e., HVT) may be administered to avians that will have a relatively long lifespan, such as breeders and layers.

In some embodiments, a final vaccine composition may be formed by mixing an ampoule of an immunologic composition with a diluent. Such an immunologic composition may have a total antigen and/or virus titer in a range from about 10³ to about 10⁷ PFU/ml of the immunogenic composition. In particular, an embodiment of an immunologic composition for use in an ampoule may have a total antigen and/or virus titer in a range from about 10⁵ to 10⁷ PFU/ml of the immunogenic composition.

In some embodiments, fractions of viral vectors in the immunogenic compositions may be determined for components that induce a response against the diseases of interest. For example, fractions of an immunogenic composition may include one or more sequences encoding an antigen and/or protein for Marek's Disease, infectious bursal disease, a Newcastle disease, Avian influenza, and/or an infectious laryngotracheitis virus (ILTV) such that each fraction is greater than about 1000 PFU. In some instances, fractions of an immunogenic composition may include one or more sequences encoding an antigen and/or protein for MD, IBD, ND, AI, and/or an ILTV such that each fraction includes greater than about 2000 PFU of the respective immunogenic agent.

In some embodiments, an immunogenic composition such as a vaccine may have a titer greater than about 6000 PFU/dose in total.

Immunological compositions and/or vaccines may be administered by various methods depending on the animals (e.g., type, age, condition), dose, and/or desired result. For example, subcutaneous (S.C.) injection may be used. In some instances, one injection may be sufficient. Alternately, it may be desired to provide two or more injections to ensure the desired immune response. Further, in some instances the route of administration may be *in ovo.* For example, in ovo vaccination, which is a form of embryo vaccination, may be conducted at a predetermined day of embryonic development (ED). In ovo vaccination may occur at various times of embryonic development between about day 15 to day 21. In particular, an in ovo administration may occur on day 18 to day 19 of embryonic development. For example, an in ovo vaccination may occur at approximately day 18 of embryonic development (ED), about 3 days before hatch.

The immunological composition may be administered to avians. For example, a vaccine described herein may be administered to an avian such as a chicken, capon, duck, goose, turkey, pheasant, grouse, quail, swan, squab, and/or pigeon. In particular embodiments, an avian treated with the vaccine described herein is a chicken.

Dosing regimens may be used to improve the economics of avian husbandry. For example, immunogenic compositions, such as vaccines may be administered to avians at different life stages in an effort to protect avian chicks, avian adults, or both.

In some embodiments, a dosing regimen may be developed to deliver effective amounts of viral vector sufficient to induce a desired effect in a given predetermined population and/or individual animals, such as reducing incidence of a disease in a given population, for example, vaccinated avians, reducing severity of and/or inhibiting symptoms of a targeted disease, and/or inducing an immune response in a population, an animal, and/or their progeny.

Determinations with respect to dosing regimens may be related to the desired results, components selected for use in the immunogenic composition, administration route, such as parenteral, subcutaneous, and/or in ovo administration, number or doses delivered, for example, a single administration or multiple doses (e.g., one or more boosters), and/or the specific properties of the animal or animal population to be treated, for example, the age, size, health status and/or condition of animals.

As stated above, treatment methods may be different based on the outcome desired. For example, an in ovo administration, as well as a subcutaneous administration to chickens, may be used to help provide or establish herd immunity in a flock, to inhibit and/or prevent conditions, and/or clinical symptoms related to the targeted avian viruses.

A dosing regimen may include vaccinating avians, such as chickens with at least one dose of an immunogenic composition as described herein. Doses of the immunogenic compositions may be in a range from about 0.01 ml to about 0.5 ml. For example, a dose of the immunogenic composition as described herein may be administered subcutaneously as a dose of about 0.2 ml. In some instances, when using in ovo administration doses may be in a range of about 0.05 ml.

Dosing regimens may also include guidance on administration routes and/or times by route of exposure. For example, it may be desirable to deliver a dose of an immunogenic composition to an avian via a subcutaneous route of exposure at a specific age, in particular, at about within the first week of hatching. In some instances, avians may be administered immunogenic compositions at an age of about 1 day subcutaneously.

In another embodiment, the immunogenic composition may be delivered to an egg, resulting in an in ovo route of administration. For example, it may be desirable to deliver a dose of an immunogenic composition to an avian via an in ovo route of exposure at a specific age of the egg, in particular, at about day 18 to about day 19 of embryonic development depending on the immunogenic compositions and desired results.

Multiple doses of immunogenic compositions may be administered in a dosing regimen. In some embodiments, two or more doses of vaccine may be given to avians.

An immunogenic composition may include an MDV-3 recombinant virus vector that has and is capable of expressing *in vivo* an exogenous avian influenza virus HA antigen, and an exogenous nucleic acid molecule encoding a Newcastle Disease Virus F antigen. In some instances, the avian influenza is of subtype H5.

A recombinant virus vector may be constructed by a recombination method comprising transfecting a cell with MDV and an insertion plasmid for the MDV Intergenic 1 (IG1) site comprising a SV40 poly A tail, the exogenous nucleic acid molecule encoding avian influenza HA, an Internal Ribosome Entry Site (IRES), the exogenous nucleic acid molecule encoding the Newcastle Disease Virus F antigen and a mouse CMV promoter, flanked by sequences from the IG1 region.

For example, a method for producing an MDV-3 recombinant virus vector that may include transfecting a cell with MDV-3 and an insertion plasmid for the MDV Intergenic 1 (IG1) site comprising a SV40 poly A tail, the exogenous nucleic acid molecule encoding avian influenza HA, an Internal Ribosome Entry Site (IRES), the exogenous nucleic acid molecule encoding the Newcastle Disease Virus F antigen, and a mouse CMV promoter, flanked by sequences from the IG1 region. After production of the MDV-3 recombinant virus vector, it may be recovered and/or purified.

Immunogenic compositions may include a veterinarily acceptable carrier such as solvents, dispersion media, coatings, adjuvants, stabilizing agents, diluents such as sucrose, casein hydrolysate, dipotassium phosphate, potassium dihydrogen phosphate, phenol red sodium salt, sodium hydroxide, and/or water, excipients such as dimethyl sulfoxide, medium, serum such as fetal calf serum or calf serum, sodium hydrogen carbonate, phenol red, hydrochloric acid, and/or purified water, preservatives, antibacterial agents, antifungal agents, isotonic agents, adsorption delaying agents, and combinations thereof.

The immunogenic composition when administered to an avian, may be sufficient to induce a desired response, such as reduction and/or inhibition of clinical systems of the targeted disease(s), immune response in the avian against Marek's disease virus, avian influenza virus and/or Newcastle disease virus.

A method for inducing an immune response in an avian against Marek's Disease Virus, Avian Influenza and Newcastle Disease Virus, comprising administering to the avian an effective amount of the recombinant virus vector, or an effective amount of the composition.

A plasmid for insertion into an MDV-3 (i.e., HVT) Intergenic 1 (IG1) site comprising a SV40 poly A tail, an exogenous nucleic acid molecule encoding avian influenza HA, an Internal Ribosome Entry Site (IRES), and an exogenous nucleic acid molecule encoding the infectious bursal disease virus VP2 antigen.

A plasmid for insertion into an MDV-3 (i.e., HVT) Intergenic 1 (IG1) site comprising a SV40 poly A tail, an exogenous sequence encoding avian influenza HA, an Internal Ribosome Entry Site (IRES), an exogenous nucleic acid molecule encoding the Newcastle Disease Virus F antigen and a mouse CMV promoter, flanked by sequences from the IG1 region. In some embodiments, the plasmid may include an exogenous sequence that encodes avian influence HA of subtype H5. Further, a plasmid may include an exogenous sequence encoding the avian influenza HA antigen that is codon optimized.

A method of vaccinating an animal or inducing an immunogenic or protective response in an animal, that includes at least one administration of the composition or viral vector as described herein.

Constructs for a live MDV (i.e., vHVT) vaccine vector may include a H5 virus gene insert. In some embodiments, embodiments of a viral vector may include multiple gene inserts of H5. A COBRA construct may include both Avian Influenza and Marek's Disease, Serotype 3. For example, vaccines that include such a COBRA construct may be administered at 1 day of age by the subcutaneous route at the target dose of 2,500 plaque-forming units (PFU) of Marek's disease vaccine in 0.2 mL per bird.

Further, an embodiment of a vaccine vector may include antigens derived from another avian influenza clades such as H9 and/or H7.

Vaccinated birds may remain clinically healthy with no clinical signs from the recombinant MDV-3 H5 vector (e.g., rHVT-H5) vaccination and/or following a challenge. Vaccinated birds may seroconvert prior to any challenge, for example, a challenge using a homologous Tk/MN/15 virus or a heterologous Egypt/14 challenge virus. For example, after a challenge vaccinated birds may have antibodies to the challenge viruses resulting in detectable antibody titers.

Further, vaccine constructs as described herein may reduce shedding in avians when the vaccinated avians are subsequently exposed to a viral avian disease.

It should be understood that the sequences, polynucleotides, and/or proteins of the invention may differ from the exact sequences illustrated and described herein. Thus, the invention contemplates deletions, additions and substitutions to the sequences shown, so long as the sequences function in accordance with the methods of the invention. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polaralanine, valine, leucine, isoleucine, proline, phenylalanine, methionine, tryptophan; and (4) uncharged polar--glycine, asparagine, glutamine, cysteine, serine threonine, tyrosine. Phenylalanine, tryptophan, and tyrosine are sometimes classified as aromatic amino acids. It is reasonably predictable that an isolated or non-naturally occurring replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the sequences illustrated and described but possessing minor amino acid substitutions that do not substantially affect the immunogenicity of the protein are, therefore, within the scope of the invention.

The invention further encompasses nucleotide sequences encoding functionally and/or antigenically equivalent variants and derivatives of the antigens of the invention and functionally equivalent fragments thereof. These functionally equivalent variants, derivatives, and fragments display the ability to retain antigenic activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan.

In some embodiments, the substitution introduces a conservative change, which replaces the amino acid with another amino acid of similar chemical structure, similar chemical properties, or similar side-chain volume. The amino acids introduced may have similar polarity, hydrophilicity, or hydrophobicity to the amino acids they replace. Conservative amino acid changes are well known in the art. Where amino acids have similar polarity, this can also be determined by reference to the hydropathy scale for amino acid side chains.

Although the present invention and its advantages have been described in detail, it should be understood that various changes, substitutions, and alterations can be made herein without departing from the spirit and scope of the invention as defined in the appended claims.

The present invention will be further illustrated in the following Examples which are given for illustration purposes only and are not intended to limit the invention in any way.

### EXAMPLES

### Example 1 Construction of vHVT509

vHVT509 contains a gene encoding a synthetic hemagglutinin (HA) protein of Avian influenza virus A (subtype H5) designed by computationally optimized broadly reactive approaches (COBRA). The purpose of this protocol was to construct and to characterize a recombinant HVT that contains the mCMV promoter, AIV HA COBRA-C, SV40 polyA tail at recombination region intergenic site 1 of MDV-3 FC-126 (herpes virus of turkey (HVT) Fc126).

The parental virus of the recombinant was MDV-3 FC-126 clone 2 (i.e., HVT Fc126 clone 2). The recombination locus was the intergene 1 locus located in the BamHI-I fragment. The donor plasmid was pCD046-COBRA-C containing mCMV promoter, AIV HA COBRA-C, and a SV40 polyA tail. Recombination occurred *in vitro* in secondary chicken embryo fibroblast (2° CEF) cells. Recombinant selection was accomplished via indirect immunofluorescence assay (IFA) using chicken anti-Avian influenza sera and PCR amplification with specific primers.

Generation of recombinant virus: An *in vitro* recombination (IVR) was performed by co-electroporation of secondary CEF cells using pCD046-COBRA-C as donor plasmid and viral DNA isolated from MDV-3 FC-126 (i.e., HVT Fc126). Co-electroporation was performed using 1×10⁷ secondary CEF in 300 µl Opti-MEM (Gibco cat#31985) and shocked at 150 volts with 950 capacitance in a 2mm electroporation cuvette. The transfected CEF cells were seeded with 2% FBS containing DMEM (Gibco cat #11960) into 96-well plate and incubated at 37 °C, 5% CO₂ incubator for 4-5 days. The cells grown in the 96-well plate were then duplicated into two 96-well plates and incubated for 2 or 3 more days at 37 °C, 5% CO₂ incubator. One set of duplicated 96-well plates was screened for a recombinant virus by IFA using chicken anti-AIV H5N2 sera (Charles River Laboratories, Lot#J0210) and rabbit anti-chicken IgY (IgG) (whole molecule)-FITC conjugated (Sigma cat#F8888, Lot#075M4812V). To obtain a pure population of recombinant virus, CEF from the IFA positive wells in a corresponding 96-well plate were recovered and process purification step.

Purification of Recombinant virus: Recombinant virus purification was conducted by dilution of the population mixture (recombinant and parent virus) in 96-well plate followed by IFA and PCR screen. After the recombinant virus was identified, further dilution was conducted from CEF in corresponding wells of the duplicated plate until only pure recombinant virus is in a well of the plate. Briefly, the wells in corresponding 96-well plate that IFA positive were collected by trypsinization with TrypLE express (Gibco # 12604) and adjusted to 1 mL with 10% FBS-DMEM. From the 1 mL stock, 5-20µl of stock, depending on the number of plaques in the well, were removed and mixed with 3-5×10⁶ CEFs in 20 mL 2% FBS-DMEM and aliquoted into a new 96-well plate in an attempt to have single HVT plaque per well. The 96-well plates were duplicated after 4 days of incubation at 37 °C, 5% CO₂ incubator and each well tested for the presence of recombinant virus by IFA and PCR. The wells that appeared to have more recombinant virus, by comparing the PCR amplification patterns, were harvested, and the purification step repeated as described above until have the pure recombinant populations.

PCR Analysis of recombinant virus: Viral DNA was extracted from vHVT509 by QIA DNeasy Blood & Tissue Kit (Qiagen cat#69506). PCR primers were designed to identify the presence of the AIV HA COBRA-C, the mCMV promoter, SV40 polyA tail, the flanking recombination arms of HVT virus. PCR amplifications were preformed using ~200ng of DNA template along with the primer pairs and PCR conditions indicated the table below.

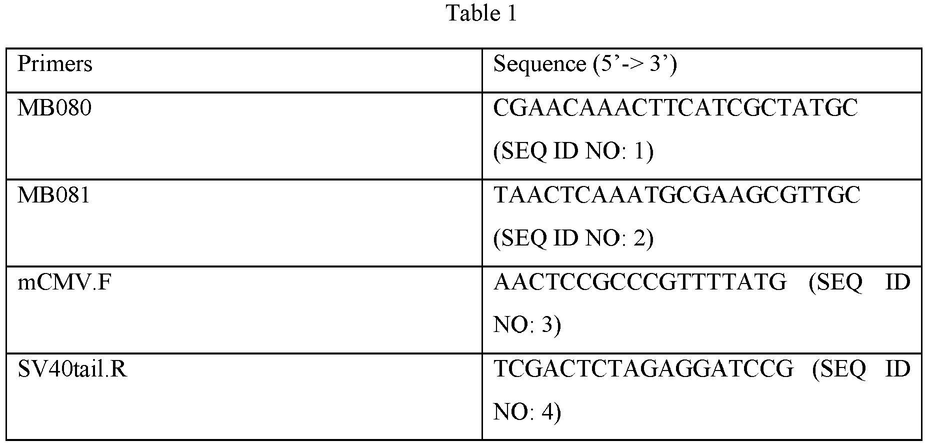

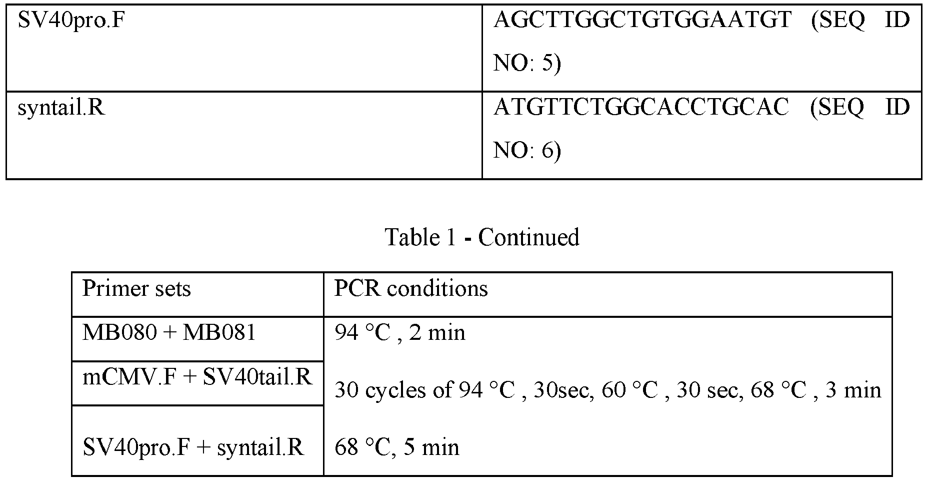

Expression analysis: Indirect immunofluorescence assay (IFA) was performed on the vHVT509. The CEFs that inoculated with vHVT509 were fixed with ice-cold 95% acetone for five minutes at room temperature and air-dried for 10 min. After rehydrating the plate with PBS, two primary antibodies 1) chicken anti-AIV H5N2 sera (Charles Rivers Laboratories, Lot#J0210) at 1:500 dilution and 2) L78 monoclonal antibody against HVT (Merial Select, Gainesville, GA) at 1:3000 dilution were added and incubated for 1 hour at 37 °C. After three washes with PBS, two secondary antibodies, 1) rabbit anti-chicken IgG-FITC (Sigma cat#F8888, lot#075M4812V) at 1:500 dilution and 2) donkey anti-mouse IgG-Alexa Fluor 568 (Molecular Probe #A10037, lot#1752099) at 1:300 dilution were added. The plates were incubated at 37 °C for 1 hour and followed by three times with PBS. The cells were observed to identify the IFA positive plaques with a fluorescent microscope using fluorescein isothiocyanate (FITC)- and tetramethylrhodamine isothiocyanate (TRITC)-filters of Nikon Eclipse Ti inverted microscope.

Genomic DNA analysis: The genomic DNA of vHVT509 was conducted by nucleotide sequence determination of part of recombination arm region as well as the inserted gene cassette. Primer set, MB080 and MB081 were used to amplify the entire inserted gene cassette including part of recombination arm. PCR amplification was performed as follows: 94 °C for 2 min, 30 cycles of 94 °C for 30 sec, 60 °C for 30 sec, 68 °C for 3 min, and final extension at 68 °C for 5 min. The 3.6 kb PCR product was purified using QIAquick Gel Extraction Kit (Qiagen #28706) as per the manufacturer's instruction. Total eight PCR reactions of 25 µl per reaction were pooled and purified using the kit. The final elution was confirmed by agarose gel electrophoresis.

Serial Passages for Genetic Stability Analysis: The pre-MSV stocks (p.3) were passed into secondary CEF by blind passages. Briefly, 100 µl of previous passage have been inoculated into CEF monolayers in T25 flask. The cells were incubated in 37 °C, 5% CO₂ incubator until it makes good size of virus plaques (typically 3-4 days). The virus infected CEF was collected by trypsinization with TrypLE express (Gibco # 12604) and adjusted to total 1 mL with 10% FBS-DMEM and 10% DMSO for LN2 storage. Approximately 50-100 µl of the stock was passed to fresh CEF culture in T25 flask. Total 13 passages (X+12) from pre-MSV (X-1) have been conducted. *In vitro* characterization of X+12 stocks was conducted for PCR screening, the nucleotide sequence analysis of the transgene cassette, and the expression of AIV HA COBRA-C by dual IFA as described earlier.

### Results

Recombinant virus: After two rounds of plaque purification, pure recombinant virus (vHVT509) was isolated. The vHVT509 was tested by IFA and PCR to validate the appropriate transgene insertion as well as no remnant parental virus. The vHVT509 was passed from one well of 96-well plate to T-25 flasks, T-150 flasks, and finally roller bottles (850 cm surface area) to generate pre-MSV stock.

PCR analysis: PCR amplification with various primers listed in Table 1 confirmed that the vHVT509 has expected amplification pattern and amplicon at pre-MSV stock (FIG. 4) and at X+12 passage level (FIG. 5).

**Table 2**

| Primer sets | Expected Amplicons (bp) | |
|---|---|---|
| | vHVT509 | MDV-3 FC-126 (i.e., HVT Fc126) |
| MB080 + MB081 | 3648 | 323 |
| mCMVF + SV40tail.R | 3319 | none |
| SV40pro.F + syntail.R | None | none |

Expression analysis: IFA results indicate that vHVT509 expresses the AIV HA COBRA-C genes in virus-infected CEF (FIG. 6). Over 300 vHVT509 plaques were counted using FITC-filter and TRITC-filter of microscope. The overall expressions of HA COBRA-C gene are matching with HVT plaque at pre-MSV stock and X+12 passage level. Refer to FIG. 7 for the FITC and TRITC-filtered images of the counted virus plaques.

**Table 3**

| Virus | Dual IFA | |
|---|---|---|
| | Anti-AIV H5N2 sera | Anti-HVT mAb |
| | Positive plaques | Positive plaques |
| vHVT509 pre-MSV | 318 | 318 |
| vHVT509 X+12 | 435 | 435 |

Genomic analysis: Analysis of vHVT509 genomic DNA was performed by PCR amplification and followed by nucleotide sequence determination. The primers to amplify the portion of vHVT509 genome and sequence determination were described in the table below. The primer set, MB080 and MB081, were used to amplify the entire mCMV promoter, HA COBRA-C gene, SV40 poly A tail sequences including the part of flanking region (intergene 1 arms). Total 3648 bp PCR amplicon was purified (FIG. 8) and nucleotide sequences were determined using the sequencing primers described in the table below.

**Table 4**

| Primers | Sequences (5' to 3') |
|---|---|
| MB081 | TAACTCAAATGCGAAGCGTTGC (SEQ ID NO: 2) |
| mCMV.F | AACTCCGCCCGTTTTATG (SEQ ID NO: 3) |
| H5coRP1 | TTAGAGGCTTCACTCCATC (SEQ ID NO: 14) |
| H5coFP | ATGGAGAAAATAGTGCTTCT (SEQ ID NO: 15) |
| H5coFP1 | CATCAGGGGTGAGCTCAGC (SEQ ID NO: 16) |
| H5coFP2 | GCCCCAAATATGTGAAATC (SEQ ID NO: 17) |
| MB080 | CGAACAAACTTCATCGCTATGC (SEQ ID NO: 1) |

Sequencing reactions were conducted by the Eurofins MWG Operon (www.operon.com) and the contig (FIG. 9 & FIG. 10) was generated using Contig Express module of Vector NTI Advance 11.5 package.

The nucleotide sequences of vHV509 pre-MSV and X+12 passage stock contig and sequencing primer location are shown in FIG. 11. Sequencing primers are underlined with direction of the primer. Black = recombination arm regions, Blue = mCMV promoter, Red = AIV HA COBRA-C, Gray = synthetic poly A, Underline = Sequencing primer. The recombination arms are colored black and the AIV HA COBRA-C gene is colored red.

FIG. 12 shows the amino acid sequence of AIV HA COBRA-C gene in vHVT509.

FIG. 13 shows in text format of the nucleotide sequence of vHVT509.

Conclusions: The data from PCR screening, and nucleotide analysis of vHVT509 indicated that the mCMV promoter, AIV HA COBRA-C sequences, SV40 poly A tail cassette were inserted between intergene 1 arm of MDV-3 FC-126 (i.e., HVT Fc126) genome. The vHVT509 expresses the AIV HA COBRA-C protein in virus infected CEF. The PCR screening and sequence analysis of vHVT509 X+12 samples indicate that the vHVT509 have maintained the transgene cassette after serial passages. Also, vHVT509 have expressed the HA COBRA-C protein stably after passages (at X+12 level).

### Example 2 Construction of vHVT522

vHVT522 comprises a gene encoding a synthetic hemagglutinin (HA) protein of Avian Influenza virus A (subtype H5) designed by computationally optimized broadly reactive approaches (COBRA). A synthetic Infectious Bursal Disease Virus structural capsid protein VP2.

The purpose of the protocol was to construct an insertion plasmid for the Intergenic I (IG1) site that will contain a SV40 poly A tail, COBRA-C (H5 HA protein), Internal Ribosome Entry Site (IRES), and Infectious Bursal Disease Virus capsid protein (IBDV VP2) for homologous recombination into Vaxxitek virus right IG1 Arm and IBDV VP2.

The donor plasmid was pVP2-IRES [MOD]-COBRA-C, whose backbone is composed of a synthetically ordered plasmid containing the right Intergenic I arm of Vaxxitek virus, SV40 poly A tail, COBRA-C (H5 HA protein), IRES, and Infectious Bursal Disease Virus capsid protein. The plasmid contains a mCMV promoter from the parent virus. Recombination occurred *in vitro* in secondary chicken embryo fibroblast (2° CEF) cells. Recombinant selection was accomplished via indirect immunofluorescence assay (IFA) using chicken anti-Avian influenza sera and PCR amplification with specific primers. Plasmid diagrams are shown in FIG. 14 and FIG. 15.

The plasmid pVP2-IRES-Cobra-C was previously created by cloning a synthetic COBR-C fragment (Genscript Lot U5872BA260-3) into pVP2-IRES-Cobra-A. The pVP2-IRES-Cobra-C plasmid was further modified by replacing the HindIII-BlpI fragment by a new synthetic fragment VP2+COBC to remove an intervening stretch of sequence between the end of IRES sequence and start of COBRA-C sequence present in pVP2-IRES-Cobra-C. The new and correct donor plasmid is called pVP2-IRES[MOD]-Cobra-C. This plasmid is used to generate recombinant virus.

Recombinant generation: An *in vitro* recombination (IVR) was performed by co-electroporation of 2° CEF cells using a linearized pVP2-IRES [MOD]-COBRA-C as the donor plasmid and viral DNA isolated from vHVT013. Co-electroporation was performed using 1×10⁷ 2° CEF in 300 µl Opti-MEM and shocked at 150 volts with 950 capacitance in a 2mm electroporation cuvette. The transfected cells were seeded into 96-well plate and incubated for 4 days. The cells grown in the 96-well plate were then duplicated into two 96-well plates. One set of 96-well plates was used for IFA using chicken polyclonal sera against AIV H5 HA to identify positive wells containing recombinants and another set of 96-well plates was used for recovering the infected cells from the corresponding positive wells.

Purification of the recombinant: The recombinant viral purification methods were performed first by 96-well plate duplication and IFA selection for the wells containing the most IFA positive plaques with the least amount of IFA negative plaques. Wells matching those criteria were then harvested and adjusted to 1 mL in DMEM+2% FBS. From the 1 mL stock 10-20 µl was removed and mixed with 1×10⁷ 2° CEFs in 10 mL DMEM+2% FBS and aliquoted onto a new 96-well plate in an attempt to have single HVT plaques per well. The supernatant of the wells that contained single plaques were tested for the absence of parental virus by PCR. This process was repeated for few rounds until all tested wells showed pure recombinant banding patterns and no parental virus. Four of the PCR pure wells were harvested and frozen (P0), vHVT-VP2-IRES[MOD]-COBRA C #2, #5, #6 and #12. Out of the 4, two of the sister clones (#5 and #6) were expanded into T-25 flask (P1) and eventually T-150 flask (P2). One of the two at P2 stages of expansion, vHVT-VP2-IRES[MOD]-COBRA-C, #6, was then expanded in to 3X 850 cm2 roller bottles, harvested and frozen as the P3, pre-MSV stock, (vHVT-522 IBD-AI (P3) #6 11-May-2018 AK). Twelve serial passages were performed in T-25 flasks beyond the pre-MSV stock (X+12).

PCR analysis: Viral DNA was extracted from vHVT522 (pre-MSV) and vHVT522 X+12 by QIA DNAeasy Blood & Tissue Kit (Qiagen cat#69506). PCR primers were designed to specifically identify the presence of the H5 HA COBRA-C coding sequence that should be present only in the recombinant virus but absent from the parental vHVT013 virus. PCR primers were also designed to amplify the entire cassette, including part of the recombination arm, promoter, and recombinant genes to confirm by sequencing the integrity of virus (Table 5). PCR was performed using 500 ng of DNA template along with the specified primer pairs indicated in Table 1. PCR cycling conditions are as follows: 94 °C - 2 mins; 40 cycles of 94 °C - 30 secs, 58 °C - 45 secs, 68 °C - 1 or 6 mins; 68 °C - 5 mins (the extension times were adjusted according to the expected product size).

Expression analysis: Indirect immunofluorescence assay (IFA) was performed on the vHVT522. The CEFs that were inoculated with vHVT522 were fixed with ice-cold 95% acetone for 20 minutes at -20 °C and air-dried for 10 min. The plates were then rehydrated with PBS and two sets of dual staining were performed to check simultaneous expression of antigens in each viral plaque. In one set, two primary antibodies, chicken Infectious Bursal Disease (IBD) sera (Charles Rivers Laboratories, Lot. G0117) at 1:500 dilution and L78.2 monoclonal antibody against HVT (Boehringer Ingelheim Animal Health, BIAH, Gainesville, GA) at 1:3,000 dilution was added to check expression of VP2 and MDV-3 (HVT) antigens. In the second set, two primary antibodies, H5N2 Chicken serum Inactivated (Charles River Laboratories, Lot. J0210) at 1:300 dilution and L78.2 monoclonal antibody against HVT (Boehringer Ingelheim Animal Health, BIAH, Gainesville, GA) at 1:3,000 dilution was added. All plates with primary antibodies were incubated for 1 hour at 37 °C. After three washes with PBS, two secondary antibodies, Alexa Fluor 568 Goat anti-Chicken IgG (Invitrogen, Cat.# A11041, lot#1868220) at 1:300 dilution and Anti-mouse IgG-FITC (Sigma, Cat.# F2012-1 mL, lot#5LBG3032) at 1:300 dilution were added. The plates were incubated at 37 °C for 1 hour and followed by three washes with PBS. The cells were observed to identify the IFA positive plaques with a fluorescent microscope using fluorescein isothiocyanate (FITC)- and tetramethylrhodamine isothiocyanate (TRITC)-filters of Nikon Eclipse Ti inverted microscope.

Genomic analysis: The genomic DNA of vHVT522 was conducted by nucleotide sequence determination of part of recombination arm region as well as the inserted gene cassette. Primer set, MB080 and MB081 were used to amplify the entire inserted gene cassette including part of recombination arm. PCR amplification was performed as follows: 94 °C for 2 min, 30 cycles of 94 °C for 30 sec, 58 °C for 45sec, 68 °C for 6 min, and final extension at 68 °C for 5 min. The 5.6 kb PCR product was gel purified using Qiagen Gel Extraction Kit (Cat. No. 28706, Lot. #157036708) and the entire fragment was sequenced using the sequencing primers listed in the table below.

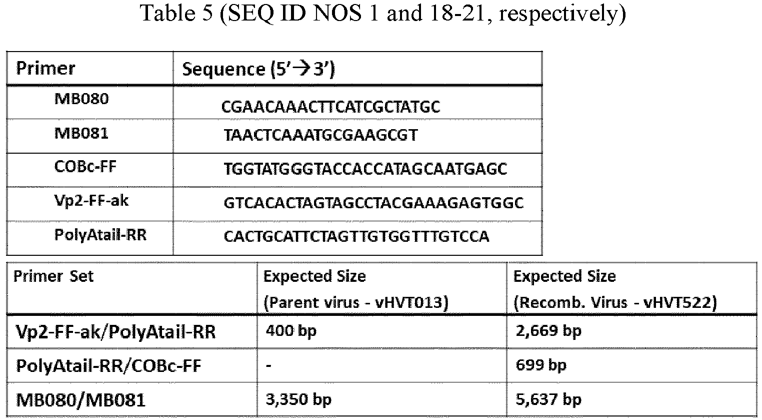

Southern Blot Analysis: Total genomic DNA was extracted from vHVT013-69 TC5 (pre-MSV of Vaxxitek), vHVT522 pre-MSV, and vHVT522 pre-MSV+13 by first transferring the material to a 50 mL conical tube and centrifuging at 1500 rpm for 10 minutes. For southern blot, the pre-MSV material was passaged one more time beyond the 12 passage (hence pre-MSV +13) in order to harvest enough cells for viral genomic DNA extraction. The cell pellets were resuspended with 1 mL of PBS. 14 mL of cell lysis buffer was added along with 150 µL of proteinase K (20 mg/mL) and incubated for 2 hours at 55 °C. After the incubation 600 µL of 5M NaCl was added. An equal total volume of phenol was added and gently shook for several minutes. The material was then centrifuged at 3,000 rpm for 10 minutes. The aqueous phase (upper phase) was transferred into a new 50 mL tube and the extraction was repeated two more times with chloroform as described above. The final aqueous phase was transferred into a polycarbonate centrifuge tube and 30 mL of 100% ethanol was added. After gently mixing, the solution was placed at -20 °C overnight. The next day the solution was centrifuged at 20,000 rpm for 30 minutes. The supernatant was poured off and the DNA pellet was rinsed with 70% ice-cold ethanol. The DNA pellet was air dried for 10 minutes. 500 µL of 20mM HEPES was added to the dried pellet. The total genomic DNA was stored at 4 °C.

For each restriction digest, 10 µg of genomic DNA (3 µg for the donor plasmid) was used in at a concentration of a 1 µg DNA per 10 µL of restriction digestion mix. The genomic DNA of vHVT013 (negative control), pVP2-IRES[MOD]-COBRA-C donor plasmid, vHVT522 pre-MSV, and vHVT522 pre-MSV+13 were each digested overnight at 37 °C with BamHI, EcoRV, and NotI restriction endonucleases. After restriction digestion, the amount of genomic DNA and donor plasmid was first equilibrated by performing a dot blot and probing for VP2 and NDV-F. A total of 3 µg of vHVT522 pre-MSV, 2 µg of vHVT522 pre-MSV+13, 1 µg of vHVT013 and 1 ng of donor plasmid in total 40 uL of 1X loading buffer were loaded on to 1% agarose gel and run at 25V overnight in cold-room. Each of the digestion and run was duplicated for each of the probe used.

The agarose gel was placed into depurination solution, denaturation solution, and neutralization solution, each for 7 minutes. A vacuum transfer station set a 5Hg was used to transfer the DNA from the agarose gel onto a positively charged Nylon membrane for about 2 hours. After transfer, the membrane was treated with 0.4M NaOH and then neutralized with SSC-HCL buffer. The membrane was then air dried and UV cross-linked. The membranes were pre-hybridized for 1hr and then hybridized with a probe over night at 55 °C by following the manufacturers' instructions for North2South Chemiluminescent Hybridization and Detection Kit (Thermo Scientific).

The probes used were isolated by digestion from a donor plasmid pVP2-IRES[MOD]-COBRA-C. The VP2 probe was produced by digestion of the donor plasmid with MScI and NotI restriction endonucleases and gel purifying the 1.3 Kb fragment. The H5 Probe was produced by digestion of the donor plasmid with NcoI and NotI restriction endonucleases and gel purifying the 1.7 Kb fragment. Each of the bands were gel extracted and purified using Qiagen's Gel Extraction kit (Qiagen, cat# 28706). By following the manufacturers' instructions of the North2South Biotin Random Prime Labeling Kit (Thermo Scientific), the gel extracted products were labeled through the incorporation of biotinylated nucleotides. One membrane was probed with VP2 and the other with H5 probe. Following the overnight hybridization, several stringency washes were performed until the membranes were placed in blocking buffer and then after 30 minutes the addition of Streptavidin-HRP to the blocking buffer. After rinsing the membranes of any unbound Streptavidin-HRP the substrate solution of luminal and peroxide were added. The membranes were then placed in the Biorad Chemidoc Imaging System^{™} and images were captured with chemiluminescent detection mode.

### Results

PCR Analysis: PCR analysis of vHVT522 pre-MSV (X) and pre-MSV+12 (X+12) was performed using the PCR primers listed in Table 5 and shown on the vHVT522 map in FIG. 19. As shown in FIG. 20, the sizes of the PCR products after gel electrophoresis correspond well with the expected sizes and the banding patterns. There is no evidence of the parental vHVT013 virus in vHVT522 in FIG. 20.

Expression Analysis: Recombinant vHVT522 viral plaques were visualized using both the TRITC and FITC filters for the dual staining. The FITC filter (green) shows HVT expression, expected in all viral plaques, whereas TRITC filter (red) shows expression of the recombinant antigens, either VP2 or H5 HA, depending on the type of primary antibody used for staining. Over 300 plaques were counted comparing FITC-HVT and TRITC-VP2 or FITC-HVT and TRITC-H5 HA in both vHVT522 (P3) Pre-MSV stock as wells as vHVT522 X+12, that was passed *in vitro* for 12 rounds beyond the P3 (pre-MSV) stage. A summary of the plaque count is shown in Table 6 and representative figure is shown in FIG. 21 All plaques observed were positive for dual staining.

**Table 6**

| Double staining | vHVT522 (P3) pre-MSV | vHVT522 X+12 |
|---|---|---|
| HVT / IBDV VP2 | 398/ 398 | *461*/*461* |
| HVT / H5 HA | 420/420 | 413 / 413 |

Genomic Analysis: The genomic DNA of vHVT522 pre-MSV Stock (P3) and X+12 was used as a template to amplify part of recombination arm region as well as the entire inserted gene cassette using the primer set MB080 and MB081. The 5.6 kb PCR product was gel purified and the entire fragment was sequenced using the sequencing primers listed in Table 7 and shown on schematically in FIG. 22

**Table 7 (SEQ ID NOS 22, 15, 23-25, 1, 18, 26, 3, and 27-36, respectively)**

| **Primer Name** | **Sequence (5' → 3')** |
|---|---|
| H5coFFP1 | GAAGCTCTGCGATCTAGATGG |
| H5coFP | ATGGAGAAAATAGTGCTTCT |
| H5coFP1 | GAGCTCAGCATGTCCATACC |
| H5coFP2 | TCAAACAGATTAGTCCTTGCG |
| H5coRP | TCCAATGGATCGTTACAATGC |
| MB080 | CGAACAAACTTCATCGCTATGC |
| MB081 | TAACTCAAATGCGAAGCGT |
| mCMV-Ak1CORR | TCTAGAACTCGTCGATCGCAGC |
| mCMVF | AACTCCGCCCGTTTTATG |
| mCMVP1 | TCAGCACG GTCATGCACTCT |
| VP2-AK1 | GAGATAACCCAGCCAATCACATCC |
| VP2-AK2 | ATGGTAGCTACATGCGACAGC |
| VP2-AK3 | CTACTGCAGACTAGTGAGTCGG |
| VP2-AK4 | GCTCTGCAGTGTGTAGTGAGC |
| VP2-F | AGATTGCAGGAGCATTTGGCT |
| IRES-F | CCTGGCGACAGGTGCCTC |
| H5coRP2 | GCTGGACTGGCCTTCTCC |
| H5coRP1 | TTGTGTCAACCTGCTCTGTCG |
| IRES-R | TTGAATACGCTTGAGGAGAGCC |

PCR using the following templates: vHVT522 pre-MSV (lane 1), vHVT522 pre-MSV+12 (lane 2) were set up using primer-pairs MB080 and MB081 to amplify the entire inserted gene cassette including part of recombination arm. The 5.6kb PCR product was gel extracted and used for sequence analysis using the primers listed on Table 7. Sequence analysis was performed by Eurofins MWG Operon LLC.

Sequencing contigs were assembled from the sequencing reactions from the primers indicated on Table 7. Refer to FIG. 24 for vHVT522 pre-MSV and FIG. 25 for vHVT522 pre-MSV+12 sequencing contigs.

Sequencing results obtained from vHVT522 pre-MSV+12 were aligned using Vector NTI software to form an alignment. The alignment matches the exact sequence as vHVT522 pre-MSV.

Southern Blot Analysis: Southern blot analysis was performed probing for the VP2 and H5 gene fragments. FIG. 30 represents the map of the pVP2-IRES [MOD]-COBRA-C plasmid that was used to prepare the two probes used in the southern blots. This plasmid contains both the H5 and VP2 genes that are in vHVT522. The VP2 probe, 1.3 Kb is flanked by MscI and NotI and the H5 probe, 1.7 Kb is flanked by NcoI and NotI restriction sites. After digestion of the plasmid by each pair of restriction endonucleases, each fragment was gel extracted and used as template to prepare a biotinylated DNA probe.

The expected size of DNA fragments obtained by restriction digestion of each construct was calculated using the Vector NTI software. As shown in FIG. 34 and FIG. 35 the expected sizes obtained correspond to the actual band obtained after developing the southern blot. The patterns of specific DNA fragments detected in both Pre-MSV and Pre-MSV+13 genomic DNA of vHVT522 are identical. However, there is what appears to be a prominent smear (shown in red arrow) between 1 and 1.6 kb marker, that doesn't change pattern with the restriction endonuclease used, which probably is arising from partially degraded genome.

Conclusions: Based on PCR, Immunofluorescence, and sequence analysis of vHVT522, there is no evidence of parental vHVT013 remaining. The data demonstrates that vHVT522 is a recombinant HVT expressing the VP2 gene from IBDV and a H5 gene (low path) from AIV under the control of mCMV promoter (VP2) and an IRES sequence (HA). The data also demonstrate that vHVT522 is stable genetically and phenotypically at 12 passages beyond the pre-MSV.

### Example 3

### Construction of vHVT523

vHVT523 comprises a gene encoding a synthetic hemagglutinin (HA) protein of Avian influenza virus (AIV) A (subtype H5) designed by computationally optimized broadly reactive approaches (COBRA). This particular protein is also called in this document as COBRA-C for **C**omputationally **O**ptimized **B**roadly **R**eactive **A**ntigen-version **C**. vHVT523 also comprises a synthetic Newcastle Disease Virus (NDV) fusion protein (F).

The purpose of this protocol was to construct a recombinant HVT virus of which the Intergenic I site in the BamHI I fragment will contain an mCMV promoter, NDV fusion protein F, Internal Ribosome Entry Site (IRES), HA protein of Avian Influenza virus (Subtype H5, COBRA-C) and an SV40 poly A tail.

The donor plasmid was pFwt-IRES[MOD]-COBRA-C, whose backbone is composed of a plasmid containing synthetic DNA fragment from the Intergenic I arm of vHVT013 virus, SV40 poly A tail, COBRA-C (H5 HA protein), IRES, NDV-F protein and mCMV promoter. Recombination occurred *in vitro* in secondary chicken embryo fibroblast (2° CEF) cells. Recombinant selection was accomplished via indirect immunofluorescent assay (IFA) using chicken anti-Avian influenza sera and PCR amplification with specific primers.

The plasmid pFwt-IRES-COBRA-C was first constructed by replacing the Saudi-Arabia H9 gene in a plasmid that was created previously (pFwt-IRES-SaH9opt) with COBRA-C gene extracted from pVP2-IRES-COBRA-C. The COBRA-C gene was extracted from pVP2-IRES-COBRA-C by using the restriction enzymes using SalI and HindIII and cloned into pFwt-IRES-SaH9opt that was also digested with same enzymes. The pFwt-IRES-Cobra-C plasmid was further modified by replacing the HindIII-BlpI fragment by a new synthetic fragment F+COBC to remove an intervening stretch of sequence between the end of IRES sequence and start of COBRA-C sequence present in pFwt-IRES-Cobra-C. The new and correct donor plasmid is called pFwt-IRES[MOD]-Cobra-C. This plasmid is used to generate recombinant virus. Diagrams of the plasmid construction are shown in FIG. 36 and FIG. 37

Recombinant virus generation: An *in vitro* recombination (IVR) was performed by co-electroporation of 2° CEF cells using a linearized pFwt-IRES [MOD]-COBRA-C as the donor plasmid and viral DNA isolated from vHVT013. Co-electroporation was performed using 1×10⁷ 2° CEF in 300µl Opti-MEM and shocked at 150 volts with 950 capacitance in a 2mm electroporation cuvette. The transfected cells were seeded into 96-well plate and incubated for 4 days. The cells grown in the 96-well plate were then duplicated into two 96-well plates. One set of 96-well plates was used for IFA using chicken polyclonal sera against AIV H5 HA to identify positive wells containing recombinants and another set of 96-well plates was used for recovering the infected cells from the corresponding positive wells.

Purification of the recombinant: The recombinant viral purification methods were performed first by 96-well plate duplication and IFA selection for the wells containing the most IFA positive plaques with the least amount of IFA negative plaques. Wells matching those criteria were then harvested and adjusted to 1 mL in DMEM+2% FBS. From the 1 mL stock 10-20µl was removed and mixed with 1×10⁷ 2° CEFs in 10 mL DMEM+2% FBS and aliquoted onto a new 96-well plate in an attempt to have single HVT plaques per well. The supernatant of the wells that contained single plaques were tested for the absence of parental virus by PCR. This process was repeated for few rounds until all tested wells showed pure recombinant banding patterns and no parental virus. Five of the PCR pure wells were harvested and frozen (P0), vHVT-Fwt-IRES[MOD]-COBRA-C, R3(P0) #1, #2, #3, #4 and #5. Out of the 5, two of the sister clones (#1 and #4) were expanded into T-25 flask (P1) and eventually T-150 flask (P2). One of the two at P2 stages of expansion, vHVT-Fwt-IRES[MOD]-COBRA-C, #1, was then expanded in to 3X 850 cm2 roller bottles, harvested and frozen as the P3, pre-MSV stock, (vHVT-523 NDV-AI (P3) #1 11-May-2018 AK). Twelve serial passages were performed in T-25 flasks beyond the pre-MSV stock (X+12).

PCR Analysis: Viral DNA was extracted from vHVT523 (pre-MSV) and vHVT523 X+12 by QIA DNAeasy Blood & Tissue Kit (Qiagen cat#69506). PCR primers were designed to specifically identify the presence of the H5 HA COBRA-C coding sequence that should be present only in the recombinant virus but absent from the parental vHVT013 virus. PCR primers were also designed to amplify the entire cassette, including part of the recombination arm, promoter, and recombinant genes to confirm by sequencing the integrity of virus. PCR was performed using 500ng of DNA template along with the specified primer pairs indicated in Table 1. PCR cycling conditions are as follows: 94 °C - 2 mins; 40 cycles of 94 °C - 30 secs, 58 °C - 45 secs, 68 °C - 1 or 6 mins; 68 °C - 5 mins (the extension times were adjusted according to the expected product size).

Expression Analysis: Indirect immunofluorescent assay (IFA) was performed on the vHVT523. The CEFs that were inoculated with vHVT523 were fixed with ice-cold 95% acetone for 20 minutes at -20 °C and air-dried for 10 min. The plates were then rehydrated with PBS and two sets of dual staining were performed to check simultaneous expression of antigens in each viral plaque. In one set, two primary antibodies, chicken Newcastle Disease virus (ND) sera (Charles Rivers Laboratories, Lot. C0117A) at 1:300 dilution and L78.2 monoclonal antibody against HVT (Boehringer Ingelheim Animal Health, BIAH, Gainesville, GA) at 1:3,000 dilution were added to check expression of NDV-F and HVT antigens, respectively. In the second set, two primary antibodies, H5N2 Chicken serum Inactivated (Charles River Laboratories, Lot. J0210) at 1:300 dilution and L78.2 monoclonal antibody against HVT (Boehringer Ingelheim Animal Health, BIAH, Gainesville, GA) at 1:3,000 dilution were added. All plates with primary antibodies were incubated for 1 hour at 37 °C. After three washes with PBS, two secondary antibodies, Alexa Fluor 568 Goat anti-Chicken IgG (Invitrogen, Cat.# A11041, lot#1868220) at 1:300 dilution and Anti-mouse IgG-FITC (Sigma, Cat.# F2012-1 mL, lot#5LBG3032) at 1:300 dilution were added. The plates were incubated at 37 °C for 1 hour and followed by three washes with PBS. The cells were observed to identify the IFA positive plaques with a fluorescent microscope using fluorescein isothiocyanate (FITC)- and tetramethylrhodamine isothiocyanate (TRITC)-filters of Nikon Eclipse Ti inverted microscope.

Genomic Analysis: The genomic DNA of vHVT523 was conducted by nucleotide sequence determination of part of recombination arm region as well as the inserted gene cassette. Primer set, MB080 and MB081 were used to amplify the entire inserted gene cassette including part of recombination arm. PCR amplification was performed as follows: 94 °C for 2 min, 30 cycles of 94 °C for 30 sec, 58 °C for 45sec, 68 °C for 6 min, and final extension at 68 °C for 5 min. The 5.9 kb PCR product was gel purified using Qiagen Gel Extraction Kit (Cat. No. 28706, Lot. #157036708) and the entire fragment was sequenced using the sequencing primers listed in the table below.

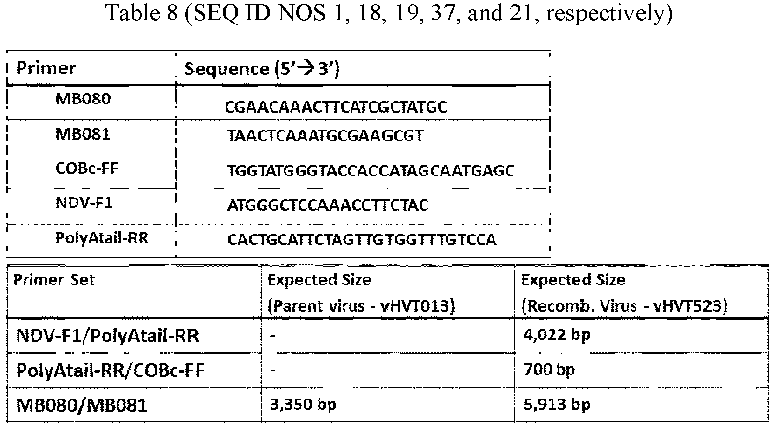

### Results

PCR Analysis: PCR analysis of vHVT523 pre-MSV (X) and pre-MSV+12 (X+12) was performed using the PCR primers listed in Table 8 and shown on the vHVT523 map in FIG. 41 As shown in FIG. 42, the sizes of the PCR products after gel electrophoresis correspond well with the expected sizes and the banding patterns. Further, there is no evidence of the parental vHVT013 virus in vHVT523.

Expression Analysis: Recombinant vHVT523 viral plaques were visualized using both the TRITC and FITC filters for the dual staining. The FITC filter (green) shows HVT expression, expected in all viral plaques, whereas TRITC filter (red) shows expression of the recombinant antigens, either NDV-F or H5 HA, depending on the type of primary antibody used for staining. Over 300 plaques were counted comparing FITC-HVT and TRITC-NDV-F or FITC-HVT and TRITC-H5 HA in both vHVT523 (P3) Pre-MSV stock as wells as vHVT523 X+12, that was passed *in vitro* for 12 rounds beyond the P3 (pre-MSV) stage. A summary of the plaque count is shown in the table below and a representative figure is shown in FIG. 43. All plaques observed were positive for dual staining.

**Table 9**

| Double staining | vHVT523 (P3) pre-MSV | vHVT523 X+12 |
|---|---|---|
| HVT / NDV F | 325/ 325 | *345*/*345* |
| HVT / H5 HA | 412/412 | 423/ 423 |

Genomic Analysis: The genomic DNA of vHVT523 pre-MSV Stock (P3) and X+12 was used as a template to amplify part of recombination arm region as well as the entire inserted gene cassette using the primer set MB080 and MB081. The 5.9 kb PCR product was gel purified and the entire fragment was sequenced using the sequencing primers listed in the table below and shown on schematically in FIG 41.

**Table 10 (SEQ ID NOS 38-41, 22, 15, 42-44, 35, 45, 36, 1, 18, 3, 27, and 46, respectively)**

| **PRIMER NAME** | **SEQUENCE (5' → 3')** |
|---|---|
| **312P2** | TATGCCCAGGGATAAGGAGG |
| **312P4** | CTGGTGGCAATATGGATT |
| **312P6** | CGTCAACAATTCAATGAGCA |
| **CMV-FF** | TTGGCTGAGCTGCGTTCTACG |
| **H5COFFP1** | GAAGCTCTGCGATCTAGATGG |
| **H5COFP** | ATGGAGAAAATAGTGCTTCT |
| **H5COFP1** | ACGAGTTCATCAGGGTACCG |
| **H5COFP2** | AGCTGGGTAATGGTTGTTTC |
| **H5CORP** | ATCGTGTCAACCTGCTTTGT |
| **H5CORP1** | TTGTGTCAACCTGCTCTGTCG |
| **H5CORRP1** | TTCCAACGGCCTCAAACTGAG |
| **IRES-R** | TTGAATACGCTTGAGGAGAGCC |
| **MB080** | CGAACAAACTTCATCGCTATGC |
| **MB081** | TAA CTC AAA TGC GAA GCG T |
| **MCMVF** | AACTCCGCCCGTTTTATG |
| **MCMVP1** | TCAGCACGGTCATGCACTCT |
| **NDVF-R** | GAGACGAAGTGTATACATTGACTGC |

PCR using the following templates: vHVT523 pre-MSV (lane 1), vHVT523 pre-MSV+12 (lane 2) were set up using primer-pairs MB080 and MB081 to amplify the entire inserted gene cassette including part of recombination arm. The 5.6kb PCR product was gel extracted and used for sequence analysis using the primers listed on Table 10. Sequence analysis was performed by Eurofins MWG Operon LLC.

Sequencing contigs were assembled from the sequencing reactions from the primers indicated on Table 10. Refer to FIG. 46 for vHVT523 pre-MSV sequencing contigs.

Sequencing results obtained from vHVT523 pre-MSV were aligned using Vector NTI software to form the above alignment. The alignment created exactly matched to the expected vHVT523 sequence.

Conclusions: Based on PCR, Immunofluorescence, and sequence analysis of vHVT523, there is no evidence of parental vHVT013 remaining. The data demonstrates that vHVT523 is a recombinant HVT expressing the Fusion (F) gene from NDV and H5 gene (low path) from AIV under the control of mCMV promoter (F) and an IRES sequence (HA). The data also demonstrate that vHVT523 is stable genetically and phenotypically at 12 passages beyond the pre-MSV.

### Example 4 Evaluation of the Efficacy of Avian Influenza-Marek's Disease, Serotype 3, Live Marek's Disease Vector Candidates (vHVT522-AI-IBDV. vHVT523-AI-NDV and vHVT509-AI), Administered to One-Day-Old SPF Chickens, Against High-Pathogenicity Avian Influenza Virus Challenges.

The aim of this study was to determine efficacy of Avian Influenza-Marek's Disease, Serotype 3, Live Marek's Disease Vector Candidates, administered to one-day-old SPF chickens, against challenge with three high-pathogenicity Avian Influenza Virus (HPAIV) strains.

The primary variables investigated were: 1. The mortality and typical clinical signs associated with High-Pathogenicity Avian Influenza and 2. The percentage of birds shedding virus and viral load in oropharyngeal swabs.

One hundred and forty-four (144), one-day-old SPF chicks, SPAFAS flock T36 were assigned into 12 groups according to the table below. All birds were vaccinated or sham-vaccinated by the subcutaneous (SQ) route, according to the table below, with vHVT522-AI-IBDV (P5 082918) at, 2,560 PFU's/0.2 mL dose; vHVT523-AI-NDV (P5 082918), at 2,360 PFU's/0.2 mL dose; and vHVT509-AI (vHVT509 X+5 071318), at 2,160 PFU's/0.2 mL dose. Birds were housed by treatment group, 18 birds per unit.

**Table 11**

| **Group** | **Vaccine PFU/dose** | **Route/ Volume (mL)** | **Number Of Birds Placed** | **Number Of Birds at Challenge** | **H5 challenge*** |
|---|---|---|---|---|---|
| 1 | vHVT522 ∼2,500 | 0.2 mL / SQ | 12 | 10 | Minnesota/12582 |
| 2 | vHVT523 ∼2,500 | 0.2 mL / SQ | 12 | 10 | Minnesota/12582 |
| 3 | vHVT509 ∼2,500 | 0.2 mL / SQ | 12 | 10 | Minnesota/12582 |
| 4 | Sham-vaccinated Challenge Controls | 0.2 mL / SQ | 12 | 10 | Minnesota/12582 |
| 5 | vHVT522 ∼2,500 | 0.2 mL / SQ | 12 | 10 | Egypt/N04915 |
| 6 | vHVT523 ∼2,500 | 0.2 mL / SQ | 12 | 10 | Egypt/N04915 |
| 7 | vHVT509 ∼2,500 | 0.2 mL / SQ | 12 | 10 | Egypt/N04915 |
| 8 | Sham-vaccinated Challenge Controls | 0.2 mL / SQ | 12 | 10 | Egypt/N04915 |
| 9 | vHVT522 ∼2,500 | 0.2 mL / SQ | 12 | 10 | Hungary/53433 |
| 10 | vHVT523 ∼2,500 | 0.2 mL / SQ | 12 | 10 | Hungary/53433 |
| 11 | vHVT509 ∼2,500 | 0.2 mL / SQ | 12 | 10 | Hungary/53433 |
| 12 | Sham-vaccinated Challenge Control | 0.2 mL / SQ | 12 | 10 | Hungary/53433 |

| | | | | | |
|---|---|---|---|---|---|
| * The birds were challenged on Study Day 28 at SEPRL, with one A/turkey/Minnesota/12582/2015, Clade 2.3.4.4 - H5N2 [Minnesota/12582]; A/Egypt/N04915/2014, H5N1, Clade 2.2.1 - H5N1 [Egypt/N04915]; or a 3rd strain to be determined, by the intrachoanal route, with ~10^{6.0} EID₅₀ per dose. | | | | | |

The test vaccines used in the study are shown in the table below.

**Table 12**

| | |
|---|---|
| Product True Name | 1. Avian Influenza-Bursal Disease-Marek's Disease, Serotype 3, Live Marek's Disease Vector [vHVT522-AI-IBDV] |
| | 2. Avian Influenza-Newcastle Disease-Marek's Disease, Serotype 3, Live Marek's Disease Vector [vHVT523-AI-NDV] |
| | 3. Avian Influenza-Marek's Disease Vaccine, H5 Subtype, Serotype 3, Live Marek's Disease Vector [vHVT509-AI] |
| Formulation | 1-3. All vaccines were diluted to target -2,500 **PFU**/0.2 mL dose. |
| Manufacturer | Merial Inc. (US Veterinary Biologics Est. 298) |
| Storage Requirements | Liquid Nitrogen |
| Applied dose and Route | 1-3. 0.2 mL per bird, administered by the subcutaneous route (SQ) |

The challenge material used in the study is shown in the table below.

**Table 13**

| Scientific Name | Avian Influenza H5 type |
|---|---|
| Strain/Relation to Test Vaccine | 1. A/turkey/Minnesota/12582/2015, Clade 2.3.4.4 - H5N2 [Minnesota/12582] |
| | 2. A/Egypt/N04915/2014, H5N1, Clade 2.2.1 -H5N1 [Egypt/N04915] |
| | 3. TBD |
| Source of Material | Southeast Poultry Research Laboratory (SEPRL) |
| Procedure for Challenge | At a dilution that will result in approximately 10^{6.0} EID₅₀/0.1 mL dose |
| Storage Requirements | -70°C |
| Applied Dose | ∼10^{6.0} EID₅₀/0.1 mL-dose |
| Route of Administration | 0.1 mL per bird by the intrachoanal route |

The animals used in the study are shown in the table below.

**Table 14**

| Species | *Gallus domesticus* |
|---|---|
| Strain / Breed | SPF chickens |
| Source | SPAFAS |
| Sex | Female/Male |
| Weight | N/A |
| Age | Day-old |
| Number | 144 one-day-old chicks |
| Physiological Status | Healthy and alert |
| Inclusion Criteria | All birds appearing healthy and alert |
| Conditioning / Acclimation | N/A |
| Serologic Status | N/A |
| Exclusion Criteria | Animals that are debilitated, suffering from disease or injury, fractious or otherwise unsuitable for inclusion in the study, in the opinion of the Investigator, were excluded from the study. |
| Animal Identification | The birds were identified according to unit placement and numbered bands. |
| Criteria for Removal from Study | After inclusion in the study, animals that are debilitated, suffering from disease or injury, fractious or otherwise unsuitable to remain in the study, in the opinion of the Investigator, were removed. |

A timeline of the study is shown in the table below.

**Table 15**

| **Study Day or Range** | **Activity** |
|---|---|
| Day 0 | One hundred and forty-four (144), one-day-old SPF chicks were assigned into Groups 1-12 according to Section 2.1. |
| | All birds were vaccinated or sham-vaccinated by the SQ route, according to Sections 1.1 and 2.1. Eighteen (18) birds were housed per unit. Birds from different groups that received the same treatment were housed together while at Merial, divided in two units. |
| Days 24-28 | All groups were reduced to ten (10) birds and neck banded for individual identification with numbered bands (numbered bands were assigned only for birds that were challenged: 10 per group, 120 birds total). Blood was collected via venipuncture from the wing or jugular vein. All groups were transported to SEPRL and placed into isolation units (ten birds per unit, one unit per Group) in the BSL-3 facility at SEPRL. Actual Study Day of activities |
| Day 28 | All the birds were challenged with HPAIV according to Sections 1.3 and 2.1 by the intrachoanal route, 0.1 mL per bird. |
| Day 29-42 | The birds were observed daily for any unfavorable reactions to the challenge and HPAIV clinical signs, including death. |
| Day 30* | Oropharyngeal swabs were collected from all birds (including birds found dead on this study day) and stored in brain and heart infusion media (BHI) at -70C until molecular testing to determine virus shedding is conducted. |
| Day 32* | Oropharyngeal swabs were collected from all the remaining birds (including birds found dead on this study day) and stored in brain and heart infusion media (BHI) at -70C until molecular testing to determine virus shedding is conducted. |
| Day 42 | Blood was collected from all the remaining birds and the birds were terminated. |

| | |
|---|---|
| *Swabs samples were collected only on Study Days 30 and 32, from all birds alive on these days and also from birds found dead on these particular study days. All the other birds that are found dead or are euthanized on any other study day will not be sampled. | |

On Study Day 24, all groups were reduced to ten (10) birds per group and neck banded for individual identification with numbered bands. Blood samples were collected via venipuncture from the wing or jugular vein.

On Study Day 25, all groups were transported to the Southeastern Poultry Research Laboratory (SEPRL) and placed into isolation units, ten (10) birds per unit.

On Study Day 28, all birds were challenged with HPAIV A/turkey/Minnesota/12582/2015; Clade 2.3.4.4; [Minnesota/12582], at 10^{5.9} EID₅₀ per dose (Groups 1-4); A/Egypt/N04915/2014, H5N1, clade 2.2.1; [Egypt/N04915], at 10^{6.5} EID₅₀ per dose (Groups 5-8); or A/domestic turkey/Hungary/53433/2016, Clade 2.3.4.4B - H5N8 [Hungary/53433] at 10^{5.9} EID₅₀ per dose, by the intrachoanal route.

On Study Days 28-42, the birds were observed daily for any unfavorable reactions to the challenge and HPAIV clinical signs, including death. The clinical signs include, but are not limited to, severe depression, nervous or respiratory system signs and/or death. At the end of the observation period (Study Day 42), the survivors were bled for serology and terminated.

On Study Days 30 and 32, Oropharyngeal swabs were collected from all birds (including birds found dead on these sample days); and stored in brain and heart infusion media (BHI) at -70 °C until molecular testing to determine virus shedding is conducted.

On Study Day 42, blood was collected from all the remaining birds and the birds were terminated.

All animals were housed in the same manner. The birds were housed in negative pressure isolation units. Water and feed were given *ad libitum.*

All birds were housed at Merial facilities until transport. Between Study Days 25 to 28, all birds were transferred to SEPRL, where they were placed in BSL-3 approved units for the remainder of the study.

### Analytical Testing

Vaccine: Each experimental vaccine preparation was titrated one to three times to determine the actual dose given (ADG). Titers were determined for all vaccine fractions including Marek's Disease Virus and Avian Influenza gene insert.

Challenge: The challenge viruses were titrated once in eggs to determine the actual challenge dose given.

Serology: The serum collected pre and post-challenge were used in hemagglutination inhibition (HI) assays to determine the antibody levels against selected AIV strains.

Virus Shedding - RT-PCR: HPAIV viral load was tested by real time RT-PCR in the collected swabs by SEPRL. Oropharyngeal swabs were collected on study days 30 and 32 and were stored in BHI media at -70 °C until processing. Viral RNA was extracted using MagMAX^{™}-96 AI/ND Viral RNA Isolation Kit^{®} (Ambion, Inc.) following the manufacturer's instructions. The resulting viral RNA extracts were quantified by one-step qRRT-PCR which targets the influenza matrix gene using 7500 FAST Real-time PCR System (Applied Biosystems, Foster City, CA, USA). The standard curves for viral RNA quantification were established with RNA extracted from dilutions of the same titrated stocks of the challenge viruses. The titer of shedding (Log₁₀/mL) were measured by RT-PCR. A positive swab result for each bird was defined as one or more occurrences of a positive RT-PCR result post challenge in each bird. For analysis, all the negative samples were considered to be lower than the limit of detection established for each virus.

Animal Disposition: All the birds dying during the study and terminated birds at Merial were disposed of by incineration at the experimental farm. Birds that died or terminated at the end of the study at SEPRL were disposed of according to recommended and approved procedures at SEPRL BSL-3 facility.

### Results

The table below shows the number of birds positive for HPAIV and percent protection/infection by study group.

**Table 16**

| **Group** | **Vaccine PFU/dose*** | **H5 challenge**** | **# Positive/ Total # birds** | **% Protection (% Infection)** |
|---|---|---|---|---|
| 1 | vHVT522 2,560 | Minnesota/12582 | 0/10 | 100 |
| 2 | vHVT523 2,360 | Minnesota/12582 | 0/10 | 100 |
| 3 | vHVT509 2,160 | Minnesota/12582 | 0/10 | 100 |
| 4 | Sham-vaccinated Challenge Controls | Minnesota/12582 | 10/10 | (100%) |
| 5 | vHVT522 2,560 | Egypt/N04915 | 0/10 | 100 |
| 6 | vHVT523 2,360 | Egypt/N04915 | 1/10 | 90 |
| 7 | vHVT509 2,160 | Egypt/N04915 | 0/10 | 100 |
| 8 | Sham-vaccinated Challenge Controls | Egypt/N04915 | 10/10 | (100%) |
| 9 | vHVT522 2,560 | Hungary/53433 | 0/10 | 100 |
| 10 | vHVT523 2,360 | Hungary/53433 | 1/10 | 90 |
| 11 | vHVT509 2,160 | Hungary/53433 | 0/10 | 100 |
| 12 | Sham-vaccinated Challenge Controls | Hungary/53433 | 10/10 | (100%) |

| | | | | |
|---|---|---|---|---|
| "All birds were challenged on Study Day 28 at SEPRL, by the intrachoanal route with HPAIV A/turkey/Minnesota/12582/2015; Clade 2.3.4.4; [Minnesota/12582], at 10^{5.9} EID₅₀ per dose (Groups 1-4); A/Egypt/N04915/2014, H5N1, clade 2.2.1; [Egypt/N04915], at 10^{6.5} EID₅₀ per dose (Groups 5-8); or Aldomestic turkey/Hungary/53433/2016, Clade 2.3.4.48 - H5N8 [Hungary/53433] at 10^{5.9} EID₅₀) per dose. | | | | |

A summary of the shedding results is shown in the table below and in FIGs 55-57. Boxplots of the shedding titers for study days 30 and 32 are shown in FIG. 51 and FIG, 52 respectively.

**Table 17**

| **Group** | **Vaccine PFU/dose*** | **H5 challenge**** | **Oropharyngeal Shedding Average EID₅₀/ml* / Std** | |
|---|---|---|---|---|
| | | | **Day 30** | **Day 32** |
| 1 | vHVT522 | Minnesota/12582 | 2.5 / 1.4 | 2.6 / 1.9 |
| | 2,560 | | | |
| 2 | vHVT523 | Minnesota/12582 | 3.1 / 1.3 | 3.8 / 2.2 |
| | 2,360 | | | |
| 3 | vHVT509 | Minnesota/12582 | 1.6 / 0.9 | 1.2 / 0.4 |
| | 2,160 | | | |
| 4 | Sham-vaccinated | Minnesota/12582 | 7.3 / 0.6 | N/A |
| | Challenge Controls | | | |
| 5 | vHVT522 | Egypt/N04915 | 2.9 / 1.4 | 3.0 / 1.4 |
| | 2,560 | | | |
| 6 | vHVT523 | Egypt/N04915 | 3.0 / 1.2 | 3.5 / 1.6 |
| | 2,360 | | | |
| 7 | vHVT509 | Egypt/N04915 | 2.5 / 1.1 | 2.2 / 1.3 |
| | 2,160 | | | |
| 8 | Sham-vaccinated | Egypt/N04915 | 6.6 / 0.6 | N/A |
| | Challenge Controls | | | |
| 9 | vHVT522 | Hungary/53433 | 1.8 / 0.9 | 1.7 / 1.2 |
| | 2,560 | | | |
| 10 | vHVT523 | Hungary/53433 | 2.8 / 1.4 | 2.9 / 1.5 |
| | 2,360 | | | |
| 11 | vHVT509 | Hungary/53433 | 1.3 / 0.0 | 1.4 / 0.4 |
| | 2,160 | | | |
| 12 | Sham-vaccinated | Hungary/53433 | 6.0 / 1.2 | 6.6 / N/A |
| | Challenge Controls | | | |

| | | | | |
|---|---|---|---|---|
| *The limit of detection for the RT-PCR used was 1.1 for Minnesota/12582; 1.7 for Egypt/N04915; and 1.4 for Hungary/53433. All samples with titers lower than 1.1 (Groups 1-4); lower than 1.7 (Groups 5-8); or lower than 1.4 (Groups 9-12) were considered negative. For the purposes of statistical calculations, all the negative samples were assigned the value of "1.0" (Groups 1-4); "1.6" (Groups 5-8); or "1.3" (Groups 9-12) for the calculations **All sham-vaccinated challenged (except one bird in Group 12) were either dead or were euthanized due to clinical signs before the sample collection for Study Day 32. | | | | |

The table below shows the frequency of positive oropharyngeal shedding by day and group.

**Table 18**

| **Group** | **Vaccine PFU/dose*** | **H5 challenge**** | **Oropharyngeal swabs # of Bird Shedding / # of Birds tested** | |
|---|---|---|---|---|
| | | | **Day 30** | Day 32 |
| 1 | vHVT522 | Minnesota/12582 | 6 / 10 | 5 / 10 |
| | 2,560 | | | |
| 2 | vHVT523 | Minnesota/12582 | 9 / 10 | 7 / 10 |
| | 2,360 | | | |
| 3 | vHVT509 | Minnesota/12582 | 5 / 10 | 3 / 10 |
| | 2,160 | | | |
| 4 | Sham-vaccinated | Minnesota/12582 | 10 / 10 | N/A |
| | Challenge Controls | | | |
| 5 | vHVT522 | Egypt/N04915 | 6 / 10 | 6 / 10 |
| | 2,560 | | | |
| 6 | vHVT523 | Egypt/N04915 | 8 / 10 | 7 / 10 |
| | 2,360 | | | |
| 7 | vHVT509 | Egypt/N04915 | 5 / 10 | 2 / 10 |
| | 2,160 | | | |
| 8 | Sham-vaccinated | Egypt/N04915 | 10 / 10 | N/A |
| | Challenge Controls | | | |
| 9 | vHVT522 | Hungary/53433 | 3 / 10 | 1 / 10 |
| | 2,560 | | | |
| 10 | vHVT523 | Hungary/53433 | 7 / 10 | 8 / 10 |
| | 2,360 | | | |
| 11 | vHVT509 | Hungary/53433 | 0 / 10 | 1 / 10 |
| | 2,160 | | | |
| 12 | Sham-vaccinated | Hungary/53433 | 10 / 10 | 1 / 1 |
| | Challenge Controls | | | |

| | | | | |
|---|---|---|---|---|
| *All sham-vaccinated challenged birds were either dead or were euthanized due to clinical signs before the sample collection (except for one bird in Group 12) - no samples collected on Day 32 for Groups 7 and 8. | | | | |

The following table shows a summary of HI results by day and group.

**Table 19**

| **Group** | **Vaccine PFU/dose*** | **HI Log₂GMT*** | | **# of Birds with antibodies / # of Birds tested** | |
|---|---|---|---|---|---|
| | | **Day 24** | **Day 42** | **Day 24** | **Day 42** |
| 1 | vHVT522 | 3.2 | 5.9 | 8 / 10 | 10 / 10 |
| | 2,560 | | | | |
| 2 | vHVT523 | 25 | 6.3 | 3 / 10 | 10 / 10 |
| | 2,360 | | | | |
| | | | | | |

| **Group** | **Vaccine PFU/dose*** | **HI Log₂GMT*** | | **# of Birds with antibodies / # of Birds tested** | |
|---|---|---|---|---|---|
| | | **Day 24** | **Day 42** | **Day 24** | **Day 42** |
| 3 | vHVT509 | 3.3 | 4.7 | 9 / 10 | 10/10 |
| | 2,160 | | | | |
| 4 | Sham-vaccinated | 2.0 | N/A | 0 / 10 | N/A |
| | Challenge Controls | | | | |
| 5 | vHVT522 | 3.5 | 7.5 | 9 / 10 | 10 / 10 |
| | 2,560 | | | | |
| 6 | vHVT523 | 2.9 | 6.8 | 6 / 10 | 9 / 9 |
| | 2,360 | | | | |
| 7 | vHVT509 | 3.8 | 6.0 | 9 / 10 | 10 / 10 |
| | 2,160 | | | | |
| 8 | Sham-vaccinated | 2.0 | N/A | 0 / 10 | N/A |
| | Challenge Controls | | | | |
| 9 | vHVT522 | 3.6 | 5.3 | 10 / 10 | 10 / 10 |
| | 2,560 | | | | |
| 10 | vHVT523 | 2.8 | 6.0 | 6 / 10 | 9 / 9 |
| | 2,360 | | | | |
| 11 | vHVT509 | 4.2 | 5.2 | 10 / 10 | 10 / 10 |
| | 2,160 | | | | |
| 12 | Sham-vaccinated | 2.0 | N/A | 0 / 10 | N/A |
| | Challenge Controls | | | | |

| | | | | | |
|---|---|---|---|---|---|
| "The limit of detection for the HI test was 8. Titers below 8 GMT (3 log2 GMT) were considered negative and are expressed as 4 GMT (2 log2 GMT).The samples were tested against the challenge virus strain for the respective groups (Groups 1-4 [Minnesota/12582]; Groups 5-8 using [Egypt/N04915]; and Groups 9-12 using [Hungary/53433]). All tested samples were used in the average GMT calculations (negative samples were assigned a value of 4GMT (2 log2 GMT) for calculation purposes). **All sham-vaccinated challenged birds were either dead or were euthanized due to clinical signs before the sample collection - no samples collected on Day 42 for Groups 4, 8 and 12. | | | | | |

The following table shows the number of birds positive for HPAIV and percent/infection by Group. No birds were excluded from the study.

**Table 20**

| **Group** | **Challenge Material** | **Vaccine Dose** | **Route/Volume** | **# birds** | **# Positive/ Total # birds** | **% Protection (% Infection)** |
|---|---|---|---|---|---|---|
| 1 | Minnesota/12582 | vHVT522 ∼2500 | SQ/0.2mL | 10 | 0/10 | 100 |
| 2 | Minnesota/12582 | vHW523 ∼2500 | SQ/0.2mL | 10 | 0/10 | 100 |
| 3 | Minnesota/12582 | vHVT509 ∼2500 | SQ/0.2mL | 10 | 0/10 | 100 |
| 4 | Minnesota/12582 | Sham-vaccinated Challenge Controls | SQ/0.2mL | 10 | 10/10 | (100) |
| 5 | Egypt/N04915 | vHVT522 ∼2500 | SQ/0.2mL | 10 | 0/10 | 100 |
| 6 | EgypVN04915 | VHVT523 ∼2500 | SQ/0.2mL | 10 | 1/10 | 90 |
| 7 | Egypt/N04915 | vHVT509 ∼2500 | SQ/0.2mL | 10 | 0/10 | 100 |
| 8 | Egypt/N04915 | Sham-vaccinated Challenge Controls | SQ/0.2mL | 10 | 10/10 | (100) |
| 9 | Hungary/53433 | vHVT522 ∼2500 | SQ/0.2mL | 10 | 0/10 | 100 |
| 10 | Hungary/53433 | vHVT523 ∼2500 | SQ/0.2mL | 10 | 1/10 | 90 |
| 11 | Hungary/53433 | vHVT509 ∼2500 | SQ/0.2mL | 10 | 0/10 | 100 |
| 12 | Hungary/53433 | Sham-vaccinated Challenge Controls | SQ/0.2mL | 10 | 10/10 | (100) |

The frequency of clinical signs such as severe depression, nervous or respiratory system signs and/or death is shown in the following table.

**Table 21**

| | | | # with Sign | Total # Birds |
|---|---|---|---|---|
| **Clinical Sign** | **Group** | **Vaccine_Dose** | | |
| **NEUROSGN** | **1** | **vHVT522** ∼**2500** | 0 | 10 |
| | **2** | **vHVT523** ∼2500 | 0 | 10 |
| | **3** | **vHVT509** ∼**2500** | 0 | 10 |
| | **4** | **Sham-vaccinated Challenge Controls** | 0 | 10 |
| | **5** | **vHVT522** ∼**2500** | 0 | 10 |
| | **6** | **vHVT523** ∼**2500** | 0 | 10 |
| | **7** | **vHVT509** ∼**2500** | 0 | 10 |
| | **8** | **Sham-vaccinated Challenge Controls** | 0 | 10 |
| | **9** | **vHVT522** ∼**2500** | 0 | 10 |
| | **10** | **vHVT523** ∼**2500** | 0 | 10 |
| | **11** | **vHVT509** ∼**2500** | 0 | 10 |
| | **12** | **Sham-vaccinated Challenge Controls** | 1 | 10 |
| | | **Total** | 1 | 120 |
| **DEPRESS** | **Group** | **Vaccine_Dose** | | |
| | **1** | **vHVT522** ∼**2500** | 0 | 10 |
| | **2** | **vHVT523** ∼**2500** | 0 | 10 |
| | **3** | **vHVT509** ∼**2500** | 0 | 10 |
| | **4** | **Sham-vaccinated Challenge Controls** | 1 | 10 |
| | **5** | **vHVT522** ∼**2500** | 0 | 10 |
| | **6** | **vHVT523** ∼**2500** | 1 | 10 |
| | **7** | **vHVT509** ∼**2500** | 0 | 10 |
| | **8** | **Sham-vaccinated Challenge Controls** | 1 | 10 |
| | **9** | **vHVT522** ∼**2500** | 0 | 10 |
| | **10** | **vHVT523** ∼**2500** | 0 | 10 |
| | **11** | **vHVT509** ∼**2500** | 0 | 10 |
| | **12** | **Sham-vaccinated Challenge Controls** | 1 | 10 |
| | | **Total** | 4 | 120 |

**Table 21- Continued**

| | | | **# with Sign** | **Total # Birds** |
|---|---|---|---|---|
| **RESPDIS** | **Group** | **Vaccine_Dose** | | |
| | **1** | **vHVT522 ∼2500** | 0 | 10 |
| | **2** | **vHVT523 ∼2500** | 0 | 10 |
| | **3** | **vHVT509 ∼2500** | 0 | 10 |
| | **4** | **Sham-vaccinated Challenge Controls** | 1 | 10 |
| | **5** | **vHVT522 ∼2500** | 0 | 10 |
| | **6** | **vHVT523 ∼2500** | 1 | 10 |
| | **7** | **vHVT509 ∼2500** | 0 | 10 |
| | **8** | **Sham-vaccinated Challenge Controls** | 0 | 10 |
| | **9** | **vHVT522 ∼2500** | 0 | 10 |
| | **10** | **vHVT523 ∼2500** | 0 | 10 |
| | **11** | **vHVT509 ∼2500** | 0 | 10 |
| | **12** | **Sham-vaccinated Challenge Controls** | 0 | 10 |
| | | **Total** | 2 | 120 |
| **OTHERCO** | **Group** | **Vaccine_Dose** | | |
| | **1** | **vHVT522 ∼2500** | 0 | 10 |
| | **2** | **vHVT523 ∼2500** | 0 | 10 |
| | **3** | **vHVT509 ∼2500** | 0 | 10 |
| | **4** | **Sham-vaccinated Challenge Controls** | 0 | 10 |
| | **5** | **vHVT522 ∼**2500 | 0 | 10 |
| | **6** | **vHVT523 ∼**2500 | 0 | 10 |
| | **7** | **vHVT509 ∼**2500 | 0 | 10 |
| | **8** | **Sham-vaccinated Challenge Controls** | 0 | 10 |
| | **9** | **vHVT522 ∼2500** | 0 | 10 |
| | **10** | **vHVT523 ∼2500** | a | 10 |
| | **11** | \| **vHVT509 ∼2500** | 0 | 10 |
| | **12** | **Sham-vaccinated Challenge Controls** | 0 | 10 |
| | | **Total** | 0 | 120 |

The following table shows the frequency of mortality by study group.

**Table 22**

| | | | | **Number of Animals** | **Euthanized** | | **Found Dead** | |
|---|---|---|---|---|---|---|---|---|
| | | | | | **N** | **%** | **N** | **%** |
| **Group** | **Challenge_Material** | **Vaccine_Dose** | **Route_Volume** | 10 | 10 | 100.0 | 0 | 0 |
| **1** | **Minnesota/12582** | **vHVT522 ∼2500** | **SQ/0.2mL** | | | | | |
| **2** | **Minnesota/12582** | **vHVT523 ∼2500** | **SQ/0.2mL** | 10 | 10 | 100.0 | 0 | 0 |
| **3** | **Minnesota/12582** | **vHVT509 ∼**2500 | **SQ/0.2mL** | 10 | 10 | 100.0 | 0 | 0 |
| **4** | **Minnesota/12582** | **Sham-vaccinated Challenge Controls** | **SQ/0.2mL** | 10 | 1 | 10.0 | 9 | 90.0 |
| **5** | **Egypt/N04915** | **vHVT522 ∼2500** | **SQ/0.2mL** | 10 | 10 | 100.0 | 0 | 0 |
| **6** | **Egypt/N04915** | **vHVT523 ∼2500** | **SQ/0.2mL** | 10 | 10 | 100.0 | 0 | 0 |
| **7** | **Egypt/N04915** | **vHVT509 ∼2500** | **SQ/0.2mL** | 10 | 10 | 100.0 | 0 | 0 |
| **8** | **Egyp/N04915** | **Sham-vaccinated Challenge Controls** | **SQ/0.2mL** | 10 | 1 | 10.0 | 9 | 90.0 |
| **9** | **Hungary/53433** | **vHVT522 ∼2500** | **SQ/0.2mL** | 10 | 10 | 100.0 | 0 | 0 |
| **10** | **Hungary/53433** | **vHVT523 ∼2500** | **SQ/0.2mL** | 10 | 9 | 90.0 | 1 | 10.0 |
| **11** | **Hungary/53433** | **vHVT509 ∼2500** | **SQ/0.2mL** | 10 | 10 | 100.0 | 0 | 0 |
| **12** | **Hungary/53433** | **Sham-vaccinated Challenge Controls** | **SQ/0.2mL** | 10 | 2 | 20.0 | 8 | 80.0 |

PCR/shedding results by day study group, and animal ID within each study group are shown in the following table.

**Table 23**

| | | | **DAY** | |
|---|---|---|---|---|
| | | | **30** | **32** |
| **Group** | **Vaccine dose** | **ID** | **Log10(EID50)** | **Log10(EID50)** |
| 1 | vHVT522 -2500 | 221 | 4.71 | 4.93 |
| | | 222 | 2.8 | <=1.1 |
| | | 223 | 3.62 | 5.07 |
| | | 224 | <=1.1 | <=1.1 |
| | | 225 | <=1.1 | <=1.1 |
| | | 226 | 3.58 | 4.28 |
| | | 227 | <=1.1 | <=1.1 |
| | | 228 | <=1.1 | <=1.1 |
| | | 229 | 4.05 | 1.46 |
| | | 230 | 2.48 | 4.96 |
| 2 | vHVT523 -2500 | 231 | 3.92 | 5.21 |
| | | 232 | 2.64 | 2.48 |
| | | 233 | 1.18 | <=1.1 |
| | | 234 | 4.28 | 5.89 |
| | | 235 | 2.78 | <=1.1 |
| | | 238 | 2.6/ | 4.41 |
| | | 237 | 4.48 | 4.88 |
| | | 238 | 4.68 | 5.36 |
| | | 239 | <=1.1 | <=1.1 |
| | | 240 | 3 62 | 6.28 |
| 3 | vHVT509 -2500 | 241 | 1.53 | <=1.1 |
| | | 242 | <=1.1 | 1.26 |
| | | 243 | 2.28 | <=1.1 |
| | | 244 | <=1.1 | <=1.1 |
| | | 245 | <=1.1 | <=1.1 |
| | | 246 | <=1.1 | <=1.1 |
| | | 247 | 3.52 | 1.14 |
| | | 248 | 1.18 | <=1.1 |
| | | 249 | <=1.1 | <=1.1 |
| | | 250 | 2.84 | 2.13 |
| 4 | Sham-vaccinated Challenge Controls | 251 | 7.73 | |
| | | 252 | 7.65 | |
| | | 253 | 6.69 | |
| | | 254 | 7.21 | |
| | | 255 | 7.08 | |
| | | 256 | 716 | |
| | | 257 | 8.05 | |
| | | 258 | 7.81 | |
| | | 259 | 6.97 | |
| | | 260 | 6 27 | |
| 5 | vHVT522 -2500 | 261 | 5.57 | 4.01 |
| 1 | | 262 | 4.49 | 4.02 |
| | | 263 | <= 1.7 | <= 1.7 |
| | | 264 | <= 1.7 | <= 1.7 |
| | | 265 | 3.47 | 4.33 |
| | | 266 | 3.4 | 4.29 |
| | | 267 | 232 | 2.42 |
| | | 268 | <= 1.7 | <= 1 7 |
| | | 269 | <= 1.7 | <= 1.7 |
| | | 270 | 3.49 | 4.69 |
| 6 | VHVT523 -2500 | 271 | 4.69 | 4.63 |
| | | 272 | 3.87 | 4.71 |
| | | 213 | 2.74 | 4.09 |
| | | 274 | 3.61 | 4.02 |
| | | 275 | 2.14 | <= 1.7 |
| | | 276 | 3.05 | 5.2 |
| | | 277 | <= 1.7 | <= 1.7 |
| | | 278 | 4.66 | 5.4 |
| | | 279 | <= 1.7 | <= 1.7 |
| | | 280 | 2.2 | 1.93 |
| 7 | vHVT509 -2500 | 281 | 4.14 | <= 1.7 |
| | | 282 | 332 | 4.12 |
| | | 283 | 3.92 | 5.08 |
| | | 284 | 316 | 1.7 |
| | | 285 | <= 1.7 | <= 1.7 |
| | | 286 | <= 1.7 | <= 1.7 |
| | | 287 | <= 1.7 | <= 1.7 |
| | | 280 | 2 15 | <= 1.7 |
| | | 289 | <= 1.7 | <= 1.7 |
| | | 290 | <= 1.7 | <= 1.7 |
| 8 | Sham-vaccinated Challenge Controls | 291 | 5.62 | |
| | | 292 | 6.86 | |
| | | 293 | 6 96 | |
| | | 294 | 6.56 | |

| | | | **30** | **32** |
|---|---|---|---|---|
| **Group** | **Vaccine dose** | **ID** | **Log10(EID50)** | **Log10(EID50)** |
| | | 295 | 6.88 | |
| | | 296 | 5 92 | |
| | | 291 | 7.27 | |
| | | 298 | 7.42 | |
| | | 299 | 6.1 | |
| | | 300 | 6.41 | |
| 9 | vHVT522 -2500 | 301 | 2 91 | <= 1.4 |
| | | 302 | <= 1.4 | <= 1.4 |
| | | 303 | 2 42 | <= 1.4 |
| | | 304 | <= 1.4 | <= 1.4 |
| | | 305 | 3.86 | 4 96 |
| | | 306 | <= 1.4 | <= 1.4 |
| | | 307 | <= 1.4 | <= 1.4 |
| | | 308 | <= 1.4 | <= 1.4 |
| | | 309 | <= 1.4 | <= 1.4 |
| | | 310 | <= 1.4 | <= 1.4 |
| 10 | vHVT523 -2500 | 311 | 2.54 | 2.33 |
| | | 312 | <= 1.4 | <= 1.4 |
| | | 313 | <= 1.4 | 2.04 |
| | | 314 | 4.34 | 3.67 |
| | | 315 | <= 1.4 | <= 1.4 |
| | | 316 | 2.76 | 283 |
| | | 317 | 5.2 | 5.17 |
| | | 318 | 1.62 | 2.96 |
| | | 319 | 3.34 | 1.58 |
| | | 320 | 4.09 | 5.4 |
| 11 | vHVT509 -2500 | 321 | <= 1.4 | <= 1.4 |
| | | 322 | <= 1.4 | <= 1.4 |
| | | 323 | <= 1.4 | <= 1.4 |
| | | 324 | <= 1.4 | <= 1.4 |
| | | 325 | <= 1.4 | <= 1.4 |
| | | 326 | <= 1 4 | <= 1.4 |
| | | 327 | <= 1.4 | <= 1.4 |
| | | 328 | <= 1 4 | <= 1.4 |
| | | 329 | <= 1.4 | <= 1.4 |
| | | 330 | <= 1.4 | 2.52 |
| 12 | Sham-vaccinated Challenge Controls | 331 | 3.04 | 6.62 |
| | | 332 | 6.M | |

**Table 23 - Continued**

| | | | **DAY** | |
|---|---|---|---|---|
| | | | **30** | **32** |
| **Group** | **Vaccine dose** | **ID** | **Log10(EID50)** | **Log10(EID50)** |
| | | 333 | 7.16 | |
| | | 334 | 6.71 | |
| | | 335 | 5.7 | |
| | | 336 | 6.6 | |
| | | 337 | 5.54 | |
| | | 338 | 6.03 | |
| | | 339 | 6.95 | |
| | | 340 | 6.01 | |

The following table summarizes the statistics of the observed PCR results by study group.

**Table 24**

| | | | **N** | **Mean** | **Std** | **Min** | **25th PCTL** | **Median** | **75th PCTL** | **Max** |
|---|---|---|---|---|---|---|---|---|---|---|
| **DAY** | **Group** | **Vaccine_Dose** | | | | | | | | |
| **30** | **1** | **vHVT522 ∼2500** | 10 | 2.5 | 1.4 | 1.0 | 1.0 | 2.6 | 3.6 | 4.7 |
| | **2** | **vHVT523 ∼2500** | 10 | 3.1 | 1.3 | 1.0 | 2.6 | 3.2 | 4.3 | 4.7 |
| | **3** | **vHVT509 ∼2500** | 10 | 1.6 | 0.9 | 1.0 | 1.0 | 1.1 | 2.3 | 3.5 |
| | **4** | **Sham-vaccinated Challenge Controls** | 10 | 73 | 0.6 | 6.3 | 7.0 | 72 | 77 | 8.1 |
| | **5** | **vHVT522 ∼2500** | 10 | 2.9 | 1.4 | 1.6 | 1.6 | 2.9 | 3.5 | 5.6 |
| | **6** | **vHVT523 ∼2500** | 10 | 3.0 | 1.2 | 1.6 | 2.1 | 2.9 | 3.9 | 4.7 |
| | **7** | **vHVT509 ∼2500** | 10 | 2.5 | 1.1 | 1.6 | 1.6 | 1.9 | 3.3 | 4.1 |
| | **8** | **Sham-vaccinated Challenge Controls** | 10 | 6.6 | 0.6 | 5.6 | 6.1 | 6..7 | 7.0 | 7.4 |
| | **9** | **vHVT522 ∼2500** | 10 | 1.8 | 0.9 | 1.3 | 1.3 | 1.3 | 2.4 | 3.9 |
| | **10** | **vHVT523 ∼2500** | 10 | 2.8 | 1.4 | 1.3 | 1.3 | 2.7 | 4.1 | 5.2 |
| | **11** | **vHVT509 ∼2500** | 10 | 1.3 | 0.0 | 1.3 | 1.3 | 1.3 | 1.3 | 1.3 |
| | **12** | **Sham-vaccinated Challenge Controls** | 10 | 6.0 | 1.2 | 3.0 | 57 | 6.3 | 6.7 | 7.2 |
| **32** | **1** | **vHVT622 ∼2600** | 10 | 2.6 | 1.9 | 1.0 | 1.0 | 1.2 | 4.9 | 5.1 |
| | **2** | **vHVT523 ∼2500** | 10 | 3.8 | 2.2 | 1.0 | 1.0 | 4.6 | 5.4 | 6.3 |
| | **3** | **vHVT509 ∼2500** | 10 | 1.2 | 0.4 | 1.0 | 1.0 | 1.0 | 1.1 | 2.1 |
| | **5** | **vHVT522 ∼2500** | 10 | 3.0 | 1.4 | 1.6 | 1.6 | 3.1 | 4.3 | 4.7 |
| | **6** | **vHVT523 ∼2500** | 10 | 3.5 | 1.6 | 1.6 | 1.6 | 4.1 | 4.7 | 5.4 |
| | **7** | **vHVT509 ∼2500** | 10 | 2.2 | 1.3 | 1.6 | 1.6 | 1.6 | 1.6 | 5.1 |
| | **9** | **vHVT522 ∼2500** | 10 | 1.7 | 1.2 | 1.3 | 1.3 | 1.3 | 1.3 | 5.0 |
| | **10** | **vHVT523 ∼2500** | 10 | 2.9 | 1.5 | 1.3 | 1.6 | 2.6 | 3.1 | 5.4 |
| | **11** | **vHVT509 ∼2500** | 10 | 1.4 | 0.4 | 1.3 | 1.3 | 1.3 | 1.3 | 2.5 |
| | **12** | **Sham-vaccinated Challenge Controls** | 1 | 6.6 | | 6.6 | 6.6 | 6.6 | 6.6 | 6.6 |

The following table shows the frequency of positive/negative PCR results by day and group.

**Table 25**

| | | | **Number of Animals** | **Log10(EID50)** | | | |
|---|---|---|---|---|---|---|---|
| | | | | **Negative** | | **Positive** | |
| | | | | **N** | **%** | **N** | **%** |
| **DAY** | **Group** | **Vaccine_Does** | | | | | |
| **30** | **1** | **vHVT522 ∼2500** | 10 | 4 | 40.0 | 6 | 60.0 |
| | **2** | **vHVT523 ∼2500** | 10 | 1 | 10.0 | 9 | 90.0 |
| | **3** | **vHVT509 ∼2500** | 10 | 5 | 50.0 | 5 | 50.0 |
| | **4** | **Sham-vaccinated Challenge Controls** | 10 | 0 | 0 | 10 | 100.0 |
| | **5** | **vHVT522 ∼2500** | 10 | 4 | 40.0 | 6 | 60.0 |
| | **6** | **vHVT523 ∼2500** | 10 | 2 | 20.0 | 8 | 80.0 |
| | **7** | **vHVT509 ∼2500** | 10 | **5** | 50.0 | 5 | 50.0 |
| | **8** | **Sham-vaccinated Challenge Controls** | 10 | 0 | 0 | 10 | 100.0 |
| | **9** | **vHVT522 ∼2500** | 10 | 7 | 70.0 | 3 | 30.0 |
| | **10** | **vHVT523 ∼2500** | 10 | 3 | 30.0 | 7 | 70.0 |
| | **11** | **vHVT509 ∼2500** | 10 | 10 | 100.0 | 0 | 0 |
| | **12** | **Sham-vaccinated Challenge Controls** | 10 | 0 | 0 | 10 | 100.0 |
| **32** | **1** | **vHVT622 ∼2500** | 10 | 5 | 50.0 | 5 | 50.0 |
| | **2** | **vHVT523 ∼2500** | 10 | 3 | 30.0 | 7 | 70.0 |
| | **3** | **vHVT509 ∼2500** | 10 | 7 | 70.0 | 3 | 30.0 |
| | **5** | **vHVT522 ∼2500** | 10 | 4 | 40.0 | 6 | 60.0 |
| | **6** | **vHVT523 ∼2500** | 10 | 3 | 30.0 | 7 | 70.0 |
| | **7** | **vHVT509 ∼2500** | 10 | 8 | 80.0 | 2 | 20.0 |
| | **9** | **vHVT522 ∼2500** | 10 | 9 | 90.0 | 1 | 10.0 |
| | **10** | **vHVT523 ∼2500** | 10 | 2 | 20.0 | 8 | 80.0 |
| | **11** | **vHVT509 ∼2500** | 10 | 9 | 90.0 | 1 | 10.0 |
| | **12** | **Sham-vaccinated Challenge Controls** | 1 | 0 | 0 | 1 | 100.0 |

The RT-PCR limit of detection for challenge with Minnesota/12582 was 1.1.

The RT-PCR limit of detection for challenge with Hungary/53433 was 1.4.

The RT-PCR limit of detection for challenge with Egypt/N04915 was 1.7.

All samples with titers less than the limit of detection were considered negative. For the purposes of statistical calculations, all negative samples were assigned the value of "0.1" less than the limit of detection.

The following table shows hemagglutination inhibition results in study groups challenged with Minnesota/12582 by Group, animal ID, and day.

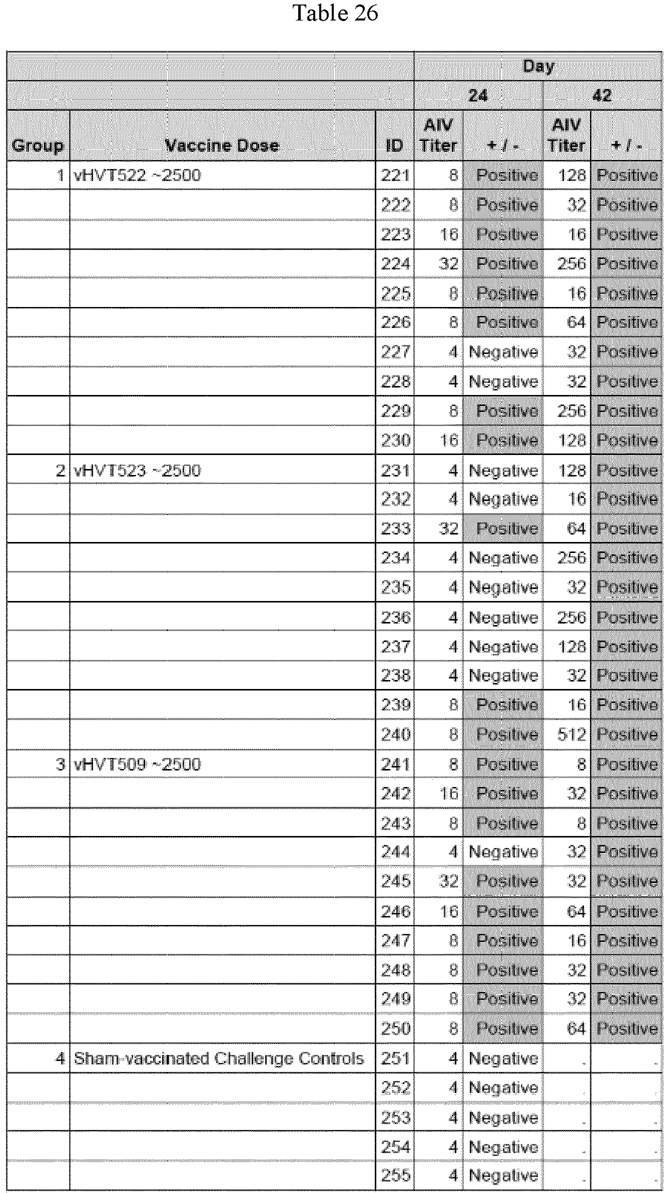

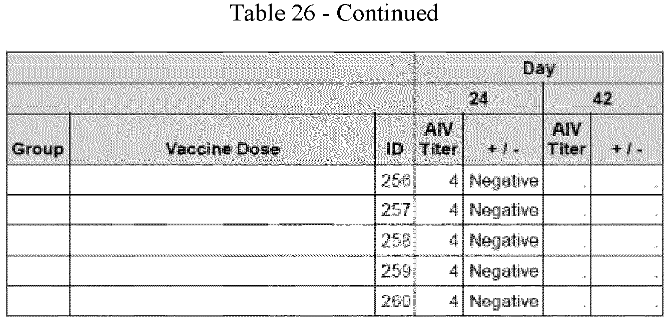

The limit of detection for the hemagglutination inhibition test was 8. All samples listed as "<8" are negative and were assigned a value of "4" for geometric mean titer calculations purposes.

The following table summarizes the geometric mean titer calculations of Minnesota/12582 hemagglutination inhibition results by day and study group.

**Table 27**

| **Day** | **Group** | **Vaccine dose** | **Route Volume** | **Geometric Mean** | **Log2 Titer** |
|---|---|---|---|---|---|
| 24 | 1 | vHVT522 ∼2500 | SQ/0.2mL | 92 | 3.2 |
| 24 | 2 | vHVT523 ∼2500 | SQ/0.2mL | 5.7 | 2.5 |
| 24 | 3 | vHVT509 ∼2500 | SQ/0.2mL | 9.8 | 3.3 |
| 24 | 4 | Sham-vaccinated Challenge Controls | SQ/0.2mL | 4.0 | 2.0 |
| 42 | 1 | vHVT522 ∼2500 | SQ/0.2mL | 59.7 | 5.9 |
| 42 | 2 | vHVT523 ∼2500 | SQ/0.2mL | 78.8 | 6.3 |
| 42 | 3 | vHVT509 ∼2500 | SQ/0.2mL | 26.0 | 4.7 |

The following table shows the frequency of positive Minnesota/12582 hemagglutination inhibition results by day and study group.

**Table 28**

| | | | **Number of Animals** | **HI** | | | |
|---|---|---|---|---|---|---|---|
| | | | | **Negative** | | **Positive** | |
| | | | | **N** | **%** | **N** | **%** |
| **DAY** | **Group** | **Vaccine_Dose** | | | | | |
| **24** | **1** | **vHVT522** ∼**2500** | 10 | 2 | 20.0 | a | 80.0 |
| | **2** | **vHVT523** ∼**2500** | 10 | 1 | 70.0 | 3 | 30.0 |
| | **3** | **vHVT509** ∼**2500** | 10 | 1 | 10.0 | 9 | 90.0 |
| | **4** | **Sham-vaccinated Challenge Controls** | 10 | 10 | 100.0 | 0 | 0 |
| **42** | **1** | **vHVT522** ∼**2500** | 10 | 0 | 0 | 10 | 100.0 |
| | **2** | **vHVT523** ∼**2500** | 10 | 0 | 0 | 10 | 100.0 |
| | **3** | **vHVT509** ∼**2500** | 10 | 0 | 0 | 10 | 100.0 |

FIG. 58 shows a bar graph of the hemagglutination inhibition results at days 24 and 42 for chickens challenged with Minnesota/12582.

The following table shows hemagglutination inhibition results in study groups challenged with Egypt/N04915 by Group, animal ID, and day.

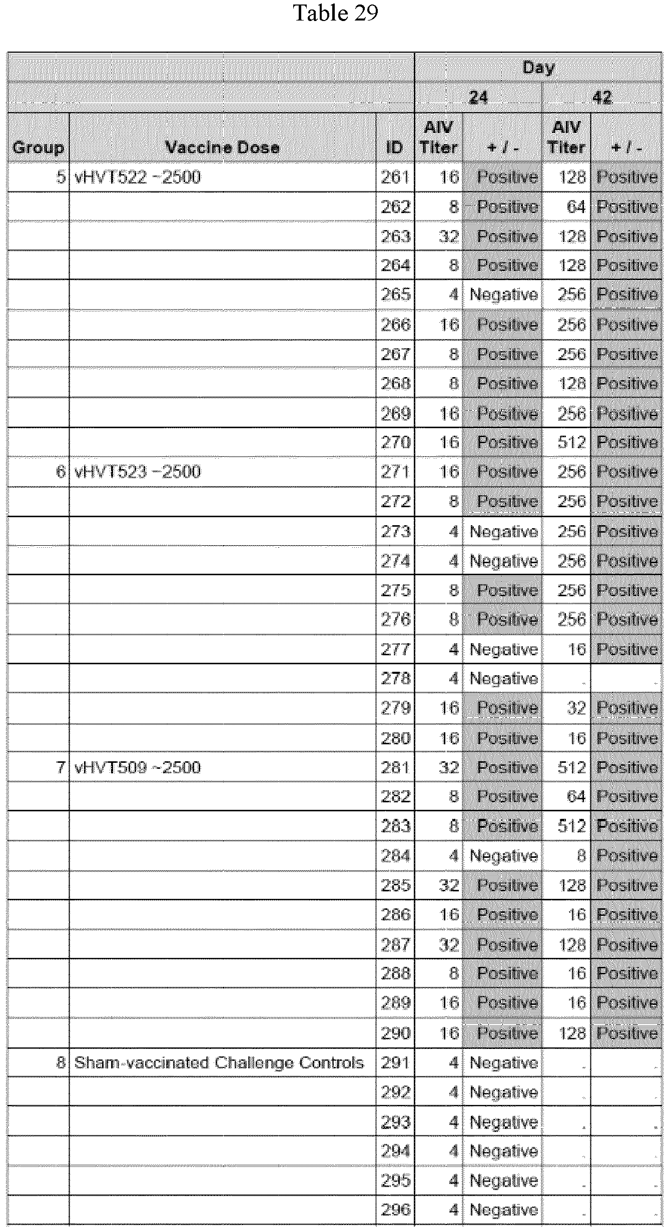

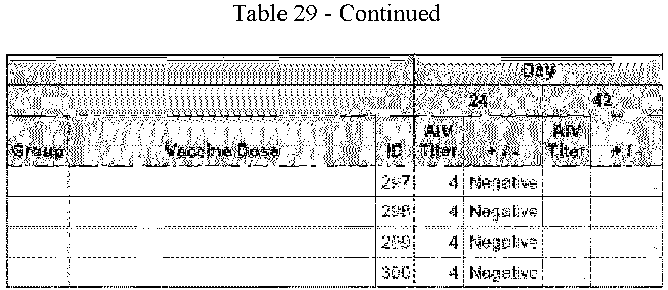

The limit of detection for the hemagglutination inhibition test was 8. All samples listed as "<8" are negative and were assigned a value of "4" for geometric mean titer calculations purposes.

The following table summarizes the geometric mean titer calculations of Egypt/N04915 hemagglutination inhibition results by day and study group.

**Table 30**

| **Day** | **Group** | **Vaccine dose** | **Route Volume** | **Geometric Mean** | **Log2 Titer** |
|---|---|---|---|---|---|
| 24 | 5 | vHVT522 ∼2500 | SQ/0.2mL | 11.3 | 3.5 |
| 24 | 6 | vHVT523 ∼2500 | SQ/0.2mL | 7 5 | 2.9 |
| 24 | 7 | vHVT509 ∼2500 | SQ/0.2mL | 13.9 | 3.8 |
| 24 | 8 | Sham-vaccinated Challenge Controls | SQ/0.2mL | 4.0 | 2.0 |
| 42 | 5 | vHVT522 ∼2500 | SQ/0.2mL | 181.0 | 7.5 |
| 42 | 6 | vHVT523 ∼2500 | SQ/0.2mL | 109.7 | 6.8 |
| 42 | 7 | vHVT509 ∼2500 | SQ/0.2mL | 64.0 | 6.0 |

The following table shows the frequency of positive Egypt/N04915 hemagglutination inhibition results by day and study group.

**Table 31**

| | | | **Number Animals** | **HI** | | | |
|---|---|---|---|---|---|---|---|
| | | | | **Negative** | | **Positive** | |
| | | | | **N** | **N %** | **N** | **%** |
| **DAY** | **Group** | **Vaccine_Dose** | 10 | 1 | 10.0 | 9 | 90.0 |
| **24** | **5** | **vHVT622 ∼2500** | | | | | |
| | **6** | **vHVT523 ∼2500** | 10 | 4 | 40.0 | 6 | 60.0 |
| | **7** | **vHVT509 ∼2500** | 10 | 1 | 10.0 | 9 | 90.0 |
| | **8** | **Sham-vaccinated Challenge Controls** | 10 | 10 | 100.0 | 0 | 0 |
| **42** | **5** | **vHVT522 ∼2500** | 10 | 0 | 0 | 10 | 100.0 |
| | **6** | **vHVT523 ∼2500** | 9 | 0 | 0 | 9 | 100.0 |
| | **7** | **vHVT509 ∼2500** | 10 | 0 | 0 | 10 | 100.0 |

FIG. 59 shows a bar graph of the hemagglutination inhibition results at days 24 and 42 for chickens challenged with Egypt/N04915.

The following table shows hemagglutination inhibition results in study groups challenged with Hungary/53433 by Group, animal ID, and day.

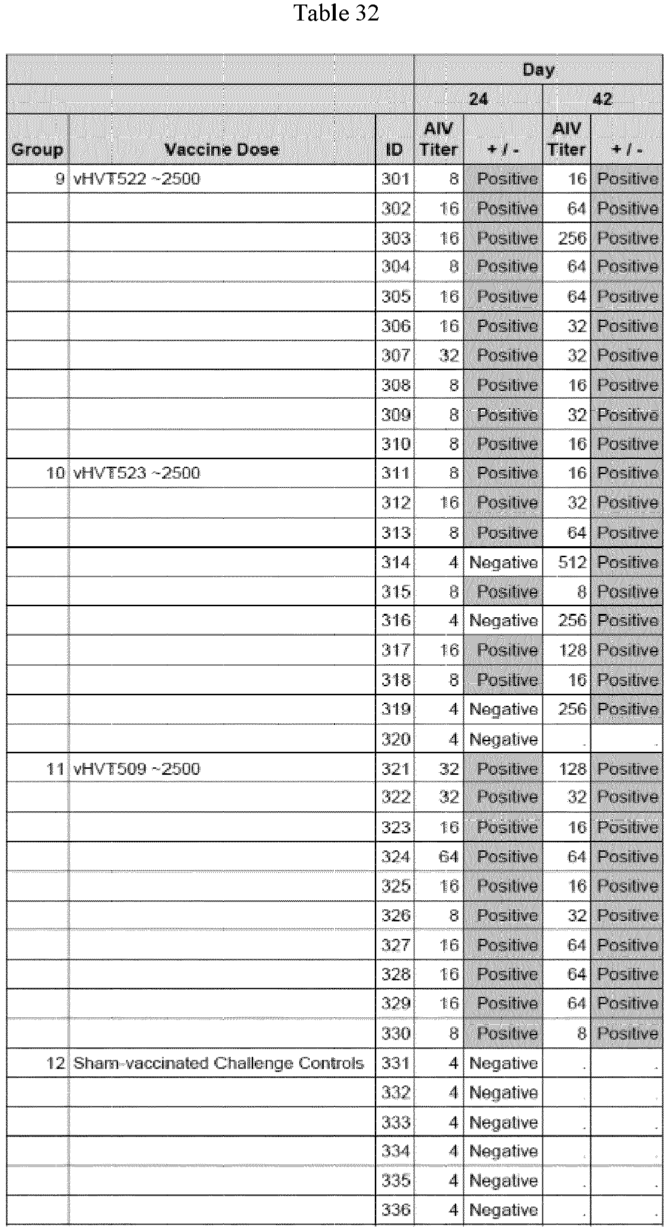

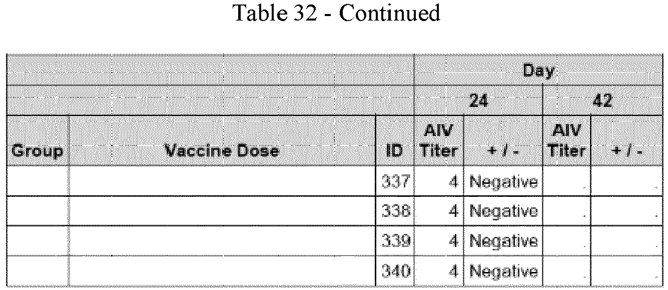

The limit of detection for the hemagglutination inhibition test was 8. All samples listed as "<8" are negative and were assigned a value of "4" for geometric mean titer calculations purposes.

The following table summarizes the geometric mean titer calculations of Hungary/53433 hemagglutination inhibition results by day and study group.

**Table 33**

| **Day** | **Group** | **Vaccine dose** | **Route Volume** | **Geometric Mean** | **Log2 Titer** |
|---|---|---|---|---|---|
| 24 | 9 | vHVT522 ∼2500 | SQ/0.2mL | 121 | 3.6 |
| 24 | 10 | vHVT523 ∼2500 | SQ/0.2mL | 7.0 | 2.8 |
| 24 | 11 | vHVT509 ∼2500 | SQ/0.2mL | 18.4 | 4.2 |
| 24 | 12 | Sham-vaccinated Challenge Controls | SQ/0.2mL | 4.0 | 2.0 |
| 42 | 9 | vHVT522 ∼2500 | SQ/0.2mL | 39.4 | 5.3 |
| 42 | 10 | vHVT523 ∼2500 | SQ/0.2mL | 64.0 | 6 0 |
| 42 | 11 | vHVT509 ∼2500 | SQ/0.2mL | 36.8 | 5.2 |

The following table shows the frequency of positive Hungary/53433 hemagglutination inhibition results by day and study group.

**Table 34**

| | | | **Number of Animals** | **HI** | | | |
|---|---|---|---|---|---|---|---|
| | | | | **Negative** | | **Positive** | |
| | | | | **N** | **%** | **N** | **%** |
| **DAY** | **Group** | **Vaccine_Dose** | 10 | 0 | 0 | 10 | 100.0 |
| **24** | **9** | **vHVT522** ∼**2500** | | | | | |
| | **10** | **vHVT523** ∼**2500** | 10 | 4 | 40.0 | 6 | 60.0 |
| | **11** | **vHVT509** ∼**2500** | 10 | 0 | 0 | 10 | 100.0 |
| | **12** | **Sham-vaccinated Challenge Controls** | 10 | 10 | 100.0 | 0 | 0 |
| **42** | **9** | **vHVT522** ∼**2500** | 10 | 0 | 0 | 10 | 100.0 |
| | **10** | **vHVT523** ∼**2500** | 9 | 0 | 0 | 9 | 100.0 |
| | **11** | **vHVT509** ∼**2500** | 10 | 0 | 0 | 10 | 100.0 |

FIG. 60 shows a bar graph of the hemagglutination inhibition results at days 24 and 42 for chickens challenged with Hungary/53433.

The following table shows the results from the viral shedding experiments by challenge material, vaccine dose, and study day. Oropharyngeal swab samples were collected on study days 30 and 32. The titer of shedding (Log₁₀/mL) were measured by RT-PCR. A positive swab result for each bird was defined as one or more occurrences of a positive RT-PCR result post challenge in each bird. Negative samples were considered to be lower than the limit of detection established for each virus. Boxplots of the shedding results for study days 30 and 32 are shown in FIG. 51 and FIG. 52 respectively.

**Table 35**

| **Challenge_Matarial** | **Vaccine_Dose** | **DAY** | **Number of Birds** | **Number Shedding** | **Percent Shedding** |
|---|---|---|---|---|---|
| Egypt/N04915 | Sham-vaccinated Challenge Controls | 30 | 10 | 10 | 100.0 |
| Egypt/N04915 | vHVT509 ∼2500 | 30 | 10 | 5 | 50 0 |
| Egypt/N04915 | vHVT509 ∼2500 | 32 | 10 | 2 | 20.0 |
| Egypt/N04915 | vHVT522 ∼2500 | 30 | 10 | 6 | 60.0 |
| Egypt/N04915 | vHVT522 ∼2500 | 32 | 10 | 6 | 60.0 |
| Egypt/N04915 | vHVT523 ∼2500 | 30 | 10 | 8 | 80.0 |
| Egypt/N04915 | vHVT523 ∼2500 | 32 | 10 | 7 | 70.0 |
| Hungary/53433 | Sham-vaccinated Challenge Controls | 30 | 10 | 10 | 100.0 |
| Hungary/53433 | Sham-vaccinated Challenge Controls | 32 | 1 | 1 | 100.0 |
| Hungary/53433 | vHVT509 ∼2500 | 30 | 10 | 0 | 0.0 |
| Hungary/53433 | vHVT509 ∼2500 | 32 | 10 | 1 | 10.0 |
| Hungary/53433 | vHVT522 ∼2500 | 30 | 10 | 3 | 30.0 |
| Hungary/53433 | vHVT522 ∼2500 | 32 | 10 | 1 | 10.0 |
| Hungary/53433 | vHVT523 ∼2500 | 30 | 10 | 7 | 70 0 |
| Hungary/53433 | vHVT523 ∼2500 | 32 | 10 | 8 | 80.0 |
| Minnesota/12582 | Sham-vaccinated Challenge Controls | 30 | 10 | 10 | 100.0 |
| Minnesota/12582 | vHVT509 ∼2500 | 30 | 10 | 5 | 50.0 |
| Minnesota/12582 | vHVT509 ∼2500 | 32 | 10 | 3 | 30 0 |
| Minnesota/12582 | vHVT522 ∼2500 | 30 | 10 | 6 | 60.0 |
| Minnesota/12582 | vHVT522 ∼2500 | 32 | 10 | 5 | 50 0 |
| Minnesota/12582 | vHVT523 ∼2500 | 30 | 10 | 9 | 90.0 |
| Minnesota/12582 | vHVT523 ∼2500 | 32 | 10 | 7 | 70.0 |

The following table presents the estimated log₁₀ differences on study day 30 between the treated groups (1, 2, and 3) and control group 4, which were challenged with Minnesota/12582.

**Table 36**

| Label | Estimated Difference | Lower Cl for Difference | Upper Cl for Difference |
|---|---|---|---|
| Group: 1 vs 4 | -4.7380 | -5.7465 | **-3.7295** |
| Group: 2 vs 4 | -4.1370 | -5.1455 | -3.1285 |
| Group: 3 vs 4 | -5.6270 | -6.6355 | -4.6185 |

Because the 95% confidence intervals for the mean log₁₀ titer differences between treated groups (1, 2, and 3) and group 4 do not contain 0, the mean titers for the treatment groups (1, 2, and 3) are all significantly less than the mean titer for group 4.

The following table presents the estimated log₁₀ differences on study day 30 between the treated groups (5, 6, and 7) and control group 8, which were challenged with Egypt/N04915.

**Table 37**

| Label | Estimated Difference | Lower Cl for Difference | Upper Cl for Difference |
|---|---|---|---|
| Group; 5 vs 8 | -3.6860 | -4.6757 | -2.6963 |
| Group: 6 vs 8 | -3.5840 | -4.5737 | -2.5943 |
| Group: 7 vs 3 | -4.1310 | -5.1207 | -3.141 3 |

Because the 95% confidence intervals for the mean log₁₀ titer differences between treated groups (5, 6, and 7) and group 8 do not contain 0, the mean titers for the treatment groups (5, 6, and 7) are all significantly less than the mean titer for group 8.

The following table presents the estimated log₁₀ differences on study day 30 between the treated groups (9, 10, and 11) and control group 12, which were challenged with Hungary/53433.

**Table 38**

| Label | Estimated Difference | Lower Cl for Difference | Upper Cl for Difference |
|---|---|---|---|
| Group: 9 vs 12 | -4.2110 | -5.1489 | -3.2731 |
| Group: 10 vs 12 | -3.2610 | -4.1989 | -2.3231 |
| Group: 11 vs 12 | -47400 | -5.6779 | -3.8021 |

Because the 95% confidence intervals for the mean log₁₀ titer differences between treated groups (9, 10, and 11) and group 12 do not contain 0, the mean titers for the treatment groups (9, 10, and 11) are all significantly less than the mean titer for group 12.

Conclusions: Under the conditions of this trial, all the tested constructs were able to provide the required clinical protection against clinical signs and mortality; all tested candidates were also able to significantly decrease the average shedding on days 30 and 32; with an overall better decrease (average titer and number of birds shedding) for all tested challenge strains in birds vaccinated with the constructs vHVT509, followed by vHVT522. Antibody levels and number of birds positive by HI pre-challenge were similar between vHVT522 and vHVT509; and slightly lower levels were observed with vHVT523. All tested constructs provide the required clinical protection, decrease in shedding and adequate HI levels pre-vaccination. Based on clinical protection, shedding and antibody levels, the constructs with better overall results were vHVT509, followed by vHVT522 and vHVT523.

### Example 5

### Efficacy of Avian Influenza-Bursal Disease-Marek's Disease, Serotype 3, Live Marek's Disease Vector (vHVT522-AI-IBDV) When Challenged with Infectious Bursal Disease Viruses, STC Classic and Variant-E strains

The aim of this study is to evaluate the immunogenicity of vHVT522-AI-IBDV when challenged with Infectious Bursal Disease Virus (STC and VAR-E strains).

The primary variable investigated is the effect of Infectious Bursal Disease Virus (IBDV) on the observation of gross lesions (STC) or body weight (B/B wt.) ratios (VAR-E). For the purposes of this study, gross lesions include peri-bursal edema and/or edema and/or macroscopic hemorrhage in the bursal tissue. Any bird with these stated lesions was recorded as positive.

The test vaccine used in the study was as follows.

**Table 39**

| Product True Name | Avian Influenza-Bursal Disease-Marek's Disease, Serotype 3, Live Marek's Disease Vector [vHVT522-AI-IBDV] |
|---|---|
| Formulation | The vaccine was diluted to target ∼2,500 **PFU**/0.2 mL dose. |
| Manufacturer | Merial Inc. (US Veterinary Biologics Est. 298) |
| Storage Requirements | Liquid Nitrogen |
| Applied dose and Route | 0.2 mL per bird, administered by the subcutaneous route (SQ) |

The challenge material used in the study was as follows.

**Table 40**

| Scientific Name | 1. Infectious Bursal Disease Virus (IBDV) |
|---|---|
| Strain/Relation to Test Vaccine | 1a. IBDV Classical-STC |
| | 1b. IBDV Variant-E |
| Source of Material | 1a. Merial, Inc. (STC) |
| | 1b. University of Delaware (VAR-E) |
| Storage Requirements | -70 °C |
| Applied Dose | 1a. 0.03 mL per bird intra-ocular (IO) route (∼10^{2.0} EID₅₀/dose) |
| | 1b. 0.03 mL per bird intra-ocular (IO) route (∼10^{3.0} EID₅₀/dose) |
| Route of Administration | Intra-ocular (IO) |

The animals used in the study were as follows.

**Table 41**

| Species | *Gallus domesticus* |
|---|---|
| Strain / Breed | SPF chickens |
| Source | SPAFAS |
| Sex | Female/Male |
| Weight | N/A |
| Age | One-day-old-chicks |
| Number | 100 one-day-old chicks |
| Physiological Status | Healthy and alert |
| Inclusion Criteria | All birds appearing healthy and alert |
| Conditioning / Acclimation | N/A |
| Serologic Status | N/A |
| Exclusion Criteria | Animals that are debilitated, suffering from disease or injury, fractious or otherwise unsuitable for inclusion in the study, in the opinion of the Investigator, were excluded from the study. |
| Animal Identification | The birds were identified according to unit placement and numbered bands. |
| Criteria for Removal from Study | After inclusion in the study, animals that are debilitated, suffering from disease or injury, fractious or otherwise unsuitable to remain in the study, in the opinion of the Investigator, were removed. |

One hundred (100) day-old SPF chicks, SPAFAS flock C17 were assigned to Groups 1-5. Twenty birds were assigned per isolation unit. All birds were vaccinated or sham-vaccinated. Groups 1-2 were vaccinated with Avian Influenza-Bursal Disease-Marek's Disease, Serotype 3, Live Marek's Disease Vector, vHVT-522 IBD-AI (P3) #6, Lot 11-May-2018 AK, at 2,050 PFU HVT/0.2 mL dose. Groups 3- 5 were inoculated with Marek's diluent only DJ612, Exp. Date JAN 20.

**Table 42**

| **Group** | **Vaccine PFU/dose*** | **Route/ Volume (mL)** | **Number Of Birds Placed** | **Number Of Birds Challenged*** | **Challenge** |
|---|---|---|---|---|---|
| 1 | vHVT522-AI-IBDV 2,500 PFU | 0.2 mL / SQ | 20 | ∼20 | IBDV STC |
| 2 | vHVT522-AI-IBDV 2,500 PFU | 0.2 mL / SQ | 20 | ∼20 | IBDV VAR-E |
| 3 | Sham-vaccinated/ IBDV STC Challenge Controls | 0.2 mL / SQ | 20 | ∼20 | IBDV STC |
| 4 | Sham-vaccinated/ IBDV VAR-E Challenge Controls | 0.2 mL / SQ | 20 | ∼20 | IBDV VAR-E |
| 5 | Sham-vaccinated, sham-challenged (Negative Controls) | 0.2 mL / SQ | 20 | ∼20 | Sham-Challenge |

| | | | | | |
|---|---|---|---|---|---|
| * Up to 20 birds were challenged in each group depending on early mortality | | | | | |

All animals were housed in the same manner. The birds were housed in negative pressure isolation units for the duration of the trial. Water and feed were given *ad libitum.*

The birds challenged with STC were observed for 4 days post-challenge. At the end of the observation period (Study Day 32), the birds were necropsied and examined for gross lesions of IBDV STC.

For purposes of this test with respect to STC, gross lesions should include peri-bursal edema and/or edema and/or macroscopic hemorrhage in the bursal tissue. Any bird with these stated lesions was recorded as positive.

The remaining birds challenged with VAR-E and sham challenged were observed for 11 days post-challenge. At the end of the observation period (Study Day 39), the birds were terminated and the sex, body weight and bursal weight were collected.

For purposes of this test with respect to VAR-E, the decreased size (atrophy) based on B/B wt. ratio was observed.

On Day 0, the birds were vaccinated or sham-vaccinated by the SQ route before they were placed in negative pressure isolation units. The vaccines were diluted with Marek's disease vaccine diluent accordingly to administer the targeted doses.

On Study Day 13, all the remaining birds were neck-banded for individual identification.

On Study Day 21, birds from Groups 1 and 3 were challenged with IBDV-STC, EP-1 Lot 080116, at 10^{1.8} EID₅₀/0.03 mL dose.

On Study Day 25, all the remaining birds challenged with IBDV-STC (Groups 1 and 3) were euthanized and necropsied to examine for gross bursal lesions.

On Study Day 28, all birds were challenged with Var-E IBDV 1084-E CP2 3-14-95 Lot 031495 at 10^{2.9} EID₅₀/0.03 mL dose; or sham-challenged.

On Study Day 39, the sex, body weight and bursal weight were collected from all birds remaining in Groups 2, 4 and 5.

### Results

The number of birds that tested positive for bursal disease following challenge with IBDV-STC are shown in the table below.

**Table 43**

| **Group** | **Vaccine Dose / Actual Dose** | **Challenge** | **# birds** | **# Positive/ Total # birds** | **% Protection (% Infection)** |
|---|---|---|---|---|---|
| 1 | vHVT522-AI-IBDV 2,050 pfu/dose | IBDV-STC | 20 | 0/20 | 100 |
| 3 | Sham-vaccinated/BDV STC Challenge Controls | IBDV-STC | 20 | 18/20 | (90) |

The mean B/B wt. ratios by study group following challenge with IVDB VAR-E or sham-vaccinated and sham-challenged are shown in the table below.

**Table 44**

| **Group** | **Vaccine** / **Actual Dose** | **Challenge** | **# birds** | **Mean Bursa Weight (g)** | **Mean Body Weight (g)** | **Mean B/BW Ratio** |
|---|---|---|---|---|---|---|
| 2 | vHVT522-AI-IBDV 2,050 pfu/0.2 ml dose | IBDV VAR-E | 19 | 1.39 | 354.05 | 0.36 |
| 4 | Sham-vaccinated/IBDV VAR-E Challenge Controls | IBDV VAR-E | 19 | 0.45 | 374.47 | 0.12 |
| 5 | Sham-vaccinated, sham-challenged (Negative Controls) | Sham-Challenge | 20 | 2.27 | 435.20 | 0.52 |

### Body weights

The body weight and bursal weight were collected and B/B wt. ratio (bursa weight/body weight ratio X 100) was calculated for each bird. The summary statistics for B/B wt. ratios are shown in the following table.

**Table 45**

| **Group** | **Vaccine Actual PFU/dose** | **N** | **Mean** | **Std** | **Min** | **Q₁** | **Median** | **Q₃** | **Max** |
|---|---|---|---|---|---|---|---|---|---|
| 2 | vHVT522-AI-IBDV 2,050 PFU/0.2 mL dose | 19 | 0.36 | 0.17 | 0.09 | 0.22 | 0.29 | 0.56 | 0.62 |
| 4 | Sham-vaccinated/ IBDV VAR-E Challenge Controls | 19 | 0.12 | 0.03 | 0.08 | 0.09 | 0.12 | 0.14 | 0.18 |
| 5 | Sham-vaccinated, sham-challenged (Negative Controls) | 20 | 0.52 | 0.15 | 0.30 | 0.44 | 0.50 | 0.57 | 1.07 |

The p-values were adjusted for multiple comparisons using Tukey-Kramer method, and can be observed pair-wise group comparisons.

The following table shows p-values and adjusted p-values of pair-wise comparisons of bursa to body weight ratios and pair-wise comparisons of bursa to body weight ratios by sex among the groups.

**Table 46**

| | | | **Sex** | | | |
|---|---|---|---|---|---|---|
| | | | **F** | | **M** | |
| **Group Comparisons** | **p-value** | **Adjusted p-value** | **p-value** | **Adjusted p-value** | **p-value** | **Adjusted p-value** |
| 2 vs. 4 | <.0001 | <.0001 | <.0001 | <.0001 | 0.0073 | 0.0747 |
| 4 vs. 5 | <.0001 | <.0001 | <.0001 | <.0001 | <.0001 | <.0001 |

The following table shows the birds that were excluded from the study by animal ID and study group. Study Day 0 was August 23, 2018.

**Table 47**

| **ID** | **Group** | **Vaccine/Target Dose** | **Challenge** | **Unit** | **Mortality Date** | **Mortality** | **Study Day** |
|---|---|---|---|---|---|---|---|
| 21 | 4 | Sham-vaccinated/IBDV VAR-E Challenge Contrails | IBDV VAR-E | 14 | 31AUG2018 | Euthanized | 8 |
| 61 | 2 | vHVT522-AI-IBDV 2,500 pfu | IBDV VAR-E | 16 | 30AUG2018 | Euthanized | 7 |

The following table shows the mortality record of animals in the study by animal ID and study group. Study Day 0 was August 23, 2018.

**Table 48**

| **ID** | **Group** | **Vaccine/Target Dose** | **Challenge** | **Unit** | **Mortality Date** | **Mortality** | **Study Day** | **EXCLUDED** |
|---|---|---|---|---|---|---|---|---|
| 47 | 3 | Sham-vaccinated/IBDV STC Challenge Controls | IBDV-STC | 15 | 17SEP2018 | Euthanized | 25 | No |
| 49 | 3 | Sham-vaccinated/BDV STC Challenge Controls | IBDV-STC | 15 | 17SEP2018 | Euthanized | 25 | No |
| 59 | 3 | Sham-vaccinated/IBDV STC **Challenge Controls** | IBDV-STC | 15 | 17SEP2018 | Euthanized | 25 | No |

The following table shows the number of birds with positive bursal disease scores and percent positive by study group. One animal in Group 3 was observed to be depressed following challenge with IBDV-STC.

**Table 49**

| **Group** | **Vaccine/Target Dose** | **Challenge** | **# birds** | **# Positive/ Total # birds** | **% Protection (% Infection)** |
|---|---|---|---|---|---|
| 1 | vHVT522-AI-IBDV 2,500 pfu | IBDV-STC | 20 | 0/20 | 100 |
| 3 | Sham-vaccinated/IBDV STC Challenge Controls | IBDV-STC | 20 | 18/20 | (90) |

The frequency of observed gross lesions is shown in the following table.

**Table 50**

| | | | **# with Sign** | **Total# Birds** |
|---|---|---|---|---|
| **Gross Lesion** | **Group** | **Vaccine/Target Dose** | | |
| **Peri Bursal Edema** | **1** | **vHVT522-AI-IBDV 2,500 pfu** | 0 | 20 |
| | **3** | **Sham-vaccinated/IBDV STC Challenge Controls** | 13 | 20 |
| | **Total** | | 13 | 40 |
| **EDEMA** | **Group** | **Vaccine/Target Dose** | | |
| | **1** | **VHVT522-AI-IBDV 2,500 pfu** | 0 | 20 |
| | **3** | **Sham-vaccinated/IBDV STC Challenge Controls** | 4 | 20 |
| | **Total** | | 4 | 40 |
| **NVL** | **Group** | **Vaccine/Target Dose** | | |
| | **1** | **VHVT522-AI-IBDV 2,500 pfu** | 20 | 20 |
| | **3** | **Sham-vaccinated/IBDV STC Challenge Controls** | 2 | 20 |
| | **Total** | | 22 | 40 |
| **Macroscopic Hemorrhage** | **Group** | **Vaccine/Target Dose** | | |
| | **1** | **VHVT522-AI-IBDV 2,500 pfu** | 0 | 20 |
| | **3** | **Sham-vaccinated/IBDV STC Challenge Controls** | 1 | 20 |
| | **Total** | | 1 | 40 |
| **Other GL** | **Group** | **Vaccine/Target Dose** | | |
| | **1** | **vHVT522-AI-IBDV 2,500 pfu** | 0 | 20 |
| | **3** | **Sham-vaccinated/IBDV STC Challenge Controls** | 1 | 20 |
| | **Total** | | 1 | 40 |

The following table shows the observed bursa weight/body weight (BlEW) ratio by Group and animal ID in those animals challenged with IBDV VAR-E and sham challenged.

**Table 51**

| | | | | **Bursa Weight (g)** | **Body Weight (g)** | **B/BW Ratio** |
|---|---|---|---|---|---|---|
| **Group** | **VaccineTarget Dose** | **Challenge** | **ID** | | | |
| **2** | **vHVT522-Al-IBDV 2,500 pfu** | **IBDV VAR-E** | **62** | 1.16 | 398.00 | 0.29 |
| | | | **63** | 2.11 | 375.00 | 0.56 |
| | | | **64** | 1.56 | 376.00 | 0.41 |
| | | | **65** | 1.01 | 391.00 | 0.26 |
| | | | **66** | 0.55 | 36000 | 0.15 |
| | | | **67** | 0.80 | 392.00 | 0.20 |
| | | | **68** | 1.08 | 384.00 | 0.28 |
| | | | **69** | 0.31 | 336.00 | 0.09 |
| | | | **70** | 0.82 | 373.00 | 0.22 |
| | | | **71** | 1.02 | 454.00 | 0.22 |
| | | | **72** | 1.52 | 402.00 | 0.38 |
| | | | **73** | 2.30 | 418.00 | 0.55 |
| | | | **74** | 2.28 | 388.00 | 0.59 |
| | | | **75** | 0.80 | 353.00 | 0.23 |
| | | | **76** | 2.38 | 403.00 | 0.59 |
| | | | **77** | 1.83 | 408.00 | 0.45 |
| | | | **78** | 0.75 | 377.00 | 0.20 |
| | | | **79** | 2.06 | 360.00 | 0.57 |
| | | | **80** | 2.10 | 340.00 | 0.62 |

**Table 51 - Continued**

| | | | | **Bursa Weight (g)** | **Body Weight (g)** | **B/BW Ratio** |
|---|---|---|---|---|---|---|
| **4** | **Sham-vaccinatedABDV VAR-E Challenge Controls** | **IBDV VAR-E** | **22** | 0.32 | 386.00 | 0.08 |
| | | | **23** | 0.36 | 397.00 | 0.09 |
| | | | **24** | 0.48 | 369.00 | 0.13 |
| | | | **25** | 0.20 | 220.00 | 0.09 |
| | | | **25** | 0.25 | 264.00 | 0.09 |
| | | | **27** | 0.42 | 323.00 | 0.13 |
| | | | **28** | 0.50 | 362.00 | 0.14 |
| | | | **29** | 0.35 | 346.00 | 0.10 |
| | | | **30** | 042 | 479.00 | 0.09 |
| | | | **31** | 0.43 | 484.00 | 0.09 |
| | | | **32** | 0.65 | 463.00 | 0.14 |
| | | | **33** | 0.54 | 435.00 | 0.12 |
| | | | **34** | 0.51 | 380.00 | 0.13 |
| | | | **35** | 0.55 | 451.00 | 0.12 |
| | | | **36** | 0.63 | 383.00 | 0.16 |
| | | | **37** | 0.70 | 384.00 | 0.18 |
| | | | **38** | 0 46 | 269.00 | 0.17 |
| | | | **39** | 0.40 | 353.00 | 0.11 |
| | | | **40** | 0 46 | 367.00 | 0.13 |
| **5** | **Sham-vaccinated, sham-challenged (Negative Controls)** | **Sham-Challenge** | **1** | 2.04 | 434.00 | 0.47 |
| | | | **2** | 1.82 | 455.00 | 0.40 |
| | | | **3** | 1.58 | 377.00 | 0.42 |
| | | | **4** | 2.30 | 429.00 | 0.54 |
| | | | **5** | 2.84 | 494.00 | 0.57 |
| | | | **6** | 2.16 | 418.00 | |
| | | | **7** | 2.12 | 464.00 | 0.59 |
| | | | **8** | 1.97 | 445.00 | 0.41 |
| | | | **9** | 2 09 | 438.00 | 0.48 |
| | | | **10** | 2.44 | 400.00 | 0.61 |
| | | | **11** | 1.83 | 426.00 | 0.43 |
| | | | **12** | 1.43 | 471.00 | 0.30 |
| | | | **13** | 2.55 | 446.00 | 0.57 |
| | | | **14** | 2.48 | 508.00 | 049 |
| | | | **15** | 1.57 | 408.00 | 0.38 |
| | | | **16** | 2.53 | 446.00 | 0.57 |
| | | | **17** | 2.86 | 472.00 | 0.61 |
| | | | **18** | 2.15 | 458.00 | 0.47 |
| | | | **19** | 1.65 | 305.00 | 0.54 |
| | | | **20** | 4.39 | 410.00 | 1.07 |

Boxplots of the bursal to body weight ratios by Group and by sex and Group are shown in FIG. 53 and FIG. 54 respectively.

The following table shows the observed mean B/BW ratios by Group.

**Table 52**

| | | | **# birds** | **Mean Bursa Weight (g)** | **Mean Body Weight (g)** | **Mean B/BW Ratio** |
|---|---|---|---|---|---|---|
| **Group** | **Vaccins/Target Dose** | **Challenge** | | | | |
| **2** | **vHVM22-AI-IBDV 2,500 pfu** | **IBDV VAR-E** | 19 | 1.39 | 384.05 | 0.36 |
| **4** | **Sham-vaccinated/IBDV VAR-E Challenge Controls** | **IBDV VAR-E** | 19 | 0.45 | 374.47 | 0.12 |
| **5** | **Sham-vaccinated sham-challenged (Negative Controls)** | **Sham-Challenge** | 20 | 2.27 | 435.20 | 0.52 |

Summary statistics for the B/B wt. ratios are shown in the table below.

**Table 53**

| | | **N** | **Mean** | **Std** | **Min** | **Q₁** | **Median** | **Q₃** | **Max** |
|---|---|---|---|---|---|---|---|---|---|
| **Group** | **Vaccine/Target Dose** | | | | | | | | |
| **2** | **vHVT522-AI-IBDV 2,500 pfu** | 19 | 0.36 | 0.17 | 0.09 | 0.22 | 0.29 | 0.56 | 0.62 |
| **4** | **Sham-vaccinated/IBDV VAR-E Challenge Controls** | 19 | 0.12 | 0.03 | 0.08 | 0.09 | 0.12 | 0.14 | 0.18 |
| **5** | **Sham-vaccinated, sham-challenged (Negative Controls)** | 20 | 0.52 | 0.15 | 0.30 | 0.44 | 0.50 | 0.57 | 1.07 |

B/B wt. ratios were modeled using linear model where group, sex and group by sex were used as fixed effects for Group 2, 4 and 5. The p-values of the fixed effects are reported in the table below.

**Table 54**

| **Type 3 Tests of Fixed Effects** | | | | |
|---|---|---|---|---|
| **Effect** | **Num DF** | **Den DF** | **F Value** | **Pr > F** |
| **Group** | 2 | 52 | 42.96 | <.0001 |
| **SEX** | 1 | 52 | 2.86 | 0.0969 |
| **Group*SEX** | 2 | 52 | 0.83 | 0.4407 |

Least squares means (LSMeans) of B/B wt. ratios by Group and LSMeans by Sex and Group are reported in the following table.

**Table 55**

| | | **Sex** | |
|---|---|---|---|
| | | **F** | **M** |
| **Group** | **LSM ± StdErr** | **LSM ± StdErr** | **LSM ± StdErr** |
| 2 | 0.36 ± 0.03 | 0.41 ± 0.04 | 0.3 ± 0.04 |
| 4 | 0.12 ± 0.03 | 0.12 ± 0.04 | 0.12 ± 0.05 |
| 5 | 0.52 ± 0.03 | 0.55 ± 0.04 | 0.49 ± 0.04 |

The results of group comparisons and group comparisons by Sex are presented in the following table. The p-values were adjusted for multiple comparisons using Tukey-Kramer method.

**Table 56**

| | | | **Sex** | | | |
|---|---|---|---|---|---|---|
| | | | **F** | | **M** | |
| **Group Comparison** | **p-value** | **Adjusted p-value** | **p-value** | **Adjusted p-value** | **p-value** | **Adjusts d p-value** |
| 2 vs. 4 | <.0001 | <.0001 | <.0001 | <.0001 | 0.0073 | 0.0747 |
| 2 vs. 5 | 0.0004 | 0.0012 | 0.0203 | 0.1774 | 0.0053 | 0.0564 |
| 4 vs. 5 | <.0001 | <.0001 | <.0001 | <.0001 | <.0001 | <.0001 |

Conclusions: Under the conditions of this trial, Avian Influenza-Bursal Disease-Marek's Disease, Serotype 3, Live Marek's Disease Vector (vHVT522-AI-IBDV), was able to provide the required protection against IBDV-STC challenge 21 days post-vaccination. Protection against IBDV-VarE was also observed by the statistically significant differences between the average B/B wt. ratios as compared with the average B/B wt. ratios of the sham-vaccinated challenged birds (VAR-E challenged controls). There also was a statistically significant difference between the average B/B wt. ratios of the challenged controls as compared with the average B/B wt. ratios of the negative controls.

### Example 6 Efficacy of Avian Influenza-Newcastle Disease-Marek's Disease, Serotype 3, Live Marek's Disease Vector (vHVT523-AI-NDV) When Challenged with Newcastle Disease Virus, GB Texas

The objective of this study was to evaluate the efficacy of vHVT523-AI-NDV in day-old birds after challenged with Newcastle Disease Virus.

The primary variable investigated was the development of Newcastle Disease Virus (NDV) clinical signs, including mortality.

The test vaccine was as follows.

**Table 57**

| Product True Name | Avian Influenza-Newcastle Disease-Marek's Disease, Serotype 3, Live Marek's Disease Vector [vHVT523-AI-NDV] |
|---|---|
| Formulation | ∼2,500 PFU/0.2 mL dose |
| Manufacturer | Merial Inc. (US Veterinary Biologics Est. 298) |
| Storage Requirements | Liquid Nitrogen |
| Applied dose and Route | 0.2 mL per dose for SQ route of administration |

The challenge material for this study was as follows.

**Table 58**

| Scientific Name | Newcastle Disease Virus |
|---|---|
| Strain/Relation to Test Vaccine | GB Texas |
| Source of Material | USDA CVB-L |
| Lot or Serial Numbers | 061303 |
| Procedure for Challenge | Dilute in TPB |
| Storage Requirements | -70 °C |
| Applied Dose | ∼10^{4.0} EID₅₀/0.1 mL-dose |
| Route of Administration | 0.1 mL per bird intramuscular (IM) route |

The animals used in this study were as follows.

**Table 59**

| Species | *Gallus domesticus* |
|---|---|
| Strain / Breed | SPF chickens |
| Source | SPAFAS |
| Sex | Female/Male |
| Weight | N/A |
| Age | One-day-old-chicks |
| Number | 40 one-day-old chicks |
| Physiological Status | Healthy and alert |
| Inclusion Criteria | All birds appearing healthy and alert |
| Conditioning / Acclimation | N/A |
| Serologic Status | N/A |
| Exclusion Criteria | Animals that are debilitated, suffering from disease or injury, fractious or otherwise unsuitable for inclusion in the study, in the opinion of the Investigator, were excluded from the study. |
| Animal Identification | The birds were identified according to unit placement and numbered bands. |
| Criteria for Removal from Study | After inclusion in the study, animals that are debilitated, suffering from disease or injury, fractious or otherwise unsuitable to remain in the study, in the opinion of the Investigator, were removed. |

Forty (40) day-old SPF chickens, SPAFAS flock W67 were assigned to Groups 1 or 2 according to the table below. Ten (10) birds were assigned per isolation unit (2 units per group). The birds were vaccinated or sham-vaccinated. Group 1 was vaccinated with Avian Influenza-Newcastle Disease-Marek's Disease, Serotype 3, Live Marek's Disease Vector (vHVT523-AI-NDV), (P3 #1), 11-May-2018 AK, at 2,390 PFU HVT/0.2 mL dose; and Group 2 was sham-vaccinated with Marek's diluent only, Lot DJ612, Exp. Date JAN 20.

**Table 60**

| **Group** | **Vaccine / Target Dose** | **Route/ Volume** | **# of Birds Vaccinated** | **# of Birds Challenged*** |
|---|---|---|---|---|
| 1 | vHVT523-AI-NDV 2,500 PFU/dose | SQ / 0.2 mL | 20 | ∼20 |
| 2 | Sham vaccinated/ Challenge Control | SQ / 0.2 mL | 20 | ∼20 |

All animals were housed in the same manner. The birds were housed in negative pressure isolation units for the duration of the trial. Water and feed were given *ad libitum.*

All birds were observed for Newcastle disease clinical signs including central nervous or respiratory signs, and/or death for up to 14 days post-challenge. At the end of the observation period, the survivors were terminated.

A timeline of the study is shown in the table below.

**Table 61**

| **Study Day or Range** | **Activity** |
|---|---|
| Day 0 | Forty (40), day-old, SPF chickens were assigned to Groups 1 and 2. Ten (10) birds were assigned per isolation unit (2 units per group). The birds were vaccinated or sham-vaccinated by the SQ route before they are placed in the isolation units. All birds were neck-banded for individual identification before challenge. |
| Day 28 | All remaining birds were challenged with NDV Texas GB by the intramuscular (IM) route, 0.1 mL per bird. |
| Day 28 to 42 | All birds were observed daily for any unfavorable reactions to the challenge, particularly nervous or respiratory clinical signs, including death. |
| Day 42 | All the remaining birds were terminated. |

### Results

The following table shows the number of birds positive for NDV and percent positive by study group.

**Table 62**

| **Group** | **Vaccine** / **Actual Dose** | **Route/ Volume** | **# birds** | **# Positive/ Total # birds** | **% Protection (% Infection)** |
|---|---|---|---|---|---|
| 1 | vHVT523-AI-NDV 2,390 pfuldose | SQ/0.2ml | 20 | 0/20 | 100 |
| 2 | Sham-vaccinated/ Challenge Control | SQ/0.2ml | 20 | 20/20 | (100) |

The frequency of positive NDV by isolation unit is shown in the following table.

**Table 63**

| | | | **NDV Result** | |
|---|---|---|---|---|
| | | | **# positive** | **Total # Birds** |
| **Group** | **Vaccine** | **Unit** | | |
| **1** | **vHVT523-AI-NDV 2,500 pfu/dose** | **89** | 0 | 10 |
| | | **90** | 0 | 10 |
| | | **Total** | 0 | 20 |
| **2** | **Sham vaccinated/Challenge Control** | **Unit** | | |
| | | **91** | 10 | 10 |
| | | **92** | 10 | 10 |
| | | **Total** | 20 | 20 |

Conclusions: Under the conditions of this trial, Avian Influenza-Newcastle Disease-Marek's Disease, Serotype 3, Live Marek's Disease Vector (vHVT523-AI-NDV) met the acceptance criteria protecting ≥95% of birds against challenge with virulent Texas GB Newcastle virus. The challenge virus virulence was observed by an infectivity and mortality rate of 100% in the sham-vaccinated challenged control group.

### Example 7 Safety of Avian Influenza-Bursal Disease-Marek's Disease Vaccine, H5 Subtype, Serotype 3, Live Marek's Disease Vector (vHVT522-IBD-AT)

The objective of this study was to evaluate the safety of Avian Influenza-Bursal Disease-Marek's Disease Vaccine, H5 Subtype, Serotype 3, Live Marek's Disease Vector (vHVT522-IBD-AI when back-passed five times in SPF chickens and whether it would spread to non-vaccinated contacts.

### Summary

On Study Day 0, twenty (20) SPF birds, were assigned using a randomization table, into two treatments, vaccinates (15 chicks) and contacts (5 chicks). The fifteen, one-day-old chicks were vaccinated with 0.2 ml of vHVT522-IBD-AI, X+5, Lot 072419, at 8,280 VP2/AI and 7,480 pfu/0.2 ml dose of HVT, and the contacts remained untreated. Birds from Group 1 were designated back-passage 1 (BP1) and were neck-banded for identification according to the randomization table. On Study Days 7, 14, 21, 28, 35, 42 and 49, birds were selected according to the randomization table for sample collections and backpassages, according to Section 4 and Section 5. On Study Days 28, 35, 42, 49 and 77, birds were terminated and necropsied to examine for gross lesions associated with Marek's Disease. Under the conditions of this trial, Avian Influenza-Bursal Disease-Marek's Disease Vaccine, H5 Subtype, Serotype 3, Live Marek's Disease Vector (vHVT522-IBD-AI), X+5 was safe and did not revert to virulence after *in vivo* backpassages. The vaccine also did not spread to non-vaccinated contact birds. However, the study was performed using X+5 material and not MSV.

### Schedule of Events

Day 0: Twenty, one-day-old SPF chicks were assigned using a randomization table into two treatments, vaccinates (15 chicks) and contacts (5 chicks). The fifteen, one-day-old SPF chicks were vaccinated, and the contacts remained untreated. Birds from Group 1 were designated back-passage 1 (BP1) and were neck-banded for identification according to the randomization table. Initial vaccinates and contacts were housed together in the same unit (20 birds per unit).

Day 7: Five initial vaccinates from Group 1 were selected according to the randomization table, bled by cardiac puncture, and terminated. The blood samples were pooled, and an aliquot was used as inoculum, and the remaining was transferred to analytical for buffy coat extractions (BCE) to verify virus recovery. An aliquot of the BCE was saved for future molecular analysis. Twenty, one-day-old SPF chicks were assigned to Group 2, using a randomization table into two treatments, vaccinates (15 chicks) and contacts (5 chicks). The fifteen, one-day-old SPF chicks were designated BP2 and were inoculated with 0.25 ml of pooled blood from G1 (BP1). The contacts remained untreated. All birds from Group 2 were neck-banded for identification according to the randomization table. BP2 inoculated and contact birds were housed together in the same unit (20 birds per unit).

Day 14: Five initial inoculated birds (BP2) from Group 2 were selected according to the randomization table and were bled by cardiac puncture and terminated. The blood samples were pooled, and an aliquot was used as inoculum, and the remaining was transferred to analytical for buffy coat extractions (BCE) to verify virus recovery. An aliquot of the BCE was saved for future molecular analysis. Twenty, one-day-old SPF chicks were assigned to Group 3, using a randomization table into two treatments, vaccinates (15 chicks) and contacts (5 chicks). The fifteen, one-day-old SPF chicks were designated BP3 and were inoculated with 0.25 ml of pooled blood from G2 (BP2). The contacts remained untreated. All birds from Group 3 were neck-banded for identification according to the randomization table. BP3 inoculated and contact birds were housed together in the same unit (20 birds per unit).

Day 21: Five inoculated birds (BP3) from Group 3 were selected according to the randomization table and were bled by cardiac puncture and terminated. The blood samples were pooled, and an aliquot was used as inoculum, and the remaining was transferred to analytical for buffy coat extractions (BCE) to verify virus recovery. An aliquot of the BCE was saved for future molecular analysis. Twenty, one-day-old SPF chicks were assigned to Group 4, using a randomization table into two treatments, vaccinates (15 chicks) and contacts (5 chicks). The fifteen, one-day-old SPF chicks were designated BP4 and were inoculated with 0.25 ml of pooled blood from G3 (BP3). The contacts remained untreated. All birds from Group 4 were neck-banded for identification according to the randomization table. BP4 inoculated and contact birds were housed together in the same unit (20 birds per unit). All the contacts from Group 1 were wing bled, and BCE was conducted individually on these samples to verify virus recovery. These birds were not euthanized and were placed back into the unit of origin after sampling. An aliquot of the BCE samples was saved for molecular analysis.

Day 28: Five inoculated birds (BP4) from Group 4 were selected according to the randomization table and were bled by cardiac puncture and terminated. The blood samples were pooled, and an aliquot was used as inoculum, and the remaining was transferred to analytical for buffy coat extractions (BCE) to verify virus recovery. An aliquot of the BCE was saved for future molecular analysis. Twenty, one-day-old SPF chicks were assigned to Group 5, using a randomization table into two treatments, vaccinates (15 chicks) and contacts (5 chicks). The fifteen, one-day-old SPF chicks were designated BP5 and were inoculated with 0.25 ml of pooled blood from G4 (BP4). The contacts remained untreated. All birds from Group 5 were neck-banded for identification according to the randomization table. BP5 inoculated and contact birds were housed together in the same unit (20 birds per unit). Additional twenty-one day-old SPF chicks were assigned to Group 6, using a randomization table into two treatments, vaccinates (15 chicks) and contacts (5 chicks). The fifteen chicks were designated vaccinates and were vaccinated with vHVT522-IBD-AI. The contacts remained untreated. All birds from Group 6 were neck-banded for identification according to the randomization table. vHVT522-IBD-AI vaccinated, and contact birds were housed together in the same unit (20 birds per unit). All the contacts from Group 2 were wing bled, and BCE was conducted individually on these samples to verify virus recovery. These birds were not euthanized and were placed back into the unit of origin after sampling. An aliquot of the BCE samples was saved for molecular analysis. All remaining birds from Group 1 were terminated and necropsied to examine for gross lesions associated with Marek's disease.

Day 35: All the contacts from Group 3 were wing bled, and BCE was conducted individually on these samples to verify virus recovery. These birds were not euthanized and were placed back into the unit of origin after sampling. An aliquot of the BCE samples was saved for molecular analysis. All remaining birds from Group 2 were terminated and necropsied to examine for gross lesions associated with Marek's disease.

Day 42: Five inoculated birds (BP5) from Group 5, and five vaccinates from Group 6 were selected according to the randomization table and were bled by cardiac puncture and terminated. The blood samples were pooled and transferred to analytical for buffy coat extractions (BCE) to verify virus recovery. An aliquot of the BCE was saved for future genetic stability analysis. All the contacts from Group 4 were wing bled, and BCE were conducted individually on these samples to verify virus recovery. These birds were not euthanized and were placed back into the unit of origin after sampling. An aliquot of the BCE samples was saved for molecular analysis. All remaining birds from Group 3 were terminated and necropsied to examine for gross lesions associated with Marek's disease.

Day 49: All the contacts from Groups 5 and 6 were wing bled, and BCE was conducted individually on these samples to verify virus recovery. An aliquot of the BCE samples was saved for molecular analysis. All remaining birds from Group 4 were terminated and necropsied to examine for gross lesions associated with Marek's disease.

Day 77: All remaining birds from Groups 5 and 6 were terminated and necropsied to examine for gross lesions associated with Marek's disease.

### Study Design

One hundred and twenty (120) one-day-old SPF chicks (species *Gallus domesticus*), males and females, were used for this study. The study design is shown in Table 64 below.

**Table 64**

| **Group** | **Treatment (Study Day)** | **Inoculum** | **Route** | **Volume (ml)** | **Age (Days)** | **# of Birds Vaccinate s /Contacts** |
|---|---|---|---|---|---|---|
| 1 | BP1 vHVT522-IBD-AI/ BP1 Contacts (Dav 0) | vHVT522-IBD-AI/ NA | SQ/NA | 0.20 | 1 | 15/5 |
| 2 | BP2 vHVT522-IBD-AI/ BP2 vHVT522-IBD-AI Contacts (Dav 7) | Pooled blood BP1 vHVT522-IBD-AI/ NA | SQ/NA | 0.25 | 1 | 15/5 |
| 3 | BP3 vHVT522-IBD-AI/ BP3 vHVT522-IBD-AI Contacts (Day 14) | Pooled blood BP2 vHVT522-IBD-AI/ NA | SQ/NA | 0.25 | 1 | 15/5 |
| 4 | BP4 vHVT522-IBD-AI/ BP4 vHVT522-IBD-AI Contacts (Dav 21) | Pooled blood BP3 vHVT522-IBD-AI/ NA | SQ/NA | 0.25 | 1 | 15/5 |
| 5 | BP5 vHVT522-IBD-AI/ BP5 vHVT522-IBD-AI Contacts (Dav 28) | BP4 vHVT522-IBD-AI/ NA | SQ/NA | 0.25 | 1 | 15/5 |
| 6 | vHVT522-IBD-AI/ vaccinates/Contacts (Day 28) | vHVT522-IBD-AI/ NA | SQ/NA | 0.20 | 1 | 15/5 |

### Test Vaccine

The vaccine was diluted in Marek's diluent for Group 1 to yield 8,280 plaque forming units (pfu) per dose as determined by the VP2 and AI genes; 7,480 as determined by HVT; or for Group 6 to yield 9,640 plaque forming units (pfu) per dose as determined by the VP2 and AI genes; or 8,680 as determined by HVT. The vaccine was stored in liquid Nitrogen.

### Treatments

The birds were vaccinated one time or inoculated with backpassage material, at one day of age, by the SQ route. All vaccinations and inoculations were recorded on the Inoculation/Placement Record Form. Birds were sampled according to the timeline set forth above. Birds were observed for 28 days (Group 1-4) or 49 days (Groups 5 and 6), followed by necropsy. Clinical signs of Marek's, mortality, and gross lesions (when present) were recorded on the Necropsy Record Form.

### Clinical Observations

Throughout the study, all chickens were observed daily for their overall health and wellbeing and for any adverse reactions to the vaccine. All the observations were recorded in the Study Log. The birds were observed until 28 days post-hatch (Groups 1-4), or 49 days posthatch (Groups 5 and 6) for clinical signs attributed to Marek's disease including but not limited to: paralysis, locomotive signs, and severe emaciation or depression. Clinical signs, if present, were recorded on the Necropsy Record Form.

### HVT recovery - BCE for Blood samples

Blood samples were handled individually or pooled for BCE (Buffy Coat Extraction). Each BCE sample was inoculated separately on to prepared chicken embryo fibroblasts (CEF). The plates were incubated at 37°C for five days and then inspected for HVT cytopathic effect (CPE). In addition to CPE, the plaques isolated from the last backpassage was stained with specific antibodies.

### Results and Evaluation

The results are presented in Tables 65-67 below.

**Table 65**

| Results for Pooled Samples by Day and Group | | | |
|---|---|---|---|
| **Group** | **Treatment** | **Day** | **Result (MDV CPE)** |
| 1 | BP1 vHVT522-IBD-AI | 7 | Positive |
| 2 | BP2 vHVT522-IBD-AI | 14 | Positive |
| 3 | BP3 vHVT522-IBD-AI | 21 | Positive |
| 4 | BP4 vHVT522-IBD-AI | 28 | Positive |
| 5 | BP5 vHVT522-IBD-AI | 42 | Positive |
| 6 | vHVT522-IBD-AI vaccinates | 42 | Positive |

**Table 66**

| Results for Contacts by Day and Group | | | |
|---|---|---|---|
| **Group** | **Treatment** | **Day** | **Result (MDV CPE) (Sampled/Positive)** |
| 1 | BP1 vHVT522-IBD-AI Contacts | 21 | Negative (0/5) |
| 2 | BP2 vHVT522-IBD-AI Contacts | 28 | Negative (0/5) |
| 3 | BP3 vHVT522-IBD-AI Contacts | 35 | Negative (0/5) |
| 4 | BP4 vHVT522-IBD-AI Contacts | 42 | Negative (0/5) |
| 5 | BP5 vHVT522-IBD-AI Contacts | 49 | Negative (0/5) |
| 6 | vHVT522-IBD-AI vaccinates Contacts | 49 | Negative (0/5) |

| Table 67 | | | | |
|---|---|---|---|---|
| **Group** | **Type** | **Treatment** | **# Birds** | **# Positive/T otal # Birds** |
| 1 | Vaccinates | BP1 vHVT522-IBD-AI | 10 | 0/10 |
| | Contacts | BP1 Contacts | 5 | 0/5 |
| 2 | Vaccinates | BP2 vHVT522-IBD-AI | 10 | 0/10 |
| | Contacts | BP2 vHVT522-IBD-AI Contacts | 5 | 0/5 |
| 9 | Vaccinates | BP3 vHVT522-IBD-AI | 10 | 0/10 |
| | Contacts | BP3 vHVT522-IBD-AI Contacts | 5 | 0/5 |
| 4 | Vaccinates | BP4 vHVT522-IBD-AI | 10 | 0/10 |
| | Contacts | BP4 vHVT522-IBD-AI Contacts | 5 | 0/5 |
| 5 | Vaccinates | BP5 vHVT522-IBD-AI | 9 | 0/9 |
| | Contacts | BP5 vHVT522-IBD-AI Contacts | 5 | 0/5 |
| 6 | Vaccinates | vHVT522-IBD-AI vaccinates | 10 | 0/10 |
| | Contacts | Contacts | 5 | 0/5 |

### Discussion/Conclusion

Under the conditions of this trial, Avian Influenza-Bursal Disease-Marek's Disease Vaccine, H5 Subtype, Serotype 3, Live Marek's Disease Vector (vHVT522-IBD-AI), X+5 was safe and did not revert to virulence after *in vivo* backpassages. The vaccine also did not spread to non-vaccinated contact birds. However, the study was performed using X+5 material and not MSV and the backpassage portion will be repeated with MSV.

### Example 8 Evaluation of the Efficacy of Avian Influenza-Bursal Disease-Marek's Disease Vaccine, H5 Subtype, Serotype 3, Live Marek's Disease Vector (vHVT522-IBD-AI), X+5, Administered In ovo or Subcutaneously to One-Day-Old SPF Chickens. Against vMDV, GA22 Virus of Avian Influenza-Bursal Disease-Marek's Disease Vaccine, H5 Subtype, Serotype 3, Live Marek's Disease Vector (vHVT522-IBD-AI)

The objective of this study was to evaluate the efficacy of Avian Influenza-Bursal Disease-Marek's Disease Vaccine, H5 Subtype, Serotype 3, Live Marek's Disease Vector (vHVT522-IBD-AI), X+5, administered *in ovo* or subcutaneously to one-day-old SPF chicks.

### Summary

On Study Day 0, 18-19-day-old embryos, SPF, SPAFAS flock T41, in Groups 1 and 3 were vaccinated or sham-vaccinated. The embryos in Groups 2 and 4 (~46 embryos per group) were incubated without receiving any treatment. Embryos from Group 1 were *in ovo* vaccinated, with 0.05ml of vHVT522-IBD-AI, X+5, at 2,330AI /VP2 and 2,180pfu/0.05 ml dose of HVT. The inoculated embryos were placed back into a humidity-controlled incubator. Following vaccination and sham vaccination, the embryos in Group 1 were incubated and hatched separately from Groups 2-4. On Study Day 3, thirty-five (35) birds were randomly chosen from the viable, hatched chicks from Groups 1-3, and twenty-five (25) from Group 4, by using a randomization table. The 130 one-day-old SPF chickens from Groups 1-4 were assigned isolation units and numbered bands according to the randomization table. All birds in Groups 1, 3 and 4 were placed into the isolation units without receiving any treatment. The birds in Group 2 were subcutaneously (SQ) vaccinated with 0.2ml of vHVT522-IBD-AI, X+5, at 2,280 AI/VP2 and 2,070pfu/0.2 ml dose of HVT. All birds were neck banded for individual identification before placement. On Study Day 7, all birds (5 days of age) from Groups 1-3 were challenged with 0.2ml per bird, by the intraperitoneal route, with vMDV GA22, at 27,600 cells/0.2 ml dose. Between Study Days 7-52, all birds were observed daily for any unfavorable reactions to the challenge. On Study Day 52, all the remaining birds were terminated and necropsied to examine for gross lesions associated with Marek's disease. Under the conditions of this trial, birds in Groups 1 and 2, vaccinated with Avian Influenza-Bursal Disease-Marek's Disease Vaccine, H5 Subtype, Serotype 3, Live Marek's Disease Vector (vHVT522-IBD-AI), X+5, met the acceptance criteria for protection against vMDV. The virus virulence was confirmed by the infectivity observed in Group 3. The minimum protective dose was established at 2,070pfu/0.2 ml dose for one-day-old chickens when administered by the SQ route, and 2,180pfu/0.05 ml dose when administered by the *in ovo* route.

### Schedule of Events

Day 0: Eighteen to nineteen-day-old embryos in Groups 1 and 4 were vaccinated or sham-vaccinated. The embryos in Groups 2 and 3 (~90 embryos) were incubated without receiving any treatment, and the embryos in Group 4 were sham inoculated with Marek's disease vaccine diluent only. The inoculated embryos were placed back into a humidity-controlled incubator. Following vaccination and sham vaccination, the embryos in Groups 2-4 were incubated and hatched separately from Group 1.

Day 3: Thirty-five (35) birds were randomly chosen from the viable, hatched chicks from Groups 1-3, and twenty-five (25) from Group 4, by using a randomization table. The 130 one-day-old SPF chickens from Groups 1-4 were assigned isolation units and numbered bands according to the randomization table. Eleven to thirteen birds were assigned per isolation unit (3 units for Groups 1-3; and 2 units for Group 4). All birds in Groups 1, 3 and 4 were placed into the isolation units without receiving any treatment. The birds in Group 2 were SQ vaccinated. All birds were neck banded for individual identification before placement.

Day 7: All birds (5 days of age) from Groups 1-3 were challenged as described below.

Day 7-52: The birds were observed daily for any unfavorable reactions to the challenge.

Day 52: The birds were terminated and necropsied to examine for gross lesions associated with Marek's disease.

### Study Design

Two hundred and ten (210) embryos/130 one-day-old SPF chicks (species *Gallus domesticus*) SPAFAS flock T41 males and females, were used for this study. The study design is shown in Table 68 below.

**Table 68**

| **Group** | **Vaccine PFU/dose*** | **Dose Volume! Route** | **∼Number of Embryos/Birds Vaccinated** | **No. of Birds Placed** | **Challenge*** |
|---|---|---|---|---|---|
| 1 | vHVT522-IBD-AI | 0.05 ml/*in ovo* | 59 (embryos) | 35 | Yes |
| | AI/VP2=2,330 | | | | |
| | HVT = 2,180 | | | | |
| 2 | vHVT522-IBD-AI | 0.2 ml/SQ | 35** (birds) | 35 | Yes |
| | AI/VP2=2,280 | | | | |
| | HVT = 2,070 | | | | |
| 3 | Sham-vaccinated/ Challenge Controls | 0.05 ml/*in ovo* | 59 (embryos) | 35 | Yes |
| 4 | Non-Vaccinated/non-challenged (Negative controls) | N/A | N/A** | 25 | No |

| | | | | | |
|---|---|---|---|---|---|
| *All birds from Groups 1-3 were challenged with vMDV, GA22 strain at 5 days of age by the intraperitoneal route (IP). **Forty-six 18-19 day-old embryos per group (Groups 2 and 4) were set on Study Day 0 and assigned to Groups 2 and 4. Thirty-five (35) birds were used for SQ vaccination for Group 2 on Study Day 3. | | | | | |

### Test Vaccine

The vaccine was diluted in Marek's diluent to yield 2,330 plaque forming units (pfu) per dose as determined by the VP2 and AI genes; or 2,180 as determined by HVT for the *in ovo* vaccination. For the SQ vaccination, the vaccine was diluted in Marek's diluent to yield 2,280 pfu per dose as determined by the VP2 and AI genes; or 2,070 as determined by HVT. The vaccine was stored in Liquid Nitrogen. The Control Product (CP) was Marek's disease vaccine diluent (sham vaccine). The Challenge Material was virulent Marek's Disease Virus (vMDV), isolate GA22.

### Treatments

The birds in Groups 1-3 were vaccinated or sham-vaccinated one time either by the *in ovo* route (18-19 days of embryonation) or by the SQ route at one day of age, with vHVT522-IBD-AI, or Marek's diluent. Birds were neck banded on Study Day 3, and Groups 1-3 were challenged on Study Day 7. All birds were observed until Study Day 52, and all the remaining birds were necropsied at the end of the experimental period.

### Clinical Observations

Throughout the study, all chickens were observed daily for their overall health and wellbeing and for any adverse reactions to the vaccine. All the observations were recorded in the Study Log. At the end of the observation period (Study Day 52), all the remaining birds were euthanized and necropsied and examined for gross lesions of Marek's disease. Lesions included, but were not limited to, the following organs: liver, heart, spleen, gonads, kidneys, skin, muscle lesions and nerve enlargement. One to five birds per unit were selected form three different units in order to necropsy 1-15 birds at a time until all the birds in the study were necropsied. The order of the units in which the birds were necropsied was randomly chosen according to a randomization table. Gross lesions and mortality were recorded in the Necropsy Record Form.

### Results and Evaluation

The results are presented in Tables 69-70 below.

**Table 69**

| Vaccination/Hatch Results | | | | | |
|---|---|---|---|---|---|
| **Group** | **Route** | **Total Eggs Set** | **Total Eggs Vaccinated** | **Total # Hatched** | **% Hatched** |
| 1 | *In ovo*/0.05ml | 59 | 59 | 49 | 83.1 |
| 2 | In ovo/0.05ml | 59 | 59 | 51 | 86.4 |

**Table 70**

| Number of Birds Positive for Marek's and Percent Positive by Group | | | | | |
|---|---|---|---|---|---|
| **Group** | **Vaccine Actual PFU/dose** | **Route / Volu me** | **Challenge** | **# Positive/ Total # Birds** | **% Protection (% Infection)** |
| 1 | vHVT522-IBD-AI | *In ovo* /0.05 ml | vMDV | 2/35 | 94.3 |
| | AI/VP2=2,330 | | | | |
| | HVT = 2,180 | | GA22 | | |
| 2 | vHVT522-IBD-AI | SQ/ 0.2 ml/ | vMDV | 2/34 | 94.1 |
| | AI/VP2=2,280 | | | | |
| | HVT = 2,070 | | GA22 | | |
| 3 | Sham-vaccinated/ Challenge Controls | *In ovo* /0.05 ml | vMDV | 32/35 | (94.4) |
| | | | GA22 | | |
| 4 | Non-Vaccinated! non-challenged (Negative controls) | N/A | N/A | 0/24 | 100 |

### Discussion/Conclusion

Under the conditions of this trial, birds in Groups 1 and 2, vaccinated with Avian Influenza-Bursal Disease-Marek's Disease Vaccine, H5 Subtype, Serotype 3, Live Marek's Disease Vector (vHVT522-IBD-AI), X+5, met the acceptance criteria for protection against vMDV. The virus virulence was confirmed by the infectivity observed in Group 3. The minimum protective dose was established at 2,070 pfu/0.2 ml dose for one-day-old chickens when administered by the SQ route and 2,180 pfu/0.05 ml dose when administered by the *in ovo* route.

### Example 9 Evaluation of the Efficacy of Avian Influenza-Bursal Disease-Marek's Disease Vaccine, H5 Subtype, Serotype 3, Live Marek's Disease Vector (vHVT522-IBD-AI), X+5, Administered In ovo or Subcutaneously to One-Day-Old SPF Chickens, Against Infectious Bursal Disease Virus, STC Classic Strain

The objective of this study was to evaluate the efficacy of Avian Influenza-Bursal Disease-Marek's Disease Vaccine, H5 Subtype, Serotype 3, Live Marek's Disease Vector (vHVT522-IBD-AI), X+5, administered *in ovo* to 18-19-day-old embryos or subcutaneously to one-day-old SPF chicks.

### Summary

On Study Day 0, 18-19-day-old embryos, SPF, SPAFAS flock T41, in Groups 1 and 3 were vaccinated or sham-vaccinated. The embryos in Group 2 (75 embryos per group) were incubated without receiving any treatment. Embryos from Group 1 were *in ovo* vaccinated, with 0.05ml of vHVT522-IBD-AI, X+5, at 2,620 pfu/0.05 dose of AIV HA and IBDV VP2 and 2,490 pfu/0.05 ml dose of HVT. The inoculated embryos were placed back into a humidity-controlled incubator. Following vaccination and sham vaccination, the embryos in Group 1 were incubated and hatched separately from Groups 2 and 3. On Study Day 3, thirty-six (36) birds were randomly chosen from the viable, hatched chicks from Groups 1-3 by using a randomization table. All birds in Groups 1 and 3 were placed into the isolation units without receiving any treatment. The birds in Group 2 were subcutaneously (SQ) vaccinated with 0.2ml of vHVT522-IBD-AI, X+5, at 2,170 pfu/0.2 dose of AIV HA and IBDV VP2 and 1,950 pfu/0,2 ml dose of HVT. On Study Day 25, the number of birds were reduced to 30 birds per group (ten birds per unit) for Groups 1-3 using a randomization schedule and all the extra birds were euthanized. All the remaining birds were neck-banded for individual identification. On Study Day 31, all the remaining birds were challenged with 0.03ml per bird, by the intra-ocular route, with IBDV-STC, STC EP1, Lot 080116, at102.7 EID50/0.03 ml dose. Between Study Days 32-35, all birds were observed daily for any unfavorable reactions to the challenge. On Study Day 35, all the remaining birds were euthanized and necropsied to examine for the presence of gross bursal lesions. Under the conditions of this trial, birds in Groups 1 and 2, vaccinated with Avian Influenza-Bursal Disease-Marek's Disease Vaccine, H5 Subtype, Serotype 3, Live Marek's Disease Vector (vHVT522-IBD-AI), X+5, met the acceptance criteria for protection against IBDV-STC. The virus virulence was confirmed by the infectivity observed in Group 3. The minimum protective dose was established at 2,170 pfu/0.2 ml dose for one-day-old chickens when administered by the SQ route, and 2,620 pfu/0.05 ml dose when administered by the *in ovo* route, following 9 CFR 113.331(c)(3) requirements.

### Schedule of Events

Day 0: Eighteen to nineteen-day-old embryos in Groups 1 and 3 were vaccinated or sham-vaccinated. The embryos in Group 2 (75 embryos) were incubated without receiving any treatment. All embryos were placed back into a humidity-controlled incubator. Following vaccination and sham vaccination, the embryos in Group 1 were incubated and hatched separately from Groups 2 and 3.

Day 3: Thirty-six (36) birds were randomly chosen from the viable, hatched chicks from Groups 1-3 by using a randomization table. The 108 one-day-old SPF chickens from Groups 1-3 were assigned isolation units. Twelve birds were assigned per isolation unit (3 units per Group). All birds in Groups 1 and 3 were placed into the isolation units without receiving any treatment on Study Day 3. The birds in Group 2 were SQ vaccinated.

Day 25: The number of birds was reduced to 30 birds per group (ten birds per unit) for Groups 1-3 using a randomization schedule and all the extra birds were euthanized. All the remaining birds were neck-banded for individual identification.

Day 31: All the remaining birds were challenged (28 days of age) as described below.

Day 32-35: All birds were observed for any unfavorable reactions to the challenge for 4 days post-challenge.

Day 35: All remaining birds were euthanized and necropsied to examine for gross bursal lesions.

### Study Design

Two hundred and twenty-five (225) embryos/108 one-day-old SPF chicks (species *Gallus domesticus*) SPAFAS flock T41 males and females, were used for this study. The study design is shown in Table 71 below.

**Table 71**

| **Group** | **Vaccine PFU/dose*** | **Dose Volume/ Route** | **Number of Embryos/Birds Vaccinated** | **No. of Birds Placed** | **Number of Birds at Challenge** |
|---|---|---|---|---|---|
| 1 | vHVT522-IBD-AI | 0.05 ml/*in ovo* | 75 (embryos) | 36 | 30 |
| | AIV HA and IBDV | | | | |
| | VP2=2,620 | | | | |
| | HVT = 2,490 | | | | |
| 2 | vHVT522-IBD-AI | 0.2 ml/SQ | 36 (birds)¹ | 36 | 30 |
| | AI/VP2=2,280 | | | | |
| | HVT = 2,070 | | | | |
| 3 | Sham-vaccinated/ Challenge Controls | 0.05 ml/*in ovo* | 59 (embryos) | 36 | 30 |

| | | | | | |
|---|---|---|---|---|---|
| ¹Seventy-five (75) 18-19 day-old embryos were set on Study Day 0 and assigned to Group 2. Thirty-six (36) birds were used for AQ vaccination for Group 2 on Study Day 3. | | | | | |

### Test Vaccine

The vaccine was diluted in Marek's diluent to yield 2,620 plaque forming units (pfu) per dose as determined by the AIV HA and IBDV VP2 genes; or 2,490 as determined by HVT for the *in ovo* vaccination. For the SQ vaccination, the vaccine was diluted in Marek's diluent to yield 2,170 pfu per dose as determined by the IBD2 and AIV HA genes; or 1,950 as determined by HVT. The vaccine was stored in Liquid Nitrogen. The Control Product (CP) was Marek's disease vaccine diluent (sham vaccine). The Challenge material was Infectious Bursal Disease Virus (IBDV-STC) lot number STC EP1, lot 080116, stored at -70°C and a dose of 10^{2.7} EID₅₀/0.03 ml dose (Intra-ocular route)

### Treatments

The birds were vaccinated one time, either by the *in ovo* route (18-19 days of embryonation) or by the SQ route (one day of age), with vHVT522-IBD-AI. All vaccinations were recorded on the Hatch/Vaccination Record Form from Groups that received *in ovo* vaccination or sham-vaccination (Groups 1 and 3).

Birds were challenged at 28 days of age (Study Day 31). The challenge was recorded on the Challenge Record Form by an un-blinded personnel. All Challenge birds were observed for 4 days post-challenge, followed by euthanasia and necropsy to examine for the presence of gross lesions at the bursa of Fabricius. Gross lesions and mortality were recorded on the IBDV STC Score Record Form.

### Clinical Observations

Throughout the study, all chickens were observed daily for their overall health and wellbeing and for any adverse reactions to the vaccine or to challenge (Days 32 to 35). All the observations were recorded in the Study Log.

At the end of the observation period (Study Day 35), the birds were examined for gross lesions due to the classical-STC IBDV challenge. One to five birds per unit were selected from three different units in order to necropsy 1-15 birds at a time, until all the birds in the study were necropsied. The order of the units in which the birds were necropsied was randomly chosen according to a randomization table . Necropsy results were recorded in the IBDV-STC Score Record Form. For the purposes of this test, gross lesions should include peri-bursal edema and/or edema and/or macroscopic hemorrhage in the bursal tissue. Any bird with these stated lesions was recorded as positive.

The vaccine preparations were titrated five times to determine the actual dose given (ADG). Titers were determined for all vaccine fractions including Marek's Disease Virus, Infectious Bursal Disease Virus(VP2) and Avian Influenza gene (HA) inserts.

### Results and Evaluation

The results are presented in Tables 72-74 below.

**Table 72**

| Vaccination/Hatch Results | | | | | |
|---|---|---|---|---|---|
| **Group** | **Vaccine PFU/dose** | **Route** | **# Eggs Vaccinated** | **# of Eggs Hatched** | **% Hatched** |
| 1 | vHVT522-IBD-AI | *In* ovo/0.05ml | 75 | 69 | 92 |
| | AIV HA and IBDV | | | | |
| | VP2=2,620 | | | | |
| | HVT = 2,490 | | | | |
| 3 | Sham-vaccinated/ Challenge Controls | *In* ovo/0.05ml | 75 | 60 | 80 |

**Table 73**

| Number of Birds Positive for Marek's and Protection or Infection by Group | | | | |
|---|---|---|---|---|
| **Group** | **Vaccine PFU/dose** | **Route/ Volume** | **# Positive/ Total # Birds** | **% Protection (% Infection)** |
| 1 | vHVT522-IBD-AI | *In ovo* /0.05 ml | 3/30 | 90 |
| | AIV HA and IBDV | | | |
| | VP2=2,620 | | | |
| | HVT = 2,490 | | | |
| 2 | vHVT522-IBD-AI | SQ/ 0.2 ml/ | 0/30 | 94.1 |
| | AIV HA & IBDV VP2=2, 170 | | | |
| | HVT = 1,950 | | | |
| 3 | Sham-vaccinated/ Challenge Controls | *In ovo* /0.05 ml | 27/30 | (90) |

### Discussion/Conclusion

Under the conditions of this trial, birds in Groups 1 and 2, vaccinated with Avian Influenza-Bursal Disease-Marek's Disease Vaccine, H5 Subtype, Serotype 3, Live Marek's Disease Vector (vHVT522-IBD-AI), X+5, met the acceptance criteria for protection against IBDV-STC. The virus virulence was confirmed by the infectivity observed in Group 3. The minimum protective dose was established at 2,170 pfu/0.2 ml dose for one-day-old chickens when administered by the SQ route and 2,620 pfu/0.05 ml dose when administered by the *in ovo* route.

The related applications described above, all documents cited therein or during their prosecution ("application cited documents"), all documents cited or referenced in the application-cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

Having thus described in detail preferred embodiments of the present invention, it is to be understood that the invention defined by the above paragraphs is not to be limited to particular details set forth in the above description as many apparent variations thereof are possible without departing from the spirit or scope of the present invention.
The present invention is further described in the following numbered paragraphs:
1. A recombinant virus vector comprising:
   at least one nucleic acid sequence derived from a predetermined virus selected from: herpesvirus of turkeys (rHVT); Marek's disease virus; or Meleagrid herpesvirus;
   a first exogenous nucleic acid sequence from an avian influenza virus such that the recombinant virus vector expresses *in vivo* an exogenous avian influenza virus hemagglutinin antigen; and
   a second exogenous nucleic acid sequence from an avian disease encoding for and expressing at least one antigen of the avian disease *in vivo.*
2. The recombinant virus vector of paragraph 1 wherein the predetermined virus comprises Marek's disease virus serotype 3.
3. The recombinant virus vector of paragraph 1 wherein the predetermined virus comprises Marek's disease virus strain FC-126.
4. The recombinant virus vector of paragraph 1 wherein the avian influenza is of subtype H5.
5. The recombinant virus vector of paragraph 1 wherein the first exogenous nucleic acid sequence encoding the avian influenza HA antigen is codon optimized.
6. The recombinant virus vector of paragraph 1 wherein the second exogenous nucleic acid sequence encodes an infectious bursal disease virus antigen, a Newcastle disease antigen, or an infectious laryngotracheitis virus (ILTV) antigen.
7. The recombinant virus vector of paragraph 1 wherein the second exogenous nucleic acid sequence encodes an infectious bursal disease virus VP2 antigen or a Newcastle disease F antigen.
8. The recombinant virus vector of paragraph 1 constructed by a recombination method comprising transfecting a cell with the predetermined virus and an insertion plasmid for insertion into an Intergenic I (IG1) site comprising the first exogenous nucleic acid sequence encoding the infectious bursal disease virus VP2 antigen, an Internal Ribosome Entry Site (IRES), the second exogenous nucleic acid sequence encoding avian influenza HA and SV40 poly A tail, flanked by sequences from the IG1 region.
9. The recombinant virus vector of paragraph 1 further comprising an amino acid sequence having at least 80 percent, 85 percent, 90 percent, 95 percent, 96 percent, 97 percent, 98 percent, or 99 percent sequence identity to a sequence encoding an AIV H5 HA protein.
10. The recombinant virus vector of paragraph 1 wherein an amino acid sequence for a AIV HA COBRA-C gene as used in the recombinant virus vector has at least 80 percent, 85 percent, 90 percent, 95 percent, 96 percent, 97 percent, 98 percent, or 99 percent sequence identity to a predetermined sequence.
11. The recombinant virus vector of paragraph 1 wherein an amino acid sequence for an IBDV VP2 gene of the recombinant virus vector has at least 80 percent, 85 percent, 90 percent, 95 percent, 96 percent, 97 percent, 98 percent, or 99 percent sequence identity to a predetermined sequence.
12. The recombinant virus vector of paragraph 1 wherein an amino acid sequence for an NDV-F gene of the recombinant virus vector has at least 80 percent, 85 percent, 90 percent, 95 percent, 96 percent, 97 percent, 98 percent, or 99 percent sequence identity to a predetermined sequence.
13. A method for producing the recombinant virus vector of paragraph 1 comprising a recombination method comprising transfecting a cell with the predetermined virus and an insertion plasmid for insertion into HVT Intergenic 1 (IG1) site comprising the first exogenous nucleic acid sequence encoding the infectious bursal disease virus VP2 antigen, an Internal Ribosome Entry Site (IRES), the second exogenous nucleic acid sequence encoding avian influenza HA and SV40 poly A tail, flanked by sequences from the IG1 region.
14. A composition comprising the recombinant virus vector of paragraph 1 and a veterinarily acceptable carrier.
15. The composition of paragraph 14 wherein the predetermined virus is in an amount, when the composition is administered to an avian, sufficient to induce an immune response in the avian against Marek's Disease Virus and/or infectious bursal disease virus and/or avian influenza.
16. A method for inducing an immune response in an avian against Marek's Disease Virus, Avian Influenza, and Infectious Bursal Disease Virus, comprising administering to the avian an effective amount of the recombinant virus vector of paragraph 1, or an effective amount of the composition of paragraph 14.
17. A recombinant virus vector comprising and expressing *in vivo*:
   an exogenous avian influenza virus HA antigen, and
   an exogenous nucleic acid molecule encoding a Newcastle Disease Virus F antigen.
18. The recombinant virus vector of paragraph 1 or 17 wherein the avian influenza is of subtype H5.
19. The recombinant virus vector of paragraph 17 wherein the exogenous nucleic acid molecule encoding the avian influenza HA antigen is codon optimized.
20. The recombinant virus vector of paragraph 17 constructed by a recombination method comprising transfecting a cell with a predetermined virus and an insertion plasmid for the Intergenic 1 (IG1) site comprising a mouse CMV promoter the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.
21. A method for producing the recombinant virus vector of paragraph 17 comprising a recombination method comprising transfecting a cell with predetermined virus and an insertion plasmid for the Intergenic 1 (IG1) site comprising a mouse CMV promoter, the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.
22. The method of paragraph 13 or 21 further comprising recovering the recombinant virus vector.
23. A composition comprising the recombinant virus vector of paragraph 21 and a veterinarily acceptable carrier.
24. The composition of paragraph 23 wherein the recombinant virus vector is in an amount, when the composition is administered to an avian, sufficient to induce an immune response in the avian against Marek's Disease Virus and/or avian influenza virus and/or Newcastle Disease Virus.
25. The composition of paragraph 14 or 23 wherein the avian is a chicken, capon, duck, goose, turkey, pheasant, grouse, quail, swan, squab, or pigeon.
26. The composition of paragraph 14 or 23 wherein the avian is a chicken.
27. A method for inducing an immune response in an avian against Marek's Disease Virus, Avian Influenza and Newcastle Disease Virus, comprising administering to the avian an effective amount of the recombinant virus vector of paragraph 17, or an effective amount of the composition of paragraph 23.
28. The method of paragraph 16 or 27 wherein the avian is a chicken, capon, duck, goose, turkey, pheasant, grouse, quail, swan, squab, or pigeon.
29. The method of paragraph 16 or 27 wherein the avian is a chicken.
30. A plasmid for insertion into an Intergenic 1 (IG1) site of a predetermined virus comprising an exogenous nucleic acid sequence encoding the infectious bursal disease virus VP2 antigen, an Internal Ribosome Entry Site (IRES), an exogenous nucleic acid sequence encoding avian influenza HA and SV40 poly A tail, flanked by sequences from the IG1 region.
31. A plasmid for insertion into an Intergenic 1 (IG1) site of a predetermined virus comprising a mouse CMV promoter, the exogenous nucleic acid sequence encoding the Newcastle Disease Virus F antigen, an Internal Ribosome Entry Site (IRES), the exogenous nucleic acid sequence encoding avian influenza HA and SV40 poly A tail, flanked by sequences from the IG1 region.
32. The plasmid of paragraph 30 or 31 wherein the avian influenza is of subtype H5.
33. The plasmid of paragraph 30 or 31 wherein the exogenous nucleic acid molecule encoding the avian influenza HA antigen is codon optimized.
34. The recombinant virus vector of paragraph 21 wherein an amino acid sequence for a AIV HA COBRA-C gene as used in the recombinant virus vector has at least 80 percent, 85 percent, 90 percent, 95 percent, 96 percent, 97 percent, 98 percent, or 99 percent sequence identity to a predetermined sequence.
35. The recombinant virus vector of paragraph 21 wherein an amino acid sequence for an IBDV VP2 gene of the recombinant virus vector has at least 80 percent, 85 percent, 90 percent, 95 percent, 96 percent, 97 percent, 98 percent, or 99 percent sequence identity to a predetermined sequence.
36. The recombinant virus vector of paragraph 21 wherein an amino acid sequence for an NDV-F gene of the recombinant virus vector has at least 80 percent, 85 percent, 90 percent, 95 percent, 96 percent, 97 percent, 98 percent, or 99 percent sequence identity to a predetermined sequence.
37. A composition or vaccine comprising:
   a recombinant virus vector comprising:
      a predetermined virus selected from Marek's Disease serotype 3 (MDV-3) or Meleagrid herpesvirus 1 (MeHV-1);
      at least one Avian Influenza A virus polynucleotide coding for and expressing a synthetic hemagglutinin (HA) protein gene; and
      at least one heterologous polynucleotide coding for and expressing at least one antigen of an avian pathogen comprising at least one of:
         an Infectious Bursal Disease Virus (IBDV) capsid protein (VP2); or
         a Newcastle Disease Virus fusion protein (NDV-F); and
   a pharmaceutically or veterinarily acceptable carrier, excipient, vehicle, or adjuvant.
38. The composition or vaccine of paragraph 37 wherein the at least one Avian Influenza A virus polynucleotide has at least 80 percent, 90 percent, 92 percent, 95 percent or 99 percent sequence identity to an AIV H5 HA reference sequence.
39. The composition or vaccine of paragraph 37 wherein the at least one heterologous polynucleotide has at least 85 percent, 90 percent, 92 percent, 95 percent, or 99 percent sequence identity to a polypeptide having a predetermined reference sequence.
40. The composition or vaccine of paragraph 37 wherein the synthetic hemagglutinin (HA) protein of Avian Influenza virus A (subtype H5) is encoded by an amino acid sequence having at least 90 percent sequence identity to an AIV reference sequence; wherein the at least one heterologous polynucleotide comprises the Newcastle Disease Virus fusion protein (NDV-F) and has at least 90 percent sequence identity to an NDV-F reference sequence; and wherein the composition or vaccine further comprises a pharmaceutically or veterinarily acceptable carrier, excipient, vehicle or adjuvant.
41. The composition or vaccine of paragraph 37 wherein the synthetic hemagglutinin (HA) protein of Avian Influenza virus A (subtype H5) is encoded by an amino acid sequence having at least 90 percent sequence identity to an AIV reference sequence; wherein the at least one heterologous polynucleotide comprises the IBDV VP2 protein and has at least 90 percent sequence identity to an IBDV VP2 reference sequence; and wherein the composition or vaccine further comprises a pharmaceutically or veterinarily acceptable carrier, excipient, vehicle or adjuvant.

## Claims

1. An avian influenza virus (AIV) subtype H5 haemagglutinin (AIV-H5-HA) antigen, wherein the AIV-H5-HA antigen comprises an amino acid sequence having the sequence set forth in SEQ ID NO: 48.

2. A polynucleotide encoding the AIV-H5-HA antigen according to claim 1.

3. A plasmid comprising the polynucleotide of claim 2.

4. A recombinant virus vector encoding and expressing an AIV-H5-HA antigen comprising an amino acid sequence having the sequence set forth in SEQ ID NO: 48.

5. The recombinant virus vector of claim 4, wherein the recombinant virus vector comprises:
at least one nucleic acid sequence derived from herpesvirus of turkeys (rHVT); Marek's disease virus, or Meleagrid herpesvirus;
a first exogenous nucleic acid sequence encoding and expressing the AIV-H5-HA antigen, such that the recombinant virus vector expresses the AIV-H5-HA antigen *in vivo*; and
a second exogenous nucleic acid sequence from an avian pathogen encoding for and expressing at least one antigen of the avian pathogen *in vivo.*

6. The recombinant virus vector of claim 4 or 5, wherein the recombinant vector is a recombinant herpesvirus of turkeys (rHVT) vector.

7. A composition or vaccine comprising the AIV-H5-HA antigen, polynucleotide, plasmid or recombinant virus vector of any of claims 1 to 6 and optionally a pharmaceutically or veterinarily acceptable carrier, excipient, vehicle, or adjuvant.

8. The AIV-H5-HA antigen, polynucleotide, plasmid, recombinant virus vector or composition of any of claims 1 to 7, which is capable of inducing a protective immune response against avian influenza in an avian.

9. The AIV-H5-HA antigen, polynucleotide, plasmid, recombinant virus vector or composition of any of claims 1 to 8 for use in a method of inducing a protective immune response against avian influenza in an avian.
